# EUROPEAN PATENT APPLICATION

(11) **EP 4 578 961 A2**
(43) Date of publication of application: **02.07.2025**
(21) Application number: 25162925.9
(22) Date of filing: 14.02.2020
(51) Int. Cl.: C12Q 1/6883

(54) **SALIVARY BIOMARKERS OF BRAIN INJURY**

(30) Priority: 14.02.2019 US 201962805761 P; 07.08.2019 US 201962884104 P
(62) Divisional of application: 20708645.5
(71) Applicant: Marker Diagnostics UK Limited, London EC4Y 8EH (GB)
(72) Inventor: BELLI, Antonio, Romsey, SO51 6BN (GB); DI PIETRO, Valentina, Birmingham, B29 6QJ (GB)
(74) Representative: Haseltine Lake Kempner LLP

(57) **Abstract**

Methods of diagnosing, monitoring, treating, and predicting the course of traumatic brain injury (TBI), including mild traumatic brain injury (mTBI), include determining a level of at least one RNA biomarker (e.g., miRNA) in a saliva sample from a subject. Also described are sensor elements, detection systems, compositions, and kits for diagnosing, monitoring, treating, and predicting the course of TBI.

## Description

### FIELD

The present invention relates to compositions, kits, systems and methods for diagnosing and/or monitoring brain injury, including, without limitation, traumatic brain injury (TBI). More particularly, the present invention relates to the diagnosis and monitoring of TBI using RNA biomarkers.

### BACKGROUND

Traumatic brain injury (TBI) is the leading cause of death and disability under the age of 45 years in Western countries. Its healthcare burden and social costs are expected to continue to rise and, by 2020, the World Health Organization projects TBI to become the third leading cause of disability worldwide.

Despite many studies, no reliable biomarkers have been found to assess the severity of TBI and predict recovery. This is especially true for mild TBI (mTBI), which remains currently difficult to assess in clinical practice. Although TBI patients are initially assessed by the Glasgow Coma Score (GCS) and neuroimaging techniques, which require costly equipment, the current diagnostic tools are lacking in the ability to precisely define and quantify the actual severity of the brain injury, thus leading to an easy detection of severe but not of mTBI, which represents the vast majority of cases (75-90%).

The correct diagnosis of mTBI is particularly important in patients, such as athletes, soldiers and children, who are at greater risk of repetitive mTBI and a catastrophic form of brain injury known as second impact syndrome (SIS) where the synergistic effects of repeated TBI result in profound damage and even death. Early diagnosis and evaluation of the severity of TBI thus becomes crucial for patients' wellbeing and ultimately saving their life.

The subject technology was devised with these issues in mind.

### SUMMARY

The disclosure provides methods and detection systems relating to diagnosing, monitoring, and treating traumatic brain injury (TBI) including mild traumatic brain injury (mTBI) in a human subject who has suffered an injury to the head by detecting one or more miRNA molecules in a biological sample from the subject. In the context of the following disclosure, the terms 'level' and 'amount' in reference to the level or amount of an miRNA molecule or molecules in a biological sample are used interchangeably.

In one aspect, the disclosure relates to a method of diagnosing and treating traumatic brain injury (TBI) in a human subject in need thereof. The method involves: obtaining a saliva sample from the subject; contacting the saliva sample with a probe comprising a nucleic acid able to bind to at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof; determining an amount of the at least one RNA biomarker in the saliva sample; identifying the subject as having TBI where the amount of the at least one RNA biomarker is increased or decreased relative to a predetermined threshold value or relative to the amount of the RNA biomarker in a control sample; and treating the subject identified as having TBI according to one or more of the following: subjecting the subject to a verbal, cognitive, motor, or optical test, or any combination of the foregoing; subjecting the subject to diagnostic imaging in the form of a CT or MRI, or a combination thereof; and/or administering to the subject one or more neuroprotective therapies.

In certain embodiments of this method, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

In another aspect, the present disclosure relates to a method of diagnosing and/or monitoring traumatic brain injury (TBI) in a subject. This method involves: determining a level of at least one RNA biomarker in a saliva sample obtained from the subject, wherein the at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, and YRNA-255, or any combination thereof.

In one embodiment of this method, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

In another aspect, the present disclosure relates to a sensor element for a detection system for diagnosing and/or monitoring TBI, the sensor element comprising a substrate functionalized with a probe specific for at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

In one embodiment of the sensor element, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

In another aspect, the present disclosure relates to a detection system for diagnosing and/or monitoring TBI, comprising: a sensor element according to the present disclosure, and a detection device capable of detecting the binding of a target RNA biomarker to the probe.

In another aspect, the present disclosure relates to a method for determining a course of action for a subject suspected of having TBI, comprising applying a saliva sample obtained from the subject to a detection system according to the present disclosure, and if an upregulated level or a downregulated level of the at least one RNA biomarker is detected, providing a treatment for TBI.

In another aspect, the present disclosure relates to a method of treating a subject with suspected TBI. This method involves: determining whether an upregulated level or a downregulated level of at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof, is detectable in a saliva sample obtained from the subject, and if an upregulated level or a downregulated level of at least one RNA biomarker is detected, providing treatment for TBI to the subject.

In one embodiment of this method, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

In another aspect, the present disclosure relates to a method of detecting an RNA biomarker in a saliva sample. This method involves: obtaining a saliva sample from a human subject, contacting the saliva sample with at least one oligonucleotide primer complementary to at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof; amplifying the at least one RNA biomarker using a polymerase chain reaction; and detecting the amplified RNA biomarker.

In one embodiment of this method, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

In another aspect, the present disclosure relates to a kit for use in a method of diagnosing and/or monitoring traumatic brain injury (TBI) in saliva from a human subject, the kit comprising at least one probe specific for at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

In one embodiment of this kit, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

In another aspect, the present disclosure relates to a composition for use in a method of diagnosing and/or monitoring traumatic brain injury (TBI) in saliva from a human subject, the composition comprising at least one probe specific for at least one RNA biomarker: (a) selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or (b) selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

In one embodiment of this composition, the at least one RNA biomarker further comprises one or more miRNA: (a) selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or (b) selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

### DETAILED DESCRIPTION

The present invention provides methods of diagnosing or monitoring traumatic brain injury (TBI) in a subject, as well as methods for treating a subject identified as suffering from TBI.

The quest for TBI biomarkers has received significant impetus by the increased profile of sport concussion in the media. In the last few years many studies have focused on biomarkers that can support clinical decision making pitch-side or in a sports clinic. However, protein biomarkers reported in the literature lack specificity or sensitivity, or are not detectable for some time after injury. This may be due to the fact that following concussion, which is a form of TBI, brain-derived compounds are only released in very small amounts and the blood-brain barrier remains mostly closed.

MicroRNAs (miRNAs) are an abundant class of highly conserved, non-coding RNA molecules of approximately 22 nucleotides in length that induce mRNA degradation, translational repression or both via pairing with partially complementary sites in the 3'UTR of target genes. The human genome encodes over 2,000 miRNAs, which may target about 60% of all genes. However, despite the abundance of miRNAs, their biomolecular functions and involvement in pathology remain to be fully elucidated. They play a central role in many biological processes including cell cycle, cell metabolism, apoptosis and immune responses, and are attracting increasing interest in clinical research as potential biomarkers for the detection, identification and classification of cancers and other disease states including neurodegenerative diseases.

For the avoidance of doubt, it will be understood that "the at least one miRNA is selected from a group of miRNAs", as used herein, means that the method in question, whether carried out for a diagnostic, prognostic, or therapeutic purpose, can be carried out with any one of the listed miRNAs or with any plurality of the listed miRNAs (e.g., two, three, four, or more of the listed miRNAs). It follows that any one or more of the listed miRNAs may be explicitly excluded.

Traumatic brain injury occurs when an external force traumatically injures the brain. There are different systems for classifying TBI based on, for example, severity, type of injury and prognosis. The most commonly used system for classifying TBI is the Glasgow Coma Scale (GCS), which grades a person's level of consciousness on a scale of 3-15 based on verbal, motor, and eye-opening reactions to stimuli. In general, a TBI with a GCS score of 13 or above is defined as mild, 9-12 as moderate and 8 or below as severe. Another system, the Mayo Classification System, has three main classifications including definite moderate-severe TBI, probable mild TBI, and possible TBI. Multiple criteria are used in each diagnosis including loss of consciousness, post-traumatic amnesia, skull fracture, and evidence of neuroradiological abnormalities including subdural haematoma, cerebral contusion, and hemorrhagic contusion. The classification of TBI using the GCS or Mayo systems will be known to those skilled in the art.

As used herein, references to "mild", "moderate" and "severe" TBI are made in accordance with the GCS. References herein to "moderate-to-severe" TBI encompass both moderate and severe TBI in accordance with the GCS.

As used herein, a reference to mild TBI (mTBI) is also a reference to concussion.

The diagnosis and/or monitoring of TBI using the biomarkers of the present invention is expected to support clinical decision making and treatment regimens in a variety of contexts, including the following situations: as part of the initial assessment by paramedics to determine whether patients should be transported to a facility with neurosurgical expertise, a major trauma centre or a local trauma unit; in the emergency department of hospitals to determine appropriate treatment, including the need for a CT brain scan; pitch-side, to assist decision making as to the removal of a player from play and assessment of the need to take a player to hospital; in sports clinics, to confirm a concussive event and enable decision making with regard to returning to play; in combat situations, to determine the need to dispatch a rescue team and evacuate a victim. Thus, subjects for whom the present invention provides particular benefit can include, without limitation, accident victims, sports players, and military personnel.

In any case, but perhaps particularly where the subject is at greater risk for a TBI (e.g., where the subject is a professional athlete or enlisted in the military), a sample may be obtained from the subject at a time prior to any known or recent trauma (e.g., near the beginning of a sporting career or prior to a military deployment) and any miRNAs of interest can be assessed at that time or later, when the subject has experienced a possible TBI. Such samples may thereby provide an internal reference standard.

In some embodiments, the subject is human.

The TBI may be mild TBI (mTBI), moderate TBI or severe TBI (sTBI). In some embodiments, the TBI is moderate-to-severe TBI (m-sTBI).

The level of the miRNA or of each miRNA in the sample may be determined quantitatively or semi-quantitatively. By "quantitatively", it will be understood that the absolute amount or concentration of the miRNA or of each miRNA in the sample is determined. The absolute amount of the miRNA or of each miRNA in the sample can then be compared to a predetermined threshold (e.g. a published literature value for expected normal levels), a known level of the same or a reference miRNA in a control sample taken from a healthy subject, or the amount of a reference miRNA in the sample taken from the subject. In some embodiments, the subject is diagnosed as having a TBI when the level of the miRNA is below the predetermined threshold, or decreased relative to a reference or control sample. In other embodiments, the subject is diagnosed as having a TBI when the level of the miRNA is increased compared to the predetermined threshold.

By "semi-quantitatively", it will be understood that the level of the or each miRNA of interest is measured relative to a reference.

The reference may be an invariant miRNA, i.e. a miRNA having an expression level that remains substantially unchanged between healthy subjects and those having a TBI. A subject may be diagnosed as suffering from a TBI if the level of the miRNA or of each miRNA of interest is increased or decreased relative to that of an invariant miRNA.

In some embodiments, the level of the miRNA or of each miRNA in the sample obtained from the subject may be about 0.01 times to about 100 times, about 0.05 times to about 50 times, about 0.1 times to about 10 times, about 0.5 times to about 5 times, about 1.0 to about 3 times, or about 1.5 times to about 2.0 times lower or higher than the level in the control sample, the reference level or the published value.

Where a device or method is employed to generate a value, we may qualify that value with the term "about" in order to capture the stated value and any variation of that value inherent to the device or method employed. Where values or ranges of values are specifically disclosed, "about" may mean plus-or-minus 10% of the stated value or range. For example, about 10 minutes may mean 9-11 minutes.

The level of the miRNA or of each miRNA of interest can be determined using methods known to those skilled in the art. In some embodiments, determining the level of the miRNA or of each miRNA of interest comprises amplifying the miRNA. In some embodiments, total miRNA may be first isolated from the sample using standard techniques, for example using the miRNeasy mini kit (Qiagen). The amount of the miRNA of interest can then be determined. In some embodiments, the level of the miRNA or of each miRNA of interest in the sample is determined using PCR (polymerase chain reaction). For example, quantitative PCR may be used for quantitative determination of the level of the miRNA or of each miRNA of interest. PCR may also be used for semi-quantitative determination, by comparing the level of the miRNA or of each miRNA of interest in the sample with that of a reference (e.g. an invariant miRNA).

Suitable techniques for miRNA detection and/or quantification, which will be known to those skilled in the art, include qPCR, miRNA assays, next-generation sequencing (NGS), and multiplex miRNA profiling assays.

In some embodiments, the level of the miRNA or of each miRNA of interest is determined using in-situ hybridization, for example using a probe (e.g., a labelled probe) specific for the miRNA.

The level of miRNA may be determined in a sample which was obtained from the subject immediately after injury (i.e. less than 1 hour after injury), and/or in a sample obtained at one or more time points a few hours or days after injury. Thus, changes in the miRNA level can be detected over time to enable monitoring of a TBI. In the event miRNA levels change over time, the methods described herein for monitoring TBI can be expanded to include maintaining or adjusting the subject's treatment regimen accordingly.

Depending on the specific miRNA and the type of TBI, the level of miRNA in the subject may change significantly over time. In some embodiments, it may therefore be advantageous to measure the miRNA relatively soon after injury to enable an accurate diagnosis. In some embodiments, the level of miRNA is determined in a sample obtained from the subject no more than 72 hours, no more than 48 hours, no more than 36 hours, no more than 24 hours, no more than 12 hours, no more than 6 hours, no more than 4 hours, no more than 2 hours or no more than 1 hour after injury.

The level of some miRNAs is substantially stable over time, thus allowing a diagnosis to be made a few hours, days or even weeks after injury. In some embodiments, the level of miRNA is determined in a sample obtained from the subject up to 20, 18, 15, 12, 10, 8, 5 or 2 days from injury.

In some embodiments, the level of miRNA is determined in a sample obtained from the subject immediately after injury (e.g. at T=0 h), at 4-12 hours after injury, at 48-72 hours after injury, or at 15 days after injury.

In some embodiments, the level of miRNA is determined in a sample obtained from the subject at least 24 hours after injury. In some embodiments, the level of miRNA is determined in a sample obtained from the subject 15 days or fewer after injury. In some embodiments, the level of miRNA is determined in a sample obtained from the subject between 24 hours and 15 days after injury, or between 24 hours and 10 days after injury, or between 24 hours and 7 days after injury, or between 48 hours and 5 days after injury.

In some embodiments, the TBI is mild TBI (mTBI) or moderate-to-severe TBI (m-sTBI).

In some embodiments, the TBI is mild TBI (mTBI).

Conveniently the sample may be any appropriate fluid or tissue sample obtained from the subject. For example, the biological sample may comprise at least one of the group consisting of: urine, saliva, whole blood, plasma, serum, sputum, semen, faeces, a nasal swab, tears, a vaginal swab, a rectal swab, a cervical smear, a tissue biopsy, and a urethral swab. In some embodiments the sample is a fluid sample. Suitably, the sample is one that can be readily obtained from the individual, such as urine, saliva, blood and sputum. In some embodiments, the sample comprises saliva, blood, plasma or serum. It will be appreciated that in some embodiments the process of obtaining the sample does not form part of the invention described herein.

In some embodiments, the sample comprises or is constituted by serum. Not only does serum have practical advantages, but it is also free of anticoagulants such as heparin, a potential inhibitor of PCR reactions. Serum may also be less affected by haemolysis, compared to plasma.

In some embodiments, the sample is saliva. Saliva can be easily obtained from the patient (e.g. pitch-side, or in the field), without specialist training or medical equipment.

The diagnosis of a subject as suffering from a TBI, and in particular diagnosis of mild TBI or moderate-to-severe TBI, may facilitate in the determination of an appropriate treatment. The present invention thus provides a test that enables healthcare workers, such as physicians, clinicians, paramedics, and even non-medical personnel (e.g. teachers, sports coaches, military personnel) to decide on appropriate action for a subject suspected of having a TBI. A subject determined as having a TBI may therefore receive the most appropriate treatment as a result of a diagnosis being made. The method of the invention may thus further comprise directing appropriate therapy to a subject diagnosed with a TBI.

A subject diagnosed with TBI may be further evaluated, e.g. by CT scanning. In some embodiments, the subject is admitted to hospital. In some embodiments, if moderate-to-severe TBI can be ruled out, the subject may not need to be admitted to hospital for evaluation. A subject diagnosed with moderate-to-severe TBI may be admitted to hospital, or a specialist centre with neurotrauma expertise.

A subject diagnosed with a TBI (particularly mTBI) outside a hospital environment, for example, at a sporting event, during combat or during play, may be removed from play or combat immediately. The subject may subsequently be started on a graduated return to play or combat.

In a further aspect, there is provided a method for determining whether it is appropriate to administer to a subject a therapy for alleviating TBI, the method comprising: determining a level of at least one miRNA in a sample from the subject; and determining whether or not it is appropriate to administer a therapy for alleviating TBI, based on the level of the at least one miRNA.

It will be appreciated that the step of administering the therapy to the subject does not form a part of the claimed method, unless specifically stated.

In some embodiments the method may further comprise administering to the subject an appropriate treatment. In some embodiments, the treatment may comprise a therapy for alleviating TBI. Accordingly, the invention features methods of diagnosing and treating TBI in a subject, the method comprising the steps of (a) obtaining a sample (e.g., a sample of blood, plasma, urine, or saliva) from the subject; (b) detecting one or more miRNAs (selected from those described herein); diagnosing the patient as having a TBI when the level(s) of the miRNA(s) differ from a reference standard (as described herein); and administering a treatment for the TBI.

In a further aspect, the invention provides a method of determining an appropriate treatment to a subject suspected of suffering from a TBI, the method comprising identifying whether or not the subject has a TBI by determining a level of at least one miRNA in a sample from the subject.

If a subject is identified as having a TBI, an appropriate treatment may include one or more of the following: further evaluating the subject, for example by further tests (e.g. verbal, cognitive, motor and/or optical tests), CT and/or Mill scanning; removing the subject from activity (e.g. the activity during which the TBI was incurred); admitting the subject to hospital or a specialist clinic; surgery; and administering a therapy for alleviating TBI to the subject.

The therapy for alleviating TBI may include neuroprotective drugs, e.g. drugs to treat cerebral swelling such as mannitol and hypertonic saline, and/or other neuroprotective measures, such as avoidance of hypertensive resuscitation and the use of sedation.

In some embodiments, the subject may be subsequently monitored to track their recovery, for example in a hospital or clinic setting.

According to a further aspect of the invention, there is provided a method of detecting and/or determining a level of a target miRNA in a subject, the method comprising the steps of (a) obtaining a sample from the subject; and (b) detecting and/or determining the level of the target miRNA in the sample by contacting the sample with a probe that is specific for the target miRNA.

The sample may be any appropriate fluid or tissue sample obtained from the subject, as defined above. In some embodiments, the sample is blood, serum, plasma, urine or saliva.

In some embodiments, the method may comprise determining the level of two or more target miRNAs in the sample.

According to a further aspect of the invention, there is provided a therapy for alleviating TBI for use in a method of treating a subject in need thereof, wherein said subject is identified as having a TBI by determining a level of at least one miRNA in a sample from the subject.

The step of determining the level of the target miRNA may comprise contacting the sample with a substrate functionalized with the probe, for example a chip comprising the probe. The substrate or chip may conveniently include multiple probes, each specific for a different target miRNA.

The subject may have suffered an injury, in particular a head injury. The subject may be suspected as having a TBI. The subject may be suspected of having mTBI or concussion. In some embodiments, the sample is obtained no more than 72 hours, no more than 48 hours, no more than 36 hours, no more than 24 hours, no more than 12 hours, no more than 6 hours, no more than 4 hours, no more than 2 hours or no more than 1 hour after injury. In some embodiments the sample is obtained 24 hours or more after the injury. In further embodiments the sample is obtained from the subject 15 days or fewer after injury. In some embodiments the sample is obtained from the subject between 24 hours and 15 days after injury, or between 24 hours and 10 days after injury, or between 24 hours and 7 days after injury, or between 48 hours and 5 days after injury.

In some embodiments, the method further comprises treating the subject. The treatment may include one or more of the following: further evaluating the subject, for example by further tests (e.g. verbal, cognitive, motor and/or optical tests), CT and/or MRI scanning; removing the subject from activity (e.g. the activity during which the TBI was incurred); admitting the subject to hospital or a specialist clinic; and administering a therapy for alleviating TBI to the subject. In some embodiments, the treatment comprises administering an effective amount of a neuroprotective drug.

Thus, in yet a further aspect the invention provides a method of treating TBI, the method comprising: determining a level of at least one miRNA in a sample from the subject; and if the level of the at least one miRNA is indicative of mTBI, administering a treatment appropriate for mTBI; or if the level of the at least one miRNA is indicative of m-sTBI, administering a treatment appropriate for m-sTBI.

It will be appreciated by those skilled in the art that different treatment pathways may be used for mTBI and m-sTBI.

A subject with mTBI may be treated as follows:

An athlete diagnosed with mTBI would typically be started on a graduated return to play protocol (as defined by the Berlin Consensus Conference on Concussion and individual sport authorities, e.g. RFU, IRB, NFL, NHL, NBA, IMMAF). This would involve a period of rest followed by a gradual increase in level of exertion and exposure to contact. An athlete with mTBI would typically not be able to play for a period that varies between 6 and 23 days, depending on level of medical supervision and age. Conversely, if mTBI was excluded, the athlete would not have any restrictions and could train as normal the following day. If diagnosed in a pre-hospital setting, mTBI would be an indication for a patient to seek medical attention (e.g. in hospital or in primary care), whereas, if mTBI was excluded no medical review would be indicated.

A person with mTBI must not be left unsupervised for 24-48 hours and must not drive or operate a fork lift or open machinery until recovered.

Military personnel diagnosed with mTBI would be removed from the operational theatre and would be rested, whereas, if mTBI could be excluded, that person would continue on active duties.

In the NHS, a person with mTBI may be kept in hospital for observation or may have a CT scan according to the guidance issued by NICE, whereas if mTBI could be excluded, that patient could be discharged straightaway.

A person diagnosed with mTBI would typically be asked not to return to work or study for a certain period of time.

A person diagnosed with mTBI may be referred to a mild TBI clinic or a neurology clinic or a neurosurgery clinic. Typical interventions for mTBI consist of education, advice in regard to work, study and driving, medical management of typical sequelae such as headache or anxiety or mood disorder or post-traumatic stress disorder with medication and/or psychological intervention if necessary, and referral to other services as indicated, e.g. neuropsychology, neurovestibular or ophthalmology.

A patient with mTBI would need to be monitored for delayed post-traumatic pituitary dysfunction according to the guidance issued by the Society of British Neurological Surgeons.

In the medico-legal context, the diagnosis of mTBI is often unclear and speculative, as the radiological investigations are typically normal and symptoms are nonspecific. Objective confirmation of mTBI may lead to an award for damages and care needs. An appropriate treatment for mTBI may include: removing the subject from activity; treatment in situ or in the community; further evaluating the subject in hospital without overnight admission (typically mTBI patients are discharged with promptly with head injury advice); or admission to hospital for a period of observation (typically 1-2 days). The subject may be further evaluated using tests (e.g. verbal, cognitive, motor and/or optical tests). CT scanning is generally only required if certain indications are present, including suspected skull fracture, post-traumatic seizure, focal neurological defecit, repeated vomiting, a GCS score of less than 13 on initial assessment (less than 14 for children, or less than 15 for infants under 1 year), in accordance with NICE guidelines.

An appropriate treatment for m-sTBI may include: MRI or CT scanning (particularly within 1 hour of injury); admission to hospital (which may include admission to intensive care and/or transfer to a specialist clinic or major trauma centre with neurosurgical facilities); neuromonitoring; surgery; administering a therapy for alleviating TBI, such as administering neuroprotective drugs, e.g. drugs to treat cerebral swelling such as mannitol and hypertonic saline, and/or other neuroprotective measures, such as avoidance of hypertensive resuscitation and the use of sedation.

Thus, the present invention enables subjects with a TBI to be clinically and quickly stratified into mTBI or m-sTBI, so that they may receive the most appropriate treatment.

According to a further aspect of the invention, there is provided a detection system for diagnosing and/or monitoring TBI, the detection system comprising a sensor element comprising a substrate functionalized with a probe specific for a target miRNA The detection system can further comprise a detection device that is capable of detecting the binding of a target miRNA to the probe.

According to a yet further aspect of the invention, there is provided a sensor element for use in a detection system for diagnosing and/or monitoring TBI, the sensor element comprising a substrate functionalized with a probe specific for a target miRNA.

The sensor element may further comprise a sample addition zone for receiving a sample (e.g. a fluid sample) thereon.

The probe is capable of selectively binding the miRNA of interest. The substrate may be functionalized with a plurality of probes. The probes may all be the same, or two or more different probes may be provided. For example, in some embodiments, the substrate may be functionalized with a first probe specific for a first miRNA, and a second probe specific for a second miRNA. The first and second probes may be grouped together, for example on different portions of the sensor element.

In a further aspect of the invention, there is provided a composition for use in a method of diagnosing and/or monitoring traumatic brain injury (TBI) in a subject, the composition comprising a probe specific for a target miRNA. The composition may comprise any one of the listed miRNAs or with any plurality of the listed miRNAs (e.g., two, three, four, or more of the listed miRNAs).

The probe may comprise a biological molecule such as a protein (e.g. an antibody) or a nucleic acid. In some embodiments, the probe comprises a nucleic acid. The nucleic acid may comprise a sequence which is at least 70%, at least 75%, at least 80%, at least 85%, at least 90% or at least 95% identical to a sequence which is the complement of the full-length sequence of the target miRNA. In some embodiments, the nucleic acid comprises a sequence which is 100% identical to a sequence which is the complement of the sequence of the target miRNA (i.e. the receptor comprises a nucleic acid sequence which is the exact complement of the target miRNA sequence).

The probes may be attached to a surface of the substrate by any suitable means, such as by coupling chemistry known to those skilled in the art. In some embodiments, each probe is attached to a surface of the substrate via a linker. In some embodiments, the probe comprises a moiety for immobilizing the probe on the substrate, or for attaching the probe to a linker immobilized on the substrate.

Alternatively or in addition, the probe may comprise a detectable label. The detectable label may be, for example, radioactive, fluorescent, luminescent, or antibody-based (e.g., it may constitute a conventional tetrameric antibody or a detectable fragment thereof).

The substrate of the sensor element may be formed from any suitable material. In some embodiments, the substrate comprises or is formed from metal, plastic, glass, silica, silicon, graphite, graphene, or any combination thereof. In some embodiments, the substrate comprises multiple layers. For example, a substrate may be prepared by forming a surface or layer of graphene on a layer of silicon carbide or silica. The graphene surface may be chemically modified, for example to graphene-oxide (GO) or graphene-amine (GA). Methods for forming graphene layers, such as epitaxial growth and sublimation growth, will be known to those skilled in the art.

Conveniently, probes comprising or constituted by a nucleic acid can be attached to a GO surface via a linker, using an amide coupling reagent (e.g. (O-(7-azabenzotriazole-1-yl)-N,N,N,N'-tetramethyluronium hexafluorophosphate (HATU)). A sensor element comprising a surface functionalized with a nucleic acid probe can then be used to selectively detect its complementary miRNA.

Suitable linkers may comprise an aniline moiety (or a derivative thereof), a benzoic acid moiety (or a derivative thereof) or an ethendiamine moiety (or a derivative thereof). An aniline linker may be formed by attaching a nitrobenzene molecule (or derivative) to a graphene surface (e.g. using a diazonium salt), and reducing the nitrobenzene to aniline. The amine group of the aniline may then be used to attach to the probe. Similarly, a diazonium salt (e.g. 4-benzoic acid diazonium tetrafluoroborate) can be used to attach a benzoic acid or benzoic acid derivative to a graphene surface. An ethanediamine moiety may be attached to carboxylated graphene or graphene oxide.

The sensor element may be comprised within a test strip. The test strip may be disposable.

The detection device may be configured to detect the binding of a target miRNA to the receptor by any suitable means known to those skilled in the art, for example by detecting changes in electrical impedance, hydrogen ion concentration, or conformational changes resulting from hybridisation.

The detection device may further include a user interface to output data to a user.

In some embodiments, the detection device includes a database of treatment information. The device may be capable of identifying suitable treatment options from the database depending on the levels of the miRNA of interest. The treatment information may be provided to the user via the user interface.

Conveniently, the detection device may be portable, e.g. hand-held. The detection device may comprise a data storage unit for storing miRNA levels and other information relating to the subject. In some embodiments, the device comprises a data communication means for communicating data to other devices. For example, the device may communicate data wirelessly through WiFi, 3G, 4G, Bluetooth, or through a mobile app. This may conveniently enable the data to be easily accessed by medical professionals if necessary.

It is thus envisaged that the detection device of the invention provides an affordable, portable, point of care means for diagnosing and monitoring TBI non-invasively. The device may be used by ambulance crews, the military, schools, sports clubs and healthcare professionals, enabling the correct assessment and triage of patients suspected to have a TBI.

A further aspect of the present invention is a system for detecting and/or monitoring mTBI in a subject.

A type of detection system is based on complementarity between a target miRNA and a nucleic acid probe, generally an oligonucleotide probe. The complementary or base pairing region can be 7 or 8 or more nucleotides in length. In embodiments the complementary or base pairing region can be 9, 10, 12, 15 or more nucleotides in length or the complementary or base pairing region can be the full length of the miRNA. The substrate functionalised with an oligonucleotide can be a bead or a nanoparticle. The detection system can have further components capable of converting bound nucleic acid probe-miRNA into a detectable signal.

Another type of detection system is based on RT-PCT. Therefore the present invention embraces a detection system for detecting and/or monitoring mTBI in a subject comprising a primer pair designed for amplification of a cDNA complement of at least one miRNA described herein.

In a further aspect there is provided a kit for use in the present methods. The kit may comprise at least probe (e.g. a protein, such as an antibody, or a nucleic acid) which is capable of selectively binding the miRNA of interest. In some embodiments, the kit comprises an array comprising a plurality of probes. In some embodiments, the at least one probe is a primer for carrying out PCR. The kit may further comprise instructions for use, for example instructions for use in the diagnosis and/or monitoring of TBI. The kit may further comprise suitable buffers and reagents, such as amplification primers and enzymes (e.g. DNA polymerase, reverse transcriptase for conversion of miRNA to cDNA).

### Additional Aspects and Embodiments

In further aspects, the present disclosure provides, *inter alia,* methods, sensor elements, detection systems, kits, and compositions involving various differentially expressed RNA biomarkers suitable for use in diagnosing, monitoring, and treating traumatic brain injury (TB), including mild traumatic brain injury (mTBI), in a human subject.

The sequences and accession numbers for these RNA biomarkers are provided in **Table 1** below:

**Table 1**

| **RNA Biomarker** | **Sequence** | **Accession No.** |
|---|---|---|
| put-miR-410 | ggagaatagaacatgctgatt (SEQ ID No. 70) | |
| put-miR-1306 | ataacgtcatctagtgtg (SEQ ID No. 71) | |
| put-miR-806 | cacgagagaacgcacacc (SEQ ID No. 72) | |
| put-miR-71 | gaccttgggttgggtcgtggt (SEQ ID No. 73) | |
| put-miR-468 | ataaggaactgctctctc (SEQ ID No. 74) | |
| put-miR-476 | gtagtcaggtagagaa (SEQ ID No. 75) | |
| put-miR-293 | gggtaaaactgcagtgggcgttggtag (SEQ ID No. 76) | |
| put-miR-742 | cagggctgtgctaact (SEQ ID No. 77) | |
| put-miR-6 | gcggaccttgctcaagg (SEQ ID No. 78) | |
| put-miR-1207 | gtgaaaagacataggggg (SEQ ID No. 79) | |
| put-miR-1084 | aggaccattgcgttgcc (SEQ ID No. 80) | |
| put-miR-92 | agagactgactttgagta (SEQ ID No. 81) | |
| put-miR-188 | aaatggcgatactcagg (SEQ ID No. 82) | |
| put-miR-1352 | tggattgtgggggaacc (SEQ ID No. 83) | |
| put-miR-1146 | caactgtaagtccatt (SEQ ID No. 84) | |
| put-miR-209 | ggtcctcggatcggcc (SEQ ID No. 85) | |
| put-miR-961 | tagcttaatccagttg (SEQ ID No. 86) | |
| put-miR-1080 | tgtggattgatgctct (SEQ ID No. 87) | |
| put-miR-750 | caagagatgaggaatg (SEQ ID No. 88) | |
| put-miR-1003 | tggacttcagaacagc (SEQ ID No. 89) | |
| put-miR-1098 | aaggcgaaggatatgttg (SEQ ID No. 90) | |
| hsa-miR-934 | tgtctactactggagacactgg (SEQ ID No. 91) | |
| hsa-miR-142-5p | cataaagtagaaagcactact (SEQ ID No. 92) | |
| hsa-miR-6748-3p | tcctgtccctgtctcctacag (SEQ ID No. 93) | MIMAT0027397 |
| hsa-miR-1271-5p | cttggcacctagcaagcactca (SEQ ID No. 94) | MIMAT0005796 |
| hsa-miR-34b-3p | caatcactaactccactgccat (SEQ ID No. 95) | MIMAT0004676 |
| hsa-miR-449a | tggcagtgtattgttagctggt (SEQ ID No. 96) | MIMAT0001541 |
| hsa-miR-143-3p | tgagatgaagcactgtagctc (SEQ ID No. 48) | MIMAT0000435 |
| hsa-miR-135b-5p | tatggicttttcattcctatgtga (SEQ ID No. 40) | MIMAT0000758 |
| U6.428 | aagattagcatgaggatgacacgcaaattcgtgaagcgttccatttc ttt (SEQ ID No. 97) | |
| RNU6-4 | ggcccctgcacagggatgacacgcaaattcgtgaagcgttccatat tttt (SEQ ID No. 98) | |
| RNU6-7 | ggcccctgcgcaaggatgacatgcaaattcgtgaagcgttccatatt ttt (SEQ ID No. 99) | |
| RNU6-6 | ggcccctgtgcaaggatgacacgcaaattcgtgaagcgttccatatt ttt (SEQ ID No. 100) | |
| RNU6-45 | ggcccttgtgcaaggatgacacgcaaattcgtgaagcgttccatatt ttt (SEQ ID No. 101) | |
| RNU6-73 | ggcccctgtgcaaggatgacatgcaaattcgtgaagcgttccatatt ttt (SEQ ID No. 102) | |
| Y_RNA.255 | gugucaccaacguugguauacaaccccccacaacuaaauuug acuggcuu (SEQ ID No. 103) | |
| U6.375 | ggcccctgtgcaagaatgactcgcaaattcgtgaagcgttccatatt ttt (SEQ ID No. 104) | |
| U6.1249 | gatggcatgacccctgatcaaggacggcatgcaaatttgtgaagta tttc (SEQ ID No. 105) | |

According to an aspect of the present disclosure, there is provided a method of diagnosing and treating traumatic brain injury (TBI) in a human subject in need thereof, the method comprising: obtaining a saliva sample from the subject; contacting the saliva sample with a probe comprising a nucleic acid able to bind to at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p; determining an amount of the at least one RNA biomarker in the saliva sample; identifying the subject as having TBI where the amount of the at least one RNA biomarker is increased or decreased relative to a predetermined threshold value or relative to the amount of the RNA biomarker in a control sample; and treating the subject identified as having TBI according to one or more of the following: subjecting the subject to a verbal, cognitive, motor, or optical test, or any combination of the foregoing; subjecting the subject to diagnostic imaging in the form of a CT or MRI, or a combination thereof; and/or administering to the subject one or more neuroprotective therapies.

Examples of neuroprotective therapies that may be chosen by a clinician for treating TBI can include, without limitation, statins, progesterone, corticosteroids, cell cycle inhibitors (such as flavopiridol, a semi-synthetic flavonoid, and purine analogs roscovitine and olomoucine), autophagy inhibitors including caspase inhibitors (such as the tetrapeptide caspase-3 inhibitor (z-DEVD-fmk), the pan-caspase peptide inhibitor Boc-aspartyl fluoromethylketone, and Boc-aspartyl fluoromethylketone), inhibitors of poly (ADP-ribose) polymerase (PARP), anti-inflammatory agents (such as minocycline and anti-inflammatory cytokines (e.g., IL-10) or interleukin-1 receptor antagonist (IL-1ra)), sulfonylurea receptor 1 (SUR1)-regulated calcium channel inhibitors (such as glibenclamide), Substance P (SP) antagonists, diketopiperazines, and cyclosporine A.

In some embodiments, the saliva sample is obtained during a period of time after injury selected from immediately after the injury to any period post-injury.

In some embodiments, the saliva sample is obtained during a period of time after injury selected from immediately after the injury until the expression level of at least one of the RNA biomarkers returns to the predetermined threshold value or to the amount of the RNA biomarker in the control sample.

In some embodiments, the saliva sample is obtained during a period of time after injury selected from immediately after the injury to up to 1 year, up to 10 months, up to 8 months, up to 6 months, up to 5 months, up to 4 months, up to 3 months, up to 2 months, up to 1 month, up to 25 days, up to 20 days, up to 15 days, up to 7 days, up to 5 days, up to 3 days, up to 2 days, and/or up to 24 hours post-injury.

In some embodiments, the saliva sample is obtained during a period of time after injury selected from immediately after the injury to 15 days, from 1 hour to 15 days, from 24 hours to 15 days, from 24 hours to 7 days, and from 2 to 5 days.

In some embodiments, the method further comprises obtaining one or more additional saliva samples from the subject at one or more additional times after the injury and repeating the detecting and amplifying steps for each additional sample.

In some embodiments, the one or more additional saliva sample is obtained during a period of time after injury selected from immediately after the injury to any period post-injury.

In some embodiments, the one or more additional saliva sample is obtained during a period of time after injury selected from immediately after the injury until the expression level of at least one of the RNA biomarkers returns to the predetermined threshold value or to the amount of the RNA biomarker in the control sample.

In some embodiments, the one or more additional saliva sample is obtained during a period of time after injury selected from immediately after the injury to up to 1 year, up to 10 months, up to 8 months, up to 6 months, up to 5 months, up to 4 months, up to 3 months, up to 2 months, up to 1 month, up to 25 days, up to 20 days, up to 15 days, up to 7 days, up to 5 days, up to 3 days, up to 2 days, and/or up to 24 hours post-injury.

In some embodiments, the one or more additional saliva sample is obtained during a period of time after injury selected from immediately after the injury to 15 days, from 1 hour to 15 days, from 24 hours to 15 days, from 24 hours to 7 days, and from 2 to 5 days.

In some embodiments, the one or more additional saliva samples is obtained at day 1, 1.5, 2, 3, 4, 5, 6, 7, 8, 9, 10, 11, 12, 13, 14, or 15 after the injury.

In some embodiments, the detecting an amount of the at least one RNA biomarker is performed using a PCR-based assay, a light array assay, a laminar flow chip assay, or any assay suitable for detecting that at least one RNA biomarker.

In some embodiments, when using the PCR-based assay the predetermined threshold is equivalent to a fold change of 1.5 or more using the 2-delta delta CT (2-ΔΔCT) method.

In some embodiments, the predetermined threshold is equivalent to a fold change of 2 or more using the 2-delta delta CT (2-ΔΔCT) method.

In some embodiments, the at least one RNA biomarker further comprises one or more miRNA selected from the group consisting of hsa-miR-143-3p and hsa-miR-135b-5p.

In some embodiments, the method further comprises identifying the human subject as being fit for normal activity after undergoing successful treatment for TBI.

In another aspect of the present disclosure, there is provded a method of diagnosing and/or monitoring traumatic brain injury (TBI) in a subject, the method comprising determining a level of at least one RNA biomarker in a saliva sample obtained from the subject, wherein the at least one RNA biomarker is selected from the group consisting of Y RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p, or any combination thereof.

In some embodiments, either an upregulated level or a downregulated level of the at least one RNA biomarker is indicative of TBI.

In some embodiments, the subject is diagnosed as having TBI if the level of the at least one RNA biomarker is either above or below a predetermined threshold or increased or decreased relative to a control.

In some embodiments, the method further comprises identifying the human subject as being fit for normal activity after undergoing successful treatment for TBI.

In some embodiments, the at least one RNA biomarker further comprises one or more miRNA selected from the group consisting of hsa-miR-143-3p and hsa-miR-135b-5p.

In another aspect of the present disclosure, there is provided a sensor element for a detection system for diagnosing and/or monitoring TBI, the sensor element comprising a substrate functionalized with a probe specific for at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p.

In some embodiments, the probe comprises a nucleic acid able to bind to the at least one RNA biomarker.

In some embodiments, the probe comprises a nucleic acid having at least 70% identity with a sequence which is the complement of the sequence of the target RNA biomarker.

In some embodiments, the probe comprises a nucleic acid having at least 70% identity with a sequence which is the complement of SEQ ID NO: 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, and 105.

In some embodiments, the at least one RNA biomarker further comprises one or more miRNA selected from the group consisting of hsa-miR-143-3p and hsa-miR-135b-5p.

In another aspect of the present disclosure, there is provided a detection system for diagnosing and/or monitoring TBI, comprising a sensor element according to the present disclosure, and a detection device capable of detecting the binding of a target RNA biomarker to the probe.

In some embodiments, the detection system further comprises means to determine whether the target RNA biomarker is upregulated or downregulated.

In another aspect of the present disclosure, there is provided a method for determining a course of action for a subject suspected of having TBI, comprising applying a saliva sample obtained from the subject to a detection system according to the present disclosure, and if an upregulated level or a downregulated level of the at least one RNA biomarker is detected, providing a treatment for TBI.

In another aspect of the present disclosure, there is provided a method of treating a subject with suspected TBI, the method comprising determining whether an upregulated level or a downregulated level of at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p is detectable in a saliva sample obtained from the subject, and if an upregulated level or a downregulated level of at least one RNA biomarker is detected, providing treatment for TBI to the subject.

In some embodiments, the at least one RNA biomarker further comprises one or more miRNA selected from the group consisting of hsa-miR-143-3p and hsa-miR-135b-5p.

In another aspect of the present disclosure, there is provided a method of detecting an RNA biomarker in a saliva sample, the method comprising obtaining a saliva sample from a human subject, contacting the saliva sample with at least one oligonucleotide primer complementary to at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p; amplifying the at least one RNA biomarker using a polymerase chain reaction; and detecting the amplified RNA biomarker.

In some embodiments, the at least one RNA biomarker further comprises one or more miRNA selected from the group consisting of hsa-miR-143-3p and hsa-miR-135b-5p.

In another aspect of the present disclosure, there is provided a kit for use in a method of diagnosing and/or monitoring traumatic brain injury (TBI) in saliva from a human subject, the kit comprising at least one probe specific for at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p.

In some embodiments, the probe comprises a nucleic acid able to bind to the at least one RNA biomarker.

In some embodiments, the probe comprises a nucleic acid having at least 70% identity with a sequence which is the complement of the sequence of the target RNA biomarker.

In some embodiments, the probe comprises a nucleic acid having at least 70% identity with a sequence which is the complement of SEQ ID NO: 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, and 105.

In some embodiments, the at least one RNA biomarker further comprises one or more miRNA selected from the group consisting of hsa-miR-143-3p and hsa-miR-135b-5p.

In another aspect of the present disclosure, there is provided a composition for use in a method of diagnosing and/or monitoring traumatic brain injury (TBI) in saliva from a human subject, the composition comprising at least one probe specific for at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, put-miR-1352, put-miR-1080, put-miR-750, put-miR-1003, put-miR-1098, hsa-miR-934, and hsa-miR-142-5p.

In some embodiments, the probe comprises a nucleic acid able to bind to the at least one RNA biomarker.

In some embodiments, the probe comprises a nucleic acid having at least 70% identity with a sequence which is the complement of the sequence of the target RNA biomarker.

In some embodiments, the probe comprises a nucleic acid having at least 70% identity with a sequence which is the complement of SEQ ID NO: 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, and 105.

In some embodiments, the at least one RNA biomarker further comprises one or more miRNA selected from the group consisting of hsa-miR-143-3p and hsa-miR-135b-5p.

It will be appreciated that statements made herein in relation to any aspect of the invention may equally apply to any other aspect of the invention, as appropriate. Furthermore, the various aspects and embodiments of the present disclosure can also be used in combination with various techniques, methods, systems, compositions, devices, and/or disclosures as described in WO2017/153710A1 and WO2018/138468A1, the entire disclosures of which are hereby incorporated by reference herein.

### Further Additional Aspects and Embodiments

In further aspects, the present disclosure provides, *inter alia,* methods, sensor elements, detection systems, kits, and compositions involving various differentially expressed RNA biomarkers suitable for use in diagnosing, monitoring, and treating traumatic brain injury (TB), including mild traumatic brain injury (mTBI), in a human subject.

As shown in **Example 1** (below), in certain embodiments, the suitable RNA biomarkers were identified according to an MiRNA qPCR validation study performed in 176 saliva baseline samples (B); 42 samples form concussed players after injury (group Ca); 52 samples from concussed players post-match (group Cb) and 54 concussed players after 36-48h (group Cc); 61 uninjured players post-match (group Ub); 45 uninjured players 36-48h (group Uc); 30 musculoskeletal injury players post-match (group Mb); 24 musculoskeletal injury players 36-48h (group Mc).

The sequences and accession numbers for these RNA biomarkers are provided in **Table 2** below:

**Table 2**

| **RNA Biomarker** | **Sequence** | **miRBase Accession No.** | **SEQ ID NO.** |
|---|---|---|---|
| hsa-let-7a-5p | ugagguaguagguuguaguauaguu | MIMAT0000062 | 106 |
| hsa-let-7b-5p | ugagguaguagguugugugguu | MIMAT0000063 | 107 |
| hsa-let-7f-5p | ugagguaguagauuguauaguu | MIMAT0000067 | 108 |
| ***hsa-let-7i-5p*** | ugagguaguaguuugugcuguu | MIMAT0000415 | 29 |
| hsa-miR-103a-3p | agcagcauuguacagggcuauga | MIMAT0000101 | 109 |
| ***hsa-miR-107*** | agcagcauuguacagggcuauca | MIMAT0000104 | 39 |
| hsa-miR-1246 | aauggauuuuuggagcagg | MIMAT0005898 | 110 |
| ***hsa-miR-126-3p (miR-126*)*** | ucguaccgugaguaauaaugcg | MIMAT0000445 | 111 |
| ***hsa-miR-126-5p (=miR-126*)*** | cauuauuacuuuugguacgcg | MIMAT0000444 | 14 |
| ***hsa-miR-135b-5p*** | uaugcuuuucauuccuauguga | MIMAT0000758 | 40 |
| ***hsa-miR-142-3p*** | uguaguguuuccuacuuuaugga | MIMAT0000434 | 26 |
| hsa-miR-142-5p | cauaaaguagaaagcacuacu | MIMAT0000433 | 112 |
| ***hsa-miR-143-3p (=miR-143)*** | ugagaugaagcacuguagcuc | MIMAT0000435 | 48 |
| ***hsa-miR-144-3p (miR-144*)*** | uacaguauagaugauguacu | MIMAT0000436 | 113 |
| ***hsa-miR-144-5p (=miR-144*)*** | ggauaucaucauauacuguaag | MIMAT0004600 | 16 |
| ***hsa-miR-148a-3p*** | ucagugcacuacagaacuuugu | MIMAT0000243 | 27 |
| hsa-miR-16-1-3p | ccaguauuaacugugcugcuga | MIMAT0004489 | 114 |
| hsa-miR-206 | uggaauguaaggaagugugugg | MIMAT0000462 | 115 |
| hsa-miR-21-5p | uagcuuaucagacugauguuga | MIMAT0000076 | 1 |
| ***hsa-miR-29c-3p*** | uagcaccauuugaaaucgguua | MIMAT0000681 | 116 |
| hsa-miR-339-5p | ucccuguccuccaggagcucacg | MIMAT0000764 | 117 |
| hsa-miR-34b-3p | caaucacuaacuccacugccau | MIMAT0004676 | 118 |
| ***hsa-miR-425-5p*** | aaugacaceaucacucccguuga | MIMAT0003393 | 2 |
| hsa-miR-449a | ugacaguguauuguuagcuggu | MIMAT0001541 | 119 |
| hsa-miR-497-5p | cagcagcacacugugguuugu | MIMAT0002820 | 120 |
| ***hsa-miR-671-3p*** | uccggguucucagggcuccacc | MIMAT0004819 | 23 |
| hsa-miR-6748-3p | uccugucccugucuccuacag | MIMAT0027397 | 121 |
| hsa-miR-92a-3p | uauugcacuugucccggccugu | MIMAT0000092 | 122 |
| hsa-miR-934 | ugucuacuacuggagacacugg | MIMAT0004977 | 123 |
| put-miR-1003 | tgaacttcaaaacage | | 89 |
| put-miR-1080 | tgtggattgatgctct | | 87 |
| put-miR-1084 | aggaccattacgttgcc | | 80 |
| put-miR-1146 | caactgtaagtccatt | | 84 |
| put-miR-1204 | ageactgggcacggggg | | 124 |
| put-miR-1207 | gtgaaaagacataggggg | | 79 |
| put-miR-1306 | ataacgtcatctagtgtg | | 71 |
| put-miR-188 | aaatggcgatactcagg | | 82 |
| put-miR-209 | ggtcctcggatcggcc | | 85 |
| put-miR-323 | ataaaatgagcgttgagg | | 125 |
| put-miR-325 | ttcaaatcccacttetgacacca | | 126 |
| put-miR-444 | ctgaaaaggggacggattgggaa | | 127 |
| put-miR-465 | ccagttgtcgtgggttttt | | 128 |
| put-miR-468 | ataaggaactgctctctc | | 74 |
| put-miR-469 | gcgggggattagctcagctggg | | 129 |
| put-miR-476 | gtggtcaggtagagaa | | 75 |
| put-miR-594 | tgattttttttggcgaa | | 130 |
| put-miR-6 | gcggaccttgctcaagg | | 78 |
| put-miR-71 | gaccttgggttgggtcgtggt | | 73 |
| put-miR-742 | cagggctgtgctaact | | 77 |
| put-miR-806 | cacgaragaacgcacacc | | 72 |
| put-miR-856 | aaggatagggaggtattt | | 131 |
| put-miR-893 | accatcctctgctacca | | 132 |
| put-miR-92 | agagactgactttgagta | | 81 |
| put-miR-958 | acagggctgtgcaaaaa | | 133 |
| put-miR-961 | tagcttgatccagttg | | 86 |
| RNU4-6p | tatcgtagccaatgaggtttatccgag gcgtgattattgctaattgaaaa | | 134 |
| RNU6-4 | ggcccctgcacagggatgacacgca aattcgtgaagcgttccatattttt | | 98 |
| RNU6-45 | ggcccttgtgcaaggatgacacgcaa attcgtgaagcgttccatattttt | | 101 |
| RNU6-6 | ggcccctgtgcaaggatgacacgca aattcgtgaagcgttccatattttt | | 100 |
| RNU6-7 | ggcccctgcgcaaggatgacatgca aattcgtgaagcgttccatattttt | | 99 |
| RNU6-73 | ggcccctgtgcaaggatgacatgcaa attcgtgaagcgttccatattttt | | 102 |
| SNORA57 | tgctggcggcttcccatccgctggttct atcctcaaacgccgggacaccg | | 135 |
| SNORD3B-2 | cttctctccgtattggggagtgagagg gagagaacgcggtctgagtggtt | | 136 |
| tRNA120-AlaAGC | gcgcatgcttagcatgcatgaggtccc gggttcgatccccagcatctcca | | 137 |
| tRNA18-ArgCCT | gcactggcctcctaagccagggattgt gggttcgagtcccacctggggta | | 138 |
| tRNA27-MetCAT | gcgtcagtctcataatctgaaggtcct gagttcgagcctcagagagggca | | 139 |
| tRNA2-LeuTAA | gcataaaacttaaaattttataatcaga ggttcaactcctcttcttaaca | | 140 |
| tRNA73-ArgCCG | gcgtctgattccggatcagaagattga gggttcgagtcccttcgtggtcg | | 141 |
| tRNA8-ThrAGT | gcgcctgtctagtaaacaggagatcct gggttcgaatcccagcggtgcct | | 142 |
| tRNA9-TyrGTA | ctttttgactgtagagcaagaggtccct ggttcaaatccaggttctccct | | 143 |
| U2.3 | tcacttcacgcatcgatctggtattgca gtacctccaggaacagtgcacc | | 144 |
| U4.64 | gtatcgtagccaatgaggtttatccaa ggtgcgattattgctaattgaaa | | 145 |
| U6.1249 | gatggcatgacccctgatcaaggacg gcatgcaaatttgtgaagtatttc | | 105 |
| U6.168 | atggcccctgcgcaaggatgacacg caaatttgtgaaggattccatattt | | 146 |
| U6.375 | ggcccctgtgcaagaatgactcgcaa attcgtgaagcgttccatattttt | | 104 |
| U6.428 | aagattagcatgaggatgacacgcaa attcgtgaagcgttccatttcttt | | 97 |
| U6.601 | ggcccctgcgcaaggatgacatgca aatttgtgaagtgttccatattttt | | 147 |
| UC022CJG1 | cattgatcatcgacacttcgaacgcac ttg | | 148 |
| YRNA-245 | gtctttgttgaactctttccctccttctcat tactgtacttgaccagtct | | 149 |
| YRNA-255 | gugucaccaacguugguauacaac cccccacaacuaaauuugacuggc uu | | 103 |
| YRNA-684 | gcttcttttactctttcccttcattctcact actgtacctgattcgtctt | | 150 |

As discussed in more detail in **Example 1** (below), these biomarkers were found to be differentially expressed and statistically significant between the different group comparisons and the different time points (Ca vs Ub; Cb vs Ub; Cc vs Uc; Ca vs Mb; Cb vs Mb; Cc vs Mc; Ca vs U+M b; Cb vs U+M b; Cc vs U+M c; Ca vs B; Cb vs B; Cc vs B).

According to an aspect of the present disclosure, there is provided a method of diagnosing and treating traumatic brain injury (TBI) in a human subject in need thereof, the method comprising: obtaining a saliva sample from the subject; contacting the saliva sample with a probe comprising a nucleic acid able to bind to at least one RNA biomarker selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684; determining an amount of the at least one RNA biomarker in the saliva sample; identifying the subject as having TBI where the amount of the at least one RNA biomarker is increased or decreased relative to a predetermined threshold value or relative to the amount of the RNA biomarker in a control sample; and treating the subject identified as having TBI according to one or more of the following: administering to the subject one or more neuroprotective therapies.

Examples of neuroprotective therapies that may be chosen by a clinician for treating TBI can include, without limitation, statins, progesterone, corticosteroids, cell cycle inhibitors (such as flavopiridol, a semi-synthetic flavonoid, and purine analogs roscovitine and olomoucine), autophagy inhibitors including caspase inhibitors (such as the tetrapeptide caspase-3 inhibitor (z-DEVD-fmk), the pan-caspase peptide inhibitor Boc-aspartyl fluoromethylketone, and Boc-aspartyl fluoromethylketone), inhibitors of poly (ADP-ribose) polymerase (PARP), anti-inflammatory agents (such as minocycline and anti-inflammatory cytokines (e.g., IL-10) or interleukin-1 receptor antagonist (IL-1ra)), sulfonylurea receptor 1 (SUR1)-regulated calcium channel inhibitors (such as glibenclamide), Substance P (SP) antagonists, diketopiperazines, and cyclosporine A.

In some embodiments, the method further comprises identifying the human subject as being fit for normal activity after undergoing successful treatment for TBI.

In another aspect of the present disclosure, there is provded a method of diagnosing and/or monitoring traumatic brain injury (TBI) in a subject, the method comprising determining a level of at least one RNA biomarker in a saliva sample obtained from the subject, wherein the at least one RNA biomarker is selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG SNORD3B-2, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684, or any combination thereof.

In some embodiments, either an upregulated level or a downregulated level of the at least one RNA biomarker is indicative of TBI.

In some embodiments, the subject is diagnosed as having TBI if the level of the at least one RNA biomarker is either above or below a predetermined threshold or increased or decreased relative to a control.

In some embodiments, the method further comprises identifying the human subject as being fit for normal activity after undergoing successful treatment for TBI.

In some embodiments, the at least one RNA biomarker further comprises one or more miRNA selected from the group consisting of hsa-miR-143-3p and hsa-miR-135b-5p.

In another aspect of the present disclosure, there is provided a sensor element for a detection system for diagnosing and/or monitoring TBI, the sensor element comprising a substrate functionalized with a probe specific for at least one RNA biomarker selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684.

In some embodiments, the probe comprises a nucleic acid able to bind to the at least one RNA biomarker.

In some embodiments, the probe comprises a nucleic acid having at least 70% identity with a sequence which is the complement of the sequence of the target RNA biomarker.

In some embodiments, the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.

In some embodiments, the at least one RNA biomarker further comprises one or more miRNA selected from the group consisting of hsa-miR-143-3p and hsa-miR-135b-5p.

In another aspect of the present disclosure, there is provided a detection system for diagnosing and/or monitoring TBI, comprising a sensor element according to the present disclosure, and a detection device capable of detecting the binding of a target RNA biomarker to the probe.

In some embodiments, the detection system further comprises means to determine whether the target RNA biomarker is upregulated or downregulated.

In another aspect of the present disclosure, there is provided a method for determining a course of action for a subject suspected of having TBI, comprising applying a saliva sample obtained from the subject to a detection system according to the present disclosure, and if an upregulated level or a downregulated level of the at least one RNA biomarker is detected, providing a treatment for TBI.

In another aspect of the present disclosure, there is provided a method of treating a subject with suspected TBI, the method comprising determining whether an upregulated level or a downregulated level of at least one RNA biomarker selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684 is detectable in a saliva sample obtained from the subject, and if an upregulated level or a downregulated level of at least one RNA biomarker is detected, providing treatment for TBI to the subject.

In some embodiments, the at least one RNA biomarker further comprises one or more miRNA selected from the group consisting of hsa-miR-143-3p and hsa-miR-135b-5p.

In another aspect of the present disclosure, there is provided a method of detecting an RNA biomarker in a saliva sample, the method comprising obtaining a saliva sample from a human subject, contacting the saliva sample with at least one oligonucleotide primer complementary to at least one RNA biomarker selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684; amplifying the at least one RNA biomarker using a polymerase chain reaction; and detecting the amplified RNA biomarker.

In some embodiments, the at least one RNA biomarker further comprises one or more miRNA selected from the group consisting of hsa-miR-143-3p and hsa-miR-135b-5p.

In another aspect of the present disclosure, there is provided a kit for use in a method of diagnosing and/or monitoring traumatic brain injury (TBI) in saliva from a human subject, the kit comprising at least one probe specific for at least one RNA biomarker selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684.

In some embodiments, the probe comprises a nucleic acid able to bind to the at least one RNA biomarker.

In some embodiments, the probe comprises a nucleic acid having at least 70% identity with a sequence which is the complement of the sequence of the target RNA biomarker.

In some embodiments, the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.

In some embodiments, the at least one RNA biomarker further comprises one or more miRNA selected from the group consisting of hsa-miR-143-3p and hsa-miR-135b-5p.

In another aspect of the present disclosure, there is provided a composition for use in a method of diagnosing and/or monitoring traumatic brain injury (TBI) in saliva from a human subject, the composition comprising at least one probe specific for at least one RNA biomarker selected from the group consisting of hsa-let-7a-5p, hsa-let-7b-5p, hsa-let-7f-5p, hsa-let-7i-5p, hsa-miR-103a-3p, hsa-miR-107, hsa-miR-1246, hsa-miR-126-3p (miR-126*), hsa-miR-126-5p (=miR-126*), hsa-miR-135b-5p, hsa-miR-142-3p, hsa-miR-142-5p, hsa-miR-143-3p (=miR-143), hsa-miR-144-3p (miR-144*), hsa-miR-144-5p (=miR-144*), hsa-miR-148a-3p, hsa-miR-16-1-3p, hsa-miR-206, hsa-miR-21-5p, hsa-miR-29c-3p, hsa-miR-339-5p, hsa-miR-34b-3p, hsa-miR-425-5p, hsa-miR-449a, hsa-miR-497-5p, hsa-miR-671-3p, hsa-miR-6748-3p, hsa-miR-92a-3p, hsa-miR-934, put-miR-1003, put-miR-1080, put-miR-1084, put-miR-1146 (2), put-miR-1204, put-miR-1207, put-miR-1306, put-miR-188, put-miR-209, put-miR-323, put-miR-325, put-miR-444, put-miR-465, put-miR-468, put-miR-469, put-miR-476, put-miR-594, put-miR-6, put-miR-71, put-miR-742, put-miR-806, put-miR-856, put-miR-893, put-miR-92, put-miR-958, put-miR-961, RNU4-6p, RNU6-4, RNU6-45, RNU6-6, RNU6-7, RNU6-73, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.1249, U6.168, U6.375, U6.428, U6.601, UC022CJG1, YRNA-245, YRNA-255, and YRNA-684.

In some embodiments, the probe comprises a nucleic acid able to bind to the at least one RNA biomarker.

In some embodiments, the probe comprises a nucleic acid having at least 70% identity with a sequence which is the complement of the sequence of the target RNA biomarker.

In some embodiments, the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.

In some embodiments, the at least one RNA biomarker further comprises one or more miRNA selected from the group consisting of hsa-miR-143-3p and hsa-miR-135b-5p.

It will be appreciated that statements made herein in relation to any aspect of the invention may equally apply to any other aspect of the invention, as appropriate.

### EXAMPLES

Embodiments of the invention will now be described by way of example.

### Example 1

### Salivary Non-Coding RNA:

### Next Generation Biomarkers in Concussed Rugby Football Union Players

### Study design

The Study of Concussion in Rugby Union through MicroRNAs (SCRUM study) is a prospective observational study of players (up to 1100) in the two highest tiers of senior professional domestic rugby competition in England, the Premiership and Championship. The study is embedded within the ongoing RFU Injury Surveillance Programme for the 2017-2018 seasons.

All players, previous written consent, were asked to provide a single baseline samples of approximately 2mL of saliva during the preseason preparations (group B), before the training sessions if possible, at their clubs as well as completing a consent form detailing the processing and handling of their samples, the anonymisation of their data and their right to withdraw their voluntary consent at any time. The investigators recorded the timing of these baseline extractions in relation to preseason training sessions, other injuries, or supplement/medication use.

During the season each time a player entered the Head Injury Assessment (HIA) process (group C), he was asked to provide a saliva sample. Players who had been removed during the match for a pitch side assessment, provided a sample during this medical evaluation, called HIA1, and constituted group Ca. If players exhibited clear signs of concussion, also known as category 1 signs, no pitch side evaluation was necessary - in accordance with World Rugby guidance - and the player was immediately and permanently removed from the match. These players formed the "Immediate Permanent Removal" group (IPR). All players evaluated pitch-side or immediately and permanently removed for concussion were subsequently assessed immediately after the match (HIA2) and at 36-48h post-match (HIA3). Samples were collected at each of these assessments. Therefore, we obtained saliva samples for groups C and IPR immediately post-match (group Cb and IPRb respectively) and 36h-48h HIA (group Cc and IPRc respectively).

The HIAs at each time point take the form of a modified Sport Concussion Assessment Tool 5th Edition (SCATS) which has been well studied as a diagnostic tool in the context of sports-related concussion and is complemented by baseline performance data in these assessments collected by the clubs during their preseason preparations. In this way, we investigated the presence of concussion diagnostic miRNAs noninvasively at multiple time points, which were matched to a standardised clinical assessment and diagnostic threshold for concussion performed by a clinician with experience in rugby head injuries. All IPR players were deemed to have been concussed. Group C players were deemed to be concussed if they failed the HIA at any time point (HIA1, HIA2 or HIA3); otherwise they were cleared of concussion. Players who entered the HIA process for suspected concussion but were cleared were analysed separately.

Players diagnosed with concussion were also asked for a sample of saliva at the point at which they were cleared by their team physician to return to competitive play (Stage VI) (group Cd and IPRd), as per the Berlin Consensus Graduated Return-to-Play (GRTP) protocol.

Whenever a player was removed for an HIA, club medical teams also approached an "uninjured control' (group U) player for saliva samples after the game (group Ub) and at 36-48 hours later (group Uc). An "uninjured control" is a non-concussed player who has played approximately a matched number of minutes in the same game and same playing position of the concussed player whenever possible. This allows for the description of miRNA profiles in response to the physical stress of athletic activity and exposure to the likely subconcussive blows, which come from participation in a collision sport.

To rule out contamination from non-neurological injuries and musculoskeletal trauma, club medics collected samples from players removed from the field of play due to musculoskeletal injuries (orthopaedic controls) (group M) at the same time points as the "uninjured control" group (group Mb and group Mc).

### Ethics and dissemination

The SCRUM study runs under University of Birmingham (UoB) ethics (ERN _11-0429AP28) as part of the Repetitive Concussion in Sport (RECOS) study. This study was approved by the East of England - Essex Research Ethics Committee (REC) on 22 September 2017 - REC 17/EE/0275; IRAS 216703.

### Saliva collection

The samples were collected in Oragene^{®}-RNA RE-100 saliva self-collection kit (DNA Genotek) which contains a RNA stabilizing solution for a total of 8 weeks. Saliva was collected from each participant at enrollment following orally rinsing with tap water. The samples were then transported to the University of Birmingham where they were processed in line with the sample pot manufacturer's guidance to allow freezing and storage.

### Materials and Methods

### Next Generation Sequencing- discovery phase

All experiments were conducted at QIAGEN Genomic Services, Germany.

### RNA preparation

RNA preparation was performed according to Oragene recommendations, except the use of the miRNeasy kit (Qiagen, Germany) instead of the RNeasy kit.

### Next Generation Sequencing

### Library preparation and sequencing

The library preparation was done using the QIAseq miRNA Library Kit (QIAGEN). A total of 5ul total RNA was converted into microRNA NGS libraries. Adapters containing UMIs were ligated to the RNA. Then RNA was converted to cDNA. The cDNA as amplified using PCR (22 cycles) and during the PCR indices were added. After PCR the samples were purified. Library preparation QC was performed using either Bioanalyzer 2100 (Agilent) or TapeStation4200 (Agilent). Based on quality of the inserts and the concentration measurements the libraries were pooled in equimolar ratios. The library pools were quantified using the qPCR ExiSEQ LNA^{™} Quant kit (Exiqon). The library pools were then sequenced on a NextSeq500 sequencing instrument according to the manufacturer instructions (NEBNext Multiplex Small RNA Library Prep Set for Illumina) to make approximately 163-175 base-pair sized libraries. Raw data as demultiplexed and FASTQ files for each sample were generated using the bcl2fastq software (Illumina inc.). FASTQ data were checked using the FastQC tool (www.bioinformatics.babraham.ac.uk/proeects/fastqc/).

### Trimming

Cutadapt was used to extract information of adapter and UMI in raw reads, and output from cutadapt was used to remove adapter sequences and to collapse reads by UMI with inhouse script.

### UMI correction

According to the experiment protocol, each raw read was expected to contain, starting from 5'end, an insert sequence, the adapter sequence, 12nt-long UMI sequence, and other ligated sequence. Depending on the read length and insert length, not all parts were present in all reads. To correct PCR bias with UMI information, raw reads were processed as described below:
1. Use cutadapt on raw reads with provided adapter sequence to acquire output with information about the presence of adapter for each read.
2. Parse cutadapt output and keep only reads that fulfil all of the following requirements:
   - Reads contain adapters.
   - Insert sequence should be equal or larger than minimal insert length (default 16nt).
   - UMI sequence should be equal or longer than minimal UMI length (default 10nt).
3. Extract insert sequences from reads which do not contain full length UMI sequence from step 2 output as partial-UMI reads.
4. Examine reads with full length UMI in step 2 output and identify all unique insert+UMI combinations. Extract insert sequences from unique insert + UMI combinations as full-UMI reads.
5. Combine partial-UMI reads and full-UMI reads as output of UMI correction.

### Mapping

A reference profile of sequencing data for each sample was obtained using the whole human genome sequence GRCh37, downloaded from the Genome Reference Consortium and mirbase_20 as annotation reference. Reads were aligned to the miRbase using Bowtie2. The mapping criteria for aligning reads to spike-ins, abundant sequence and miRBase were the reads have to have perfect match to the reference sequences. For mapping to the genome the restriction of one mismatch was allowed in the first 32 bases of the read. No indels were allowed in mapping. Unaligned reads were mapped against the host reference genome and used as input for mirPara and miRbase to predict putative miRNAs.

### Differential Expression

Aligned reads were counted and differential expression analysis, p-values for significantly differentially expressed microRNAs and false discovery rate according to Benjamini-Hochberg were performed with EdgeR. For normalisation, the trimmed mean of M-values (TMM) method based on log-fold and absolute gene-wise changes in expression levels between samples was used.

### Unsupervised analysis

Principal component analysis is performed with R using TMM-normalized quantifications from defined collections of samples as input.

### MiRNA qPCR Data analysis - validation study

All experiments were conducted at QIAGEN Genomic Services, Germany.

MiRNA qPCR validation was performed in 176 saliva baseline samples (B); 42 samples form concussed players after injury (group Ca); 52 samples from concussed players post-match (group Cb) and 54 concussed players after 36-48h (group Cc); 61 uninjured players post-match (group Ub); 45 uninjured players 36-48h (group Uc); 30 musculoskeletal injury players post-match (group Mb); 24 musculoskeletal injury players 36-48h (group Mc).

### RNA extraction and qPCR

Total RNA was extracted from the samples using miRNeasy Serum/Plasma kit (QIAGEN) after manufacturer's instructions. The purified total RNA was eluted in a final volume of 32 µl. 14 µl RNA was reverse transcribed in 70 µl reactions using the miRCURY LNA RT Kit (QIAGEN). cDNA was diluted 50 x and assayed in 10 µl PCR reactions according to the protocol for miRCURY LNA miRNA PCR; each miRNA was assayed once by qPCR on the miRNA Ready-to-Use PCR, Custom panel using miRCURY LNA SYBR Green master mix. Negative controls excluding template from the reverse transcription reaction was performed and profiled like the samples. The amplification was performed in a LightCyclerp 480 Real-Time PCR System (Roche) in 384 well plates. The amplification curves were analyzed using the Roche LC software, both for determination of Cq (by the 2nd derivative method) and for melting curve analysis. The amplification efficiency was calculated using algorithms similar to the LinReg software. All assays were inspected for distinct melting curves and the Tm was checked to be within known specifications for the assay. Furthermore assays must be detected with 0 Cq less than the negative control, and with Cq<37 to be included in the data analysis. Data that did not pass these criteria were omitted from any further analysis. Cq was calculated as the 2nd derivative. Normalization was performed based on the average of hsa-miR-29c-3p and hsa-let-7b-5p (custom normalizer assays), the two more stable miRs identified across all samples by Normofinder software (33). The formula used to calculate the normalized Cq values is the difference between the custom normalizer assays mean Cq and the assay Cq (miRNA of interest). A higher value indicates that the miRNA is more abundant in that sample.

### Statistical Analyses

All analyses were carried out using SPSS 20.0 (SPSS Inc., Chicago, IL, USA).

### Group comparisons

We used the two-tailed Independent-Samples T Test procedure to compare means for two groups of cases. The two-tailed Paired-Samples T Test procedure compares the means of two variables for a single group. The procedure computes the differences between values of the two variables for each case and tests whether the average differs from 0. Bootstrapping is a method for deriving robust estimates of standard errors and confidence intervals for estimates. Bootstrapping is most useful as an alternative to parametric estimates when the parametric inference is impossible.

The One-Way ANOVA procedure produces a one-way analysis of variance for a quantitative dependent variable by a single factor (independent) variable. Analysis of variance is used to test the hypothesis that several means are equal. This technique is an extension of the two-sample t test. In addition to determining that differences exist among the means, you may want to know which means differ. There are two types of tests for comparing means: a priori contrasts and post hoc tests. Contrasts are tests set up *before* running the experiment, and post hoc tests are run *after* the experiment has been conducted.

General Linear Model Repeated Measures analyzes groups of related dependent variables that represent different measurements of the same attribute. It is possible define one or more within-subjects factors for use in GLM Repeated Measures.

### Discriminant Analysis

The Discriminant analysis (DA) was applied to the Cq values provided by Qiagen.

The main purpose of DA was to find the dimension(s) along which groups differ and to find classification function(s) to predict group membership from a combination of variables (predictors). In the present case, the two groups were concussed (group C+IPR) and uninjured subjects (group U), and the predictors were a set of biomarkers selected from the NGS study.

DA works with data that is already classified into groups to derive rules for classifying new (and as yet unclassified) individuals on the basis of their observed variable values.

In univariate terms (t-test, Oneway Anova) a significant difference among groups implies that, given a score, you can predict which group it comes from. In DA, there is often an effort to interpret the pattern of differences among the predictors as a whole in an attempt to understand the dimensions along which groups differ reliably.

With only two groups, there is only one linear combination of predictors that best separates them. The separation of two group *centroids* (multivariate version of means), represents the best linear combination of predictors that separate the groups 1 and 2. A line parallel that connects the two centroids represents the linear combination or *discriminant function*, a pattern of scores that discriminates between the two groups.

There are two facets of DA, and one or both may be emphasized in any given research application. The researcher may be interested in a decision rule for classifying cases where the number of dimensions and their meaning is irrelevant. Or the emphasis may be on interpreting the results of DA in terms of the combinations of predictors (*discriminant functions*) that separate various groups from each other.

Classification functions are used to predict group membership for new cases and to check the adequacy of classification for cases in the same sample through cross-validation.

The canonical correlation explains the percent of variance accounted by the scores for the discriminant function between these groups. A canonical correlation is a multiple-multiple correlation because there are multiple variables on both sides of the regression equation, and for each discriminant function, when squared, indicates the proportion of variance shared between groups and predictors on that function.

In **Standard (direct)** DA, like standard multiple regression, all predictors enter the equations at once and each predictor is assigned only the unique association it has with groups. Variance shared among predictors contributes to the total relationship, but not to any one predictor.

In **Stepwise** DA statistical criteria can be used to produce a reduced set of predictors.

All analyses were carried out using SPSS 20.0 (SPSS Inc., Chicago, IL, USA).

### ROC curve analyses

The diagnostic performance of a test, or the accuracy of a test to discriminate experimental cases from normal cases is evaluated using Receiver Operating Characteristic (ROC) curve analysis. ROC curves can also be used to compare the diagnostic performance of two diagnostic tests.

When one considers the results of a particular test in two populations, one population with a characteristic and the other population without the characteristic, you will rarely observe a perfect separation between the two groups. Indeed, the distribution of the test results will overlap.

In a ROC curve the true positive rate (Sensitivity) is plotted in function of the false positive rate (100-Specificity) for different cut-off points of a parameter. Each point (cut-off) on the ROC curve represents a sensitivity/specificity pair corresponding to a particular decision threshold. The area under the ROC curve (AUC) is a measure of how well a parameter can distinguish between two diagnostic groups (experimental/normal). A test with perfect discrimination (no overlap in the two distributions) has a ROC curve that passes through the upper left corner (100% sensitivity, 100% specificity). Therefore the closer the ROC curve is to the upper left corner, the higher the overall accuracy of the test.

### ROC statistics

*Sensitivity*: probability that a test result will be positive when the characteristic is present (true positive rate, expressed as a percentage).

*Specificity*: probability that a test result will be negative when the characteristic is not present (true negative rate, expressed as a percentage).

*Positive likelihood ratio*: ratio between the probability of a positive test result given the presence of the characteristic and the probability of a positive test result given the absence of the characteristic, i.e. = True positive rate / False positive rate = Sensitivity / (1-Specificity)

*Negative likelihood ratio*: ratio between the probability of a negative test result given the presence of the characteristic and the probability of a negative test result given the absence of the characteristic, i.e. = False negative rate / True negative rate = (1-Sensitivity) / Specificity

### Results

### Sample size

A total of 906 players were recruited to the study between July 2017 and April 2018. This included players from 11 clubs playing in The Premiership and 11 clubs playing in the IPA Championship. One club in The Premiership chose not to take part after consultation with the RFU as they were already participating in a concussion research project involving saliva sampling for different purposes run by another organisation. One club in the IPA Championship chose not to take part as they felt they would struggle to fulfil the requirements of the study due to limited numbers of medical staff while another club in the IPA Championship felt decided to withdraw from the study for similar reasons but after their players had provided baseline samples. This made a total of 11 clubs in The Premiership and 10 clubs in the IPA Championship participating in collecting samples through the season from head injury events.

Overall, a total of 229 head injury incidents took place across both competitions. This figure is an amalgamation of incidents in which the player was immediately and permanently removed from play due to exhibiting "Category 1" signs and symptoms, as well as all of the incidents in which players were temporarily removed from play for an off field HIA1 assessment. Samples were received from a total of 129 of these incidents making overall compliance 56%.

Samples were received from 73 of a possible 120 incidents in which the on field or subsequent diagnosis was concussion (61%). Within this group 26 (36%) were incidents in which the player was immediately and permanently removed from play due to exhibiting "Category 1" signs and symptoms. Samples were received from 42 of a possible 109 incidents in which the on field or subsequent diagnosis excluded concussion (39%).

Matched uninjured control samples were received in 71 cases (55%). Matched musculoskeletal injury control samples were received in 39 cases (30%).

### Next generation sequencing - discovery phase

MicroRNA/small RNA Next Generation Sequencing (NGS) was performed in the initial discovery phase using 15 saliva baseline samples (B), 15 samples from concussed players (consisting of 10 concussed (C) and 5 IPR) and 20 controls (consisting of 10 musculoskeletal injuries (M) and 10 uninjured players (U)). All samples analysed in this phase were collected post-match (time point b).

On average 16.3 million reads were obtained per sample. A detailed microRNA/small RNA NGS data analysis report was obtained. The differential expression analysis step attempts to distinguish biological variation from technical variation within the experiment, assuming that this varies amongst microRNAs.

P-values for significantly differentially expressed microRNAs are estimated by an exact test on the negative binomial distribution. A list of microRNAs predicted to be differentially expressed between the given experimental conditions was created. This list includes different RNA fragments, among these known microRNA, other small non-coding RNAs, and predicted microRNA (described as put-miR) differentially expressed between groups C+IP and M+U, B and C+IPR, and B and M+U.

### Selection for validation

A panel of microRNA/small RNA/put microRNA, differetially expressed in NGS analysis was selected in the group C+IPR and M+U according the following paramenters: FDR<0.5 or p-value <0.05. The Panel consisting of 168 small RNAs, 38 known microRNAs and 233 put-miRs was used for the validation study by qPCR. Following this test, the most significantly altered biomarkers were reduced to 30 known microRNAs; 34 putative miRs; 28 small non-codingRNA, which were then analysed in a further 405 samples. The Cq values obtained from the 2 qPCR validation sets were finally merged and collected in a total of 598 samples.

### Statistical analysis - validation study

### SncRNAs differentially expressed in group C vs U+M

Concussion samples were compared to uninjured and musculoskeletal injury at different time points. **Table 5** shows the sncRNAs survived to a p value <0.05 among the different comparisons: Ca vs U+M b (no samples were collected at time point a for the category U and M, for this reason the group Ca was compared to the closest time point, U+M b), Cb vs U+M b; Cc vs U+M c. The table also includes: AUC, CI, count, CT average, SD, ddcq, fold change and power analysis for each individual biomarker.

The biomarkers selected from the STEPWISE analysis are shown in grey cells of **Table 5** and presented an AUC of 0.86; 0.86 and 0.97 in Ca vs U+M b; Cb vs U+M b; Cc vs U+M c comparisons respectively.

### SncRNAs differentially expressed in group C vs U

Concussion samples were compared to uninjured control group only at different time points. **Table 6** shows the sncRNAs survived to a p value <0.05 among the different comparisons: Ca vs Ub; Cb vs Ub; Cc vs Uc. The table also includes: AUC, CI, count, CT average, SD, ddcq, fold change and power analysis for each individual biomarker. The biomarkers selected from the STEPWISE analysis are shown in grey cells of **Table 6** and presented an AUC of 0.83; 0.91 and 0.97 in the following comparisons Ca vs Ub; Cb vs Ub; Cc vs Uc respectively.

### SncRNAs differentially expressed in group C vs B

Concussion samples were compared to the baseline data of the same subjects. **Table 7** shows the sncRNAs survived to a p value <0.05 in the different comparisons: Ca vs B Cb vs B; Cc vs B. The table also includes: count, CT average, SD, ddcq, fold change and power analysis for each individual biomarker.

### SncRNAs differentially expressed in group C vs M

Concussion samples were compared to musculoskeletal injury control group only at different time points. **Table 8** shows the sncRNAs survived to a p value <0.05 among the different comparisons: Ca vs Mb; Cb vs Mb; Cc vs Mc. The table also includes: AUC, CI, count, CT average, SD, ddcq, fold change and power analysis for each individual biomarker. The biomarkers selected from the STEPWISE analysis are shown in grey cells of **Table 8** and presented an AUC 0.96; 0.83 and 0.94 in the following comparisons Ca vs Mb; Cb vs Mb; Cc vs Mc respectively.

MicroRNAs differentially expressed between Cb and U+M b groups (FDR<0.5 or p-value <0.05), selected by NGS analysis and used for the first qPCR validation step are shown in **Table 3A,** below.

**Table 3A**

| **microRNAs** | **logFC** | **logCPM** | **p-Value** | **FDR** |
|---|---|---|---|---|
| hsa-miR-199a-5p | -3.79380184 | 5.744164606 | 8.15823E-06 | 0.004797041 |
| hsa-miR-133a-3p | -4.95698696 | 10.40020121 | 7.04586E-05 | 0.020714819 |
| hsa-miR-206 | -4.6959152 | 9.788488822 | 0.000180043 | 0.03528849 |
| hsa-miR-1273h-3p | 2.629420889 | 3.481729832 | 0.001297451 | 0.190725263 |
| hsa-miR-133b | -4.20181151 | 6.465178373 | 0.002184888 | 0.256942851 |
| hsa-miR-561-5p | 1.579551555 | 4.525850808 | 0.003970504 | 0.348018465 |
| hsa-miR-126-3p | -1.41838668 | 8.449681001 | 0.004143077 | 0.348018465 |
| hsa-miR-16-1-3p | 1.43849327 | 4.458454478 | 0.005764959 | 0.352449414 |
| hsa-miR-449b-3p | 3.467958972 | 3.218975 | 0.005920497 | 0.352449414 |
| hsa-miR-449c-5p | 2.749952622 | 5.534179256 | 0.006033974 | 0.352449414 |
| hsa-miR-6748-3p | 4.143101791 | 3.188086662 | 0.007073212 | 0.352449414 |
| hsa-miR-133a-5p | -3.92797466 | 4.752927061 | 0.008472679 | 0.352449414 |
| hsa-miR-34b-3p | 2.1437498 | 4.339967792 | 0.008637715 | 0.352449414 |
| hsa-miR-6824-3p | 2.731145621 | 3.076936275 | 0.008970282 | 0.352449414 |
| hsa-miR-5195-5p | 5.376105018 | 4.09767258 | 0.009313959 | 0.352449414 |
| hsa-miR-5096 | 2.906908779 | 4.723794715 | 0.00959046 | 0.352449414 |
| hsa-miR-4488 | 3.491520052 | 3.097320079 | 0.012515423 | 0.409279535 |
| hsa-miR-92a-3p | -0.60902412 | 10.49283489 | 0.012885103 | 0.409279535 |
| hsa-miR-1 | -2.36876254 | 10.80951287 | 0.013692259 | 0.409279535 |
| hsa-miR-885-5p | 1.507019382 | 4.182851751 | 0.013921073 | 0.409279535 |
| hsa-miR-548h-5p | 2.055544755 | 3.480848725 | 0.015148132 | 0.42414769 |
| hsa-miR-1246 | 0.980484408 | 14.78271469 | 0.017085283 | 0.445746733 |
| hsa-miR-4492 | -3.18487947 | 3.53612341 | 0.017435672 | 0.445746733 |
| hsa-miR-484 | 1.274418725 | 6.148369199 | 0.019071294 | 0.449461809 |
| hsa-miR-449a | 2.203777576 | 6.338766334 | 0.019109771 | 0.449461809 |
| hsa-miR-3122 | 3.142476669 | 3.078975177 | 0.02000366 | 0.452390465 |
| hsa-miR-1180-3p | 1.165471248 | 3.889331639 | 0.021316281 | 0.457201951 |
| hsa-miR-619-5p | 2.792045377 | 3.207604869 | 0.021771521 | 0.457201951 |
| hsa-miR-2277-5p | 1.067026052 | 4.057818901 | 0.023836409 | 0.474930011 |
| hsa-miR-5699-3p | 1.876788488 | 2.955683933 | 0.024231123 | 0.474930011 |
| hsa-miR-125b-2-3p | -0.96587973 | 4.933520984 | 0.025679562 | 0.487083301 |
| hsa-miR-4449 | 2.06863176 | 3.047374886 | 0.027679506 | 0.503997815 |
| hsa-miR-671-3p | 1.125735449 | 3.934232773 | 0.02867908 | 0.503997815 |
| hsa-miR-33a-3p | 0.959071217 | 4.091785492 | 0.029142731 | 0.503997815 |
| hsa-miR-193b-5p | -1.14025756 | 4.138245693 | 0.031271072 | 0.522692794 |
| hsa-miR-34c-3p | 1.690242737 | 4.489423918 | 0.0320016 | 0.522692794 |
| hsa-miR-339-5p | 1.057419584 | 7.902671962 | 0.034354837 | 0.545963351 |
| hsa-miR-6813-3p | 2.813650185 | 2.933287004 | 0.035310016 | 0.546376034 |
| hsa-miR-671-5p | 0.838663749 | 5.600518309 | 0.047339286 | 0.598988904 |
| hsa-miR-1537-5p | 1.129462606 | 4.117629615 | 0.048138455 | 0.598988904 |
| hsa-miR-193a-3p | -1.5845588 | 3.995294293 | 0.048897053 | 0.598988904 |
| hsa-miR-130a-3p | -0.88824466 | 6.580760266 | 0.051263632 | 0.604409433 |
| hsa-miR-126-5p | -1.22112275 | 8.006357503 | 0.051621005 | 0.604409433 |

Other sncRNAs differentially expressed between Cb and U+M b groups (FDR<0.5 or p-value <0.05), selected by NGS analysis and used for the first qPCR validation step are shown in **Table 3B,** below.

**Table 3B**

| **small non-coding RNAs** | **logFC** | **logCPM** | **p-Value** | **FDR** |
|---|---|---|---|---|
| tRNA138-ArgACG | -2.27538237 | 7.674009775 | 0.000243882 | 0.204980883 |
| RNU6-11 | -1.67718785 | 4.732492163 | 0.000245185 | 0.204980883 |
| tRNA11-ArgACG | -2.21044709 | 7.59261144 | 0.000408122 | 0.204980883 |
| RNU6-36 | -1.84798113 | 4.63862015 | 0.000436362 | 0.204980883 |
| tRNA175-SerGCT | -1.84762954 | 8.390549302 | 0.001054009 | 0.290869197 |
| U3.39 | 4.655316415 | 4.424357984 | 0.001147835 | 0.290869197 |
| RNU4-6P | -1.71160894 | 4.864935322 | 0.001317818 | 0.290869197 |
| U6.375 | -1.86391998 | 4.231358228 | 0.001366655 | 0.290869197 |
| snoU13.63 | 4.286830869 | 4.29762358 | 0.001552935 | 0.290869197 |
| tRNA8-ThrAGT | -1.65324248 | 9.428427256 | 0.001556007 | 0.290869197 |
| RNU6-1 | -1.37118776 | 4.843026936 | 0.001713194 | 0.290869197 |
| RNU6-31 | -1.53604238 | 4.741724921 | 0.002351551 | 0.290869197 |
| tRNA4-ArgTCG | -1.70070174 | 10.2139807 | 0.002452994 | 0.290869197 |
| tRNA130-ValCAC | -2.21562973 | 4.853113645 | 0.002797565 | 0.290869197 |
| U3.3 | -1.72401526 | 5.267608648 | 0.002991025 | 0.290869197 |
| U4.57 | -1.55456476 | 4.807782758 | 0.003084965 | 0.290869197 |
| tRNA84-GluTTC | -1.39818637 | 10.32369425 | 0.003223874 | 0.290869197 |
| U6.428 | -1.83108856 | 4.169870903 | 0.003289229 | 0.290869197 |
| U3.4 | -1.80036039 | 5.421618508 | 0.00346512 | 0.290869197 |
| tRNA95-AsnGTT | -2.24304788 | 4.752212371 | 0.003469246 | 0.290869197 |
| uc003oif.3 | -2.59243747 | 4.028384043 | 0.003838286 | 0.290869197 |
| U3.2 | -1.73251154 | 5.414684824 | 0.003880282 | 0.290869197 |
| U3.42 | 2.126244843 | 4.003126274 | 0.003910676 | 0.290869197 |
| uc002tgp.1 | 2.810368883 | 4.510093789 | 0.004221387 | 0.290869197 |
| tRNA105-PseudoTTC | -1.59015632 | 7.983774255 | 0.004475105 | 0.290869197 |
| tRNA4-GluCTC | -1.60952388 | 8.107675756 | 0.00453217 | 0.290869197 |
| RNU6-14 | -1.86375965 | 4.058373711 | 0.004721183 | 0.290869197 |
| tRNA4-ThrAGT | -1.34446936 | 8.68050744 | 0.004730298 | 0.290869197 |
| U6.97 | -2.76317407 | 3.745083257 | 0.004815026 | 0.290869197 |
| tRNA120-AlaAGC | -1.31815493 | 9.587766586 | 0.004875694 | 0.290869197 |
| snoU13.348 | 2.256666202 | 4.075754187 | 0.005036202 | 0.290869197 |
| U1.105 | -2.32216301 | 4.207692971 | 0.005058512 | 0.290869197 |
| tRNA27-MetCAT | -2.34782418 | 6.706470837 | 0.0051084 | 0.290869197 |
| U1.10 | -2.59329176 | 4.816351388 | 0.005540106 | 0.306172302 |
| tRNA134-GluTTC | -1.3431528 | 10.39664851 | 0.006091168 | 0.32697053 |
| RNU6-6 | -1.54430018 | 4.19348578 | 0.00626447 | 0.32697053 |
| Y_RNA.75 | 1.904869354 | 5.50161645 | 0.006713926 | 0.340251318 |
| tRNA8-Undet | 1.964251986 | 4.197405842 | 0.006905386 | 0.340251318 |
| SNORA25 | -2.35025006 | 3.740784336 | 0.007062161 | 0.340251318 |
| RNU6-39 | -1.60601357 | 4.333253034 | 0.007621329 | 0.3538182 |
| tRNA119-AlaCGC | -1.25397272 | 9.514833568 | 0.007808074 | 0.3538182 |
| tRNA85-PseudoTTC | 1.126764653 | 5.8624175 | 0.008322426 | 0.3538182 |
| U6.254 | -1.80163898 | 3.954386151 | 0.008365638 | 0.3538182 |
| tRNA3-ArgCCT | -1.40848109 | 10.46544955 | 0.008483646 | 0.3538182 |
| SNORD114-2 | -3.1187271 | 3.929437693 | 0.008576479 | 0.3538182 |
| tRNA162-MetCAT | -2.31337858 | 6.829504657 | 0.008700411 | 0.3538182 |
| Y_RNA.661 | -2.13613309 | 3.924521406 | 0.008896949 | 0.3538182 |
| tRNA8-AlaTGC | -1.03417608 | 10.23229845 | 0.009262877 | 0.3538182 |
| tRNA56-ThrTGT | -1.43137722 | 10.38511882 | 0.009580922 | 0.3538182 |
| tRNA26-AsnGTT | -1.2260783 | 8.800207822 | 0.009587969 | 0.3538182 |
| tRNA73-ArgCCG | -0.87676773 | 7.646480047 | 0.009603368 | 0.3538182 |
| tRNA156-ArgACG | -1.38659506 | 6.417990561 | 0.009974671 | 0.36043089 |
| tRNA28-IleAAT | -0.96475102 | 8.290575814 | 0.010463073 | 0.370945534 |
| RNU6-33 | -1.41177741 | 4.713635974 | 0.010694162 | 0.372117242 |
| tRNA3-ProTGG | 2.564031745 | 13.63247318 | 0.01106572 | 0.37247176 |
| tRNA19-ArgTCG | -1.57386692 | 9.439040466 | 0.011100808 | 0.37247176 |
| tRNA164-MetCAT | -2.24158666 | 6.824859219 | 0.011731788 | 0.372701442 |
| tRNA13-AlaCGC | -1.05980195 | 9.619304583 | 0.011862589 | 0.372701442 |
| U6atac.4 | -2.29659983 | 4.169967341 | 0.012205483 | 0.372701442 |
| tRNA9-AlaAGC | -1.76859219 | 4.913678407 | 0.012206837 | 0.372701442 |
| tRNA132-PseudoCAC | -2.28282945 | 4.122549605 | 0.012249051 | 0.372701442 |
| RNU6-7 | -1.54429239 | 4.25135459 | 0.012297759 | 0.372701442 |
| tRNA20-MetCAT | -1.09889413 | 8.557399735 | 0.012787128 | 0.376829052 |
| U3.24 | -1.49142526 | 5.576968961 | 0.01283505 | 0.376829052 |
| U6atac.29 | -2.38352866 | 3.785989635 | 0.013059629 | 0.377523747 |
| tRNA2-LeuTAA | -1.66936742 | 3.974107686 | 0.013474016 | 0.383601155 |
| tRNA1-AsnGTT | -0.93147558 | 7.962088764 | 0.014090452 | 0.39410967 |
| tRNA40-ThrAGT | -1.20252417 | 8.00746198 | 0.014483817 | 0.39410967 |
| Y_RNA.684 | 2.380168702 | 3.849210771 | 0.014510103 | 0.39410967 |
| snoU13.86 | 3.047260829 | 3.887450563 | 0.014700195 | 0.39410967 |
| uc021qtn.1 | -2.42558461 | 4.054546636 | 0.015164662 | 0.39410967 |
| RNU6-9 | -1.27226823 | 4.707463894 | 0.01590456 | 0.39410967 |
| tRNA13-AlaTGC | -1.07694323 | 9.510390292 | 0.016125256 | 0.39410967 |
| tRNA36-ArgACG | -1.42222279 | 6.510745574 | 0.016234233 | 0.39410967 |
| Y_RNA.719 | -1.87621292 | 4.049552581 | 0.016340695 | 0.39410967 |
| RNU6-10 | -1.35736219 | 4.333057065 | 0.016389921 | 0.39410967 |
| SCARNA4 | 0.900162218 | 5.772147414 | 0.016691546 | 0.39410967 |
| tRNA10-AlaCGC | -1.08617369 | 9.364295675 | 0.016698714 | 0.39410967 |
| tRNA12-SerAGA | -2.17129695 | 3.960150632 | 0.016746309 | 0.39410967 |
| uc011ley.2 | -1.1603733 | 7.787151849 | 0.01677955 | 0.39410967 |
| U1.116 | -2.53057666 | 4.143384152 | 0.017351703 | 0.3975759 |
| tRNA7-SerGCT | -1.22744148 | 8.048077286 | 0.017683906 | 0.3975759 |
| U6.266 | -1.68215765 | 4.162672228 | 0.018006863 | 0.3975759 |
| tRNA136-AsnGTT | -1.18546894 | 6.252578135 | 0.018057145 | 0.3975759 |
| uc031qaf.1 | 3.07982529 | 4.21672176 | 0.018100789 | 0.3975759 |
| RNU4-9P | 1.971224737 | 3.892961999 | 0.018196662 | 0.3975759 |
| RNU6-48 | -1.41494718 | 4.179417866 | 0.018610429 | 0.40194249 |
| U4.64-201 | -1.46842052 | 4.573943777 | 0.019052174 | 0.405290215 |
| SNORA70.5 | -2.3066839 | 3.705457206 | 0.019552672 | 0.405290215 |
| tRNA83-AsnGTT | -0.95893311 | 7.650753844 | 0.019695599 | 0.405290215 |
| tRNA21-ArgCCT | -1.3243702 | 9.463647642 | 0.019740822 | 0.405290215 |
| RNU6-26 | -1.26772628 | 4.674223977 | 0.019843906 | 0.405290215 |
| tRNA4-AsnGTT | -0.95216219 | 7.630343605 | 0.020477143 | 0.409060595 |
| U1.28 | -1.66671858 | 4.666167958 | 0.020920233 | 0.409060595 |
| U6.168 | -1.75957771 | 3.988195609 | 0.021394709 | 0.409060595 |
| RNU6-41-201 | -1.2429829 | 4.599263654 | 0.021413125 | 0.409060595 |
| tRNA15-ThrCGT | -0.94643604 | 8.011751983 | 0.021715336 | 0.409060595 |
| tRNA88-PseudoCCT | 1.406009411 | 4.775992477 | 0.021727046 | 0.409060595 |
| SNORD116-19-201 | -2.08108498 | 3.769046556 | 0.022072958 | 0.409060595 |
| uc031qtw.1 | -1.59586877 | 3.698957807 | 0.022111714 | 0.409060595 |
| U6.422-201 | -2.18510267 | 3.771206474 | 0.022136152 | 0.409060595 |
| U2.3-201 | -1.09052752 | 5.567176812 | 0.022205525 | 0.409060595 |
| U3.20-201 | -1.55295135 | 5.225744713 | 0.022487766 | 0.410237987 |
| tRNA165-IleAAT | -0.81674152 | 8.525161575 | 0.023067622 | 0.416176914 |
| snoU13.160-201 | -1.88380693 | 3.690235583 | 0.023604159 | 0.416176914 |
| tRNA36-ThrAGT | -1.09203025 | 7.99788872 | 0.023653935 | 0.416176914 |
| tRNA6-TrpCCA | -1.06582891 | 10.59444161 | 0.023699271 | 0.416176914 |
| SNORA57-201 | -1.08263716 | 5.14915277 | 0.024108905 | 0.416665441 |
| tRNA10-PseudoTGA | -1.42087364 | 5.662435588 | 0.024173287 | 0.416665441 |
| uc001myu.3 | -2.42408101 | 4.681561536 | 0.02449142 | 0.416665441 |
| Y_RNA.428-201 | -1.2545658 | 6.369684888 | 0.024614084 | 0.416665441 |
| SCARNA1-201 | -1.61719768 | 3.852134167 | 0.025631096 | 0.427567583 |
| tRNA95-AlaAGC | -1.73027899 | 5.825175769 | 0.02592783 | 0.427567583 |
| RNU6-27-201 | -1.6140049 | 3.926299889 | 0.025940769 | 0.427567583 |
| SNORA79.1-201 | -1.15165538 | 4.196182085 | 0.026538748 | 0.433620064 |
| tRNA9-IleAAT | -0.73350055 | 8.536774944 | 0.027547951 | 0.446033785 |
| tRNA9-TyrGTA | -1.6487492 | 3.959996969 | 0.027773259 | 0.446033785 |
| tRNA23-ArgCCG | -1.31033598 | 11.27930104 | 0.029044541 | 0.450180862 |
| snoU13.318-201 | -1.91587082 | 3.698261411 | 0.029045067 | 0.450180862 |
| snoU13.328-201 | 2.684496203 | 4.280709032 | 0.029064976 | 0.450180862 |
| RNU4-4P-201 | -1.2311376 | 4.615863264 | 0.029137817 | 0.450180862 |
| Y_RNA.633-201 | -1.30999509 | 4.118055921 | 0.029229412 | 0.450180862 |
| RNU5F-4P-201 | -1.48557387 | 4.239223966 | 0.03016703 | 0.460844298 |
| tRNA7-AsnGTT | -0.88097126 | 7.861805832 | 0.031177808 | 0.472444358 |
| tRNA2-ArgCCT | -1.14460252 | 9.768619156 | 0.031689267 | 0.476353057 |
| tRNA18-ArgCCT | -1.24884324 | 9.421599432 | 0.032541851 | 0.484021239 |
| SNORD116-25-201 | 1.724675764 | 6.034751745 | 0.032861609 | 0.484021239 |
| uc02lybm.1 | -0.9249247 | 7.924193472 | 0.033450576 | 0.484021239 |
| tRNA8-SerGCT | -0.98753133 | 8.589816708 | 0.033481121 | 0.484021239 |
| tRNA11-IleAAT | -0.72451906 | 8.545784821 | 0.033487366 | 0.484021239 |
| Y_RNA.597-201 | 1.732733959 | 3.760735382 | 0.034265836 | 0.491492414 |
| snoU13.120-201 | 2.312826375 | 3.740373282 | 0.034550843 | 0.491826017 |
| uc031qpu.1 | 2.324532842 | 3.768342356 | 0.035349099 | 0.49676233 |
| RNU6-32-201 | -1.18812391 | 4.327815738 | 0.035426372 | 0.49676233 |
| tRNA100-PseudoGAA | -1.02843913 | 9.329302032 | 0.036035102 | 0.501555229 |
| SNORD116-21-201 | 2.33201432 | 3.747426753 | 0.039146195 | 0.532853476 |
| tRNA5-IleTAT | -1.08777911 | 7.207879458 | 0.039415342 | 0.532853476 |
| Y_RNA.522-201 | -1.81515534 | 4.101934504 | 0.039799275 | 0.532853476 |
| tRNA12-ArgCCT | -1.27295081 | 9.216801318 | 0.039946538 | 0.532853476 |
| U6atac.20-201 | -1.94596283 | 4.036085302 | 0.040551585 | 0.532853476 |
| RNU6-4-201 | -1.52406279 | 4.105411071 | 0.040667896 | 0.532853476 |
| SNORD3B-2-201 | -0.79576357 | 8.540015612 | 0.040838684 | 0.532853476 |
| tRNA11-AsnGTT | -1.30462038 | 4.970141103 | 0.040850618 | 0.532853476 |
| tRNA31-AsnGTT | -0.87710624 | 7.603607761 | 0.041107699 | 0.532853476 |
| Y_RNA.727-201 | -1.25639747 | 4.115195415 | 0.041225337 | 0.532853476 |
| RNU6-73-201 | -1.46456929 | 4.007858615 | 0.042069664 | 0.532853476 |
| SNORA65-201 | -0.88817274 | 4.89950806 | 0.042220138 | 0.532853476 |
| tRNA7-LeuAAG | -0.71492427 | 8.880350642 | 0.042319347 | 0.532853476 |
| tRNA144-AspGTC | -0.82964134 | 11.54055138 | 0.042320413 | 0.532853476 |
| U6atac.25-201 | -1.77774451 | 3.762252674 | 0.042537531 | 0.532853476 |
| tRNA 1-ArgCCG | -0.76847131 | 7.276226466 | 0.043525106 | 0.535998445 |
| SNORA7.4-201 | -1.13362614 | 3.738511771 | 0.043665668 | 0.535998445 |
| tRNA125-ThrCGT | -1.08045128 | 5.458664529 | 0.043842913 | 0.535998445 |
| RNU6-45-201 | -1.22266691 | 4.217769003 | 0.043929622 | 0.535998445 |
| tRNA131-GInCTG | -1.07262477 | 7.700551934 | 0.04461864 | 0.538855811 |
| U6.1249-201 | 1.668615649 | 3.756761746 | 0.044737364 | 0.538855811 |
| Y_RNA.245 | 2.25187274 | 3.754483152 | 0.045943642 | 0.545496798 |
| U6.601 | -1.35324491 | 3.970443965 | 0.046003943 | 0.545496798 |
| RNU5E-4P | -1.0837447 | 5.498269856 | 0.046159655 | 0.545496798 |
| tRNA66-AlaTGC | -0.81125784 | 9.226831102 | 0.046988475 | 0.545729532 |
| tRNA32-MetCAT | -0.82679514 | 8.99856138 | 0.047086999 | 0.545729532 |
| U4.79 | -1.25353509 | 4.433132105 | 0.047164353 | 0.545729532 |
| tRNA4-HisGTG | -1.61899083 | 3.790696889 | 0.047553836 | 0.545729532 |
| U3.13 | -1.51261982 | 3.797771351 | 0.047790048 | 0.545729532 |
| RNU6-5 | -1.02972321 | 4.777615387 | 0.048416122 | 0.545729532 |
| SNORD36A | -1.75616298 | 3.84054929 | 0.048459133 | 0.545729532 |
| tRNA1-LeuAAG | -0.6699966 | 8.883794181 | 0.048648388 | 0.545729532 |
| RNU6-29 | -1.13048434 | 4.36662749 | 0.048793274 | 0.545729532 |

Putative microRNAs differentially expressed between Cb and U+M b groups (FDR<0.5 or p-value <0.05), selected by NGS analysis and used for the fisrt qPCR validation step are shown in **Table 3C,** below.

**Table 3C**

| **putative microRNAs** | **Sequences** | **logFC** | **logCPM** | **PValue** | **FDR** |
|---|---|---|---|---|---|
| put-miR-718 | TTCAGGTTACCGCAGG | - 6.825255 | 9.009089 | 1.21E-05 | 0.01609 |
| put-miR-594 | TGATTTTTTTTGGCGAA | - 8.122089 | 13.62184 | 4.24E-05 | 0.028197 |
| put-miR-703 | GACTGCTCGAGCTGCTT | - 4.582706 | 9.340091 | 0.000177 | 0.065276 |
| put-miR-477 | TGGGCAGCAAGAATGG | - 6.350308 | 8.65459 | 0.00021 | 0.065276 |
| put-miR-727 | GGAGGATTTCAATTGG | - 4.944239 | 7.848316 | 0.000246 | 0.065276 |
| put-miR-646 | TCTGTCATGGCTGAGC | - 6.551056 | 8.808979 | 0.000322 | 0.065276 |
| put-miR-675 | TACCTCAATTCTCTAGG | 5.756095 | 8.531088 | 0.000387 | 0.065276 |
| put-miR-319 | TGTCGGCGGGGGCCCCGGAC | - 3.557921 | 10.21937 | 0.000409 | 0.065276 |
| put-miR-327 | CATTCTCTGAAACTACA | - 6.062301 | 8.473821 | 0.000441 | 0.065276 |
| put-miR-338 | GACTTGTGATTAGCGG | 5.013978 | 10.59887 | 0.000571 | 0.067661 |
| put-miR-1187 | GAGCATGTTGACTGGAGA | - 6.150807 | 8.514764 | 0.000634 | 0.067661 |
| put-miR-6 | GCGGACCTTGCTCAAGG | 8.830503 | 11.10951 | 0.000659 | 0.067661 |
| put-miR-341 | TGAGAAAACATTTGAGG | - 5.709943 | 8.260643 | 0.000661 | 0.067661 |
| put-miR-750 | CAAGAGATGAGGAATG | - 5.585098 | 8.184314 | 0.000814 | 0.07204 |
| put-miR-183 | CCAGGCTGGTCGTGATGA | - 5.716867 | 8.806919 | 0.000855 | 0.07204 |
| put-miR-980 | TCGGAAGAAGAAGCTGACCCA | - 5.533531 | 8.158978 | 0.000866 | 0.07204 |
| put-miR-798 | TAACTCTTGGAAATCTTGG | - 3.422593 | 9.962536 | 0.001016 | 0.075645 |
| put-miR-765 | AAAAAAGAGGGACAGAAATG | - 5.173073 | 7.963608 | 0.001123 | 0.075645 |
| put-miR-71 | GACCTTGGGTTGGGTCGTGGT | 3.989796 | 8.732315 | 0.001131 | 0.075645 |
| put-miR-1327 | GAAAAGCAATCGTCACAG | - 7.093524 | 9.887535 | 0.001158 | 0.075645 |
| put-miR-371 | AATATTCAGCAGTCAGACTGG | 6.778311 | 9.768186 | 0.001224 | 0.075645 |
| put-miR-354 | CAGGGTGATGACTTCTG | - 4.795128 | 7.781813 | 0.001302 | 0.075645 |
| put-miR-688 | GTGATGTCGGCTCATCGCAACCT | 2.199185 | 10.66612 | 0.001307 | 0.075645 |
| put-miR-756 | TGAGGGATGTTTGGATGCTCGCTTTGA | 2.032426 | 8.603553 | 0.001428 | 0.079215 |
| put-miR-1146 | CAACTGTAAGTCCATT | 4.770832 | 9.196252 | 0.00158 | 0.079645 |
| put-miR-1333 | TAATTGTCCTCTTGGGG | - 4.794112 | 7.786171 | 0.001619 | 0.079645 |
| put-miR-511 | AAGCAGGCGTTACAATG | - 7.089632 | 9.54354 | 0.001631 | 0.079645 |
| put-miR-1199 | TGAACTCGGGAGAAGGAAGT | - 2.994051 | 11.94621 | 0.001702 | 0.079645 |
| put-miR-705 | CCTGTCTGACTGGGTCTCC | 6.097356 | 9.460857 | 0.00175 | 0.079645 |
| put-miR-1275 | AAGAAGTAGACTGGATTGG | - 4.175643 | 7.541711 | 0.001817 | 0.079645 |
| put-miR-1204 | AGGGCTGGGCACGGGGG | - 3.329219 | 8.43806 | 0.00195 | 0.079645 |
| put-miR-857 | TAAGTGGGAGTGCTCATG | 5.519775 | 8.389269 | 0.001957 | 0.079645 |
| put-miR-806 | CACGAGAGAACGCACACC | 7.044016 | 9.996928 | 0.002048 | 0.079645 |
| put-miR-680 | GTTAAGTATGCACAGG | 6.207669 | 9.293799 | 0.002048 | 0.079645 |
| put-miR-576 | TGGCATCCTGTCTCTTGC | 5.455074 | 8.34397 | 0.002094 | 0.079645 |
| put-miR-444 | CTGAAAAGGGGACGGATTGGGAA | - 4.481465 | 8.254479 | 0.002327 | 0.083951 |
| put-miR-1132 | CACGCTGTCTTTTGTTTCTCT | - 5.505609 | 11.5441 | 0.002357 | 0.083951 |
| put-miR-1025 | TGGAAGAGGGGAAAGGAGA | - 4.492628 | 7.660657 | 0.002418 | 0.083951 |
| put-miR-496 | CTAAGAGTATGAGTAGC | 8.320903 | 10.63469 | 0.00246 | 0.083951 |
| put-miR-657 | TCAGGACATTGGACTCT | - 4.875422 | 9.301419 | 0.002633 | 0.087604 |
| put-miR-625 | GAGAAGACTGAATGCTCTTCTC | 5.988262 | 8.702871 | 0.002699 | 0.087604 |
| put-miR-40 | GCTGGAATAGCTCAGTTGC | - 4.339713 | 10.82143 | 0.00295 | 0.093474 |
| put-miR-666 | CAGCATCATGATCATTATGG | 6.351541 | 8.967721 | 0.003077 | 0.095259 |
| put-miR-1098 | AAGGCGAAGGATATGTTG | 7.117298 | 9.563651 | 0.003171 | 0.095916 |
| put-miR-538 | CATTGTGCTTCGTGGGAGAGTAGGGCA | - 1.614431 | 8.767576 | 0.003422 | 0.101205 |
| put-miR-952 | GCCGACAGGTCCGGGTAA | - 5.048736 | 10.09864 | 0.003556 | 0.101506 |
| put-miR-572 | TAGATTCAGGAACTGCCA | 7.280517 | 9.715353 | 0.003598 | 0.101506 |
| put-miR-972 | TTTAGTAGAATTGTGTTGGGT | - 3.277557 | 9.430308 | 0.003661 | 0.101506 |
| put-miR-650 | AAAAGCAAGGCGAAGAGG | - 2.912473 | 9.278157 | 0.003775 | 0.102538 |
| put-miR-134 | CAGACTGCTCGAGCTGCTGC | - 3.339267 | 9.218851 | 0.003866 | 0.102908 |
| put-miR-975 | TTTGCAGACTGGAAACT | 5.625435 | 8.451316 | 0.004252 | 0.109356 |
| put-miR-161 | TGTCTGTAGCAATGTGCT | - 6.214205 | 9.449224 | 0.004553 | 0.109356 |
| put-miR-1265 | TGGGGATAAAGTTAGGT | 3.433066 | 12.55135 | 0.004559 | 0.109356 |
| put-miR-968 | TGGCTGTTGCACTTCT | 8.03171 | 10.36384 | 0.004661 | 0.109356 |
| put-miR-1125 | TTGAAGACTGGCTCTCA | - 4.530734 | 8.355286 | 0.00501 | 0.109356 |
| put-miR-892 | CAACGGTCTTGAAAACA | 3.239723 | 8.81171 | 0.005015 | 0.109356 |
| put-miR-1306 | ATAACGTCATCTAGTGTG | - 4.744221 | 10.56016 | 0.005065 | 0.109356 |
| put-miR-948 | TGGAAGAAAACGAGGAG | 5.807023 | 8.569858 | 0.005204 | 0.109356 |
| put-miR-925 | ATTTTGGTCTGTTGGTT | 4.384338 | 8.643659 | 0.005342 | 0.109356 |
| put-miR-259 | ATGTAACCGGGCTTTTGTGCT | - 1.786601 | 8.598848 | 0.005378 | 0.109356 |
| put-miR-209 | GGTCCTCGGATCGGCC | - 4.053427 | 9.542155 | 0.005417 | 0.109356 |
| put-miR-773 | CAGGCAGAAGGGAGCTTGT | -2.64537 | 7.45467 | 0.005514 | 0.109356 |
| put-miR-910 | CACGTTCTTCTCATGGT | - 3.449747 | 7.34067 | 0.00564 | 0.109356 |
| put-miR-664 | AAAGTTGTGTTAGCTGA | 5.719212 | 8.51182 | 0.005786 | 0.109356 |
| put-miR-1068 | TATGATTGTAAACTCTGA | 5.890866 | 8.622725 | 0.005843 | 0.109356 |
| put-miR-265 | CACATGGGGGTAGAGCACTGACTGGG | 2.110742 | 12.54113 | 0.005876 | 0.109356 |
| put-miR-490 | CTGCAGGAGTGTTTGAGA | 2.057932 | 11.07639 | 0.005969 | 0.109356 |
| put-miR-1155 | CAAATGATCAAAGCAGG | 2.494173 | 8.354137 | 0.006008 | 0.109356 |
| put-miR-893 | ACCATCCTCTGCTACCA | - 2.575152 | 8.358309 | 0.006075 | 0.109356 |
| put-miR-1342 | ATCGGAAAATGTGGGAA | 5.585468 | 8.418657 | 0.006226 | 0.109356 |
| put-miR-967 | AAATGCATTGGATATGG | 7.085615 | 9.549591 | 0.006355 | 0.109356 |
| put-miR-811 | GTTGTAAAGCTCTGTTG | 1.99226 | 9.063173 | 0.006362 | 0.109356 |
| put-miR-8 12 | GTTCTGAGTTCTTTGGTTGGA | 7.523344 | 9.907158 | 0.006484 | 0.109356 |
| pul-miR-1084 | AGGACCATTGCGTTGCC | 6.454871 | 9.04042 | 0.006577 | 0.109356 |
| put-miR-626 | TTCACTACTTTATCTCTTTT | 2.849801 | 9.706572 | 0.006577 | 0.109356 |
| put-miR-127 | AGACTGTGATGACTGGGAGAGCGGGCT | - 2.251745 | 7.719415 | 0.006585 | 0.109356 |
| put-miR-219 | GAGGCTCGAGAGCAATGGC | 4.972872 | 8.410708 | 0.006604 | 0.109356 |
| put-miR-863 | AAGTTGGAGTATGTTTTAGG | 4.174184 | 8.270046 | 0.006626 | 0.109356 |
| put-miR-33 | CCATGACTGCAGATGG | - 4.437318 | 8.062018 | 0.006671 | 0.109356 |
| put-miR-1246 | CTGGAGATCTGTTTGGGC | 6.389535 | 8.993512 | 0.00675 | 0.109356 |
| put-miR-126 | GTGGGGCTTAGTGCTGA | - 4.160086 | 8.05272 | 0.006849 | 0.109356 |
| put-miR-459 | GGGATCAACCTGACAA | 7.00545 | 9.47012 | 0.00689 | 0.109356 |
| put-miR-907 | CCAACAGCTTCTGAGTTG | - 5.599664 | 9.28021 | 0.006923 | 0.109356 |
| put-miR-171 | GTCCTGTGTCTTGTACGG | 2.359948 | 10.86341 | 0.006942 | 0.109356 |
| put-miR-1163 | AGAGGACAGGGAAGCTT | 6.420538 | 9.017069 | 0.006996 | 0.109356 |
| put-miR-204 | AGAAGTCAGAACCTCTAT | 6.610111 | 9.154177 | 0.007138 | 0.109356 |
| put-miR-498 | GACAGATTTTAGCTTGTCC | 2.214527 | 8.621153 | 0.007148 | 0.109356 |
| put-miR-286 | TAACAGGCGTTGAAATTGT | 6.754071 | 9.266989 | 0.007468 | 0.112951 |
| put-miR-223 | GATGGGGGTAGAGCACTGC | 2.25836 | 12.07399 | 0.007601 | 0.113671 |
| put-miR-75 | TGGGGATTGTGGGTTCTC | - 2.009287 | 9.598075 | 0.00774 | 0.114174 |
| put-miR-548 | AGAGGACAGGGAAGCT | 6.104866 | 8.784136 | 0.007923 | 0.114174 |
| put-miR-1118 | AGAGGGTTCCTGTAGACCTAGGGAGGA | - 2.053777 | 10.22693 | 0.007925 | 0.114174 |
| put-miR-943 | AGCCAGAGTTCTGATTGTGAGTG | - 4.954652 | 9.727141 | 0.007978 | 0.114174 |
| put-miR-313 | TGAGTTCTTTGGTCAGAA | 6.693723 | 9.211486 | 0.00821 | 0.114289 |
| put-miR-659 | AGTAGCTGGTCGATTTGGC | 6.996383 | 11.79431 | 0.008416 | 0.114289 |
| put-miR-2 10 | CTTCAGACTGTGAAACTGA | - 2.997776 | 9.522779 | 0.008481 | 0.114289 |
| put-miR-144 | TAGAGATAGAGCTTATG | 5.529267 | 8.376117 | 0.008489 | 0.114289 |
| put-miR-563 | TGGAGACATTAACTATGA | 5.915791 | 8.643491 | 0.008604 | 0.114289 |
| put-miR-1140 | TGGGAAGGGCTGCCGG | - 5.145788 | 9.310603 | 0.008606 | 0.114289 |
| put-miR-497 | TTGGTAGACTCTCACTT | - 4.014605 | 9.04369 | 0.008664 | 0.114289 |
| put-miR-573 | TGGATAACTCTTTTTGTA | 6.440895 | 9.021507 | 0.008788 | 0.114289 |
| put-miR-906 | TATGGGAAGAATCTGGG | 6.054559 | 8.747883 | 0.008793 | 0.114289 |
| put-miR-231 | TATGTCATGGTGGCTTTGG | -2.9467 | 8.39848 | 0.008918 | 0.114289 |
| put-miR-85 | GAATCCCACTTCTGACACCA | - 2.952626 | 7.663083 | 0.00901 | 0.114289 |
| put-miR-884 | ATGGTCTAGAGCTACAGGT | 5870477 | 8.628199 | 0.00911 | 0.114289 |
| put-miR-918 | CAGCTTCTTCCGCTTCTT | 5.860051 | 8.60284 | 0.009174 | 0.114289 |
| put-miR-985 | GTACTGCATCTCTGCA | 5.898607 | 8.628545 | 0.009188 | 0.114289 |
| put-miR-320 | ACTGGATCCAAGAAAAG | - 2.920156 | 8.226536 | 0.009571 | 0.11795 |
| put-miR-67 | AGGACGTTGGTCAGAGC | - 2.895467 | 7.636799 | 0.009857 | 0.119419 |
| put-miR-221 | TCTCTCAATCCTCTTGG | 5.954163 | 8.658651 | 0.009869 | 0.119419 |
| put-miR-471 | AGAAGCAGAGAACGAGG | - 3.987795 | 8.44957 | 0.009973 | 0.119585 |
| put-miR-1352 | TGGATTGTGGGGGAACC | 5.478958 | 8.36409 | 0.010086 | 0.119857 |
| put-miR-610 | AGGAGATATAGCTCTTGT | 5.722079 | 8.518132 | 0.010392 | 0.122408 |
| put-miR-48 | AAAATGGATTCGAACCA | 5.632443 | 8.450136 | 0.010602 | 0.123785 |
| put-miR-529 | TGACCTTTTGCCTTCTGC | 5.426419 | 8.338572 | 0.010802 | 0.125018 |
| put-miR-424 | TAAAAAGTTCTCTGTTTTTC | -2.78953 | 7.208748 | 0.010937 | 0.12549 |
| put-miR-24 | TTGGTGCATCTGTAGTCCAAC | - 4.932824 | 10.24045 | 0.011113 | 0.126418 |
| put-miR-164 | TGAAATTCTAAATATTGCA | 5.725561 | 8.51014 | 0.011372 | 0.126957 |
| put-miR-229 | TGGGATTTAGCTCAGC | 3.799609 | 8.12666 | 0.011424 | 0.126957 |
| put-miR-525 | AAGTGGGAAGGCCCAGA | - 3.723862 | 8.317629 | 0.011446 | 0.126957 |
| put-miR-325 | TTCAAATCCCACTTCTGACACCA | - 2.927094 | 7.781125 | 0.012443 | 0.136691 |
| put-miR-188 | AAATGGCGATACTCAGG | 6.335286 | 9.481014 | 0.012559 | 0.136691 |
| put-miR-1066 | TGCTTTGATCGTAGCCC | - 3.205461 | 7.447675 | 0.012632 | 0.136691 |
| put-miR-1295 | GAGGTTAGGATATCTGGCT | - 3.324069 | 7.580857 | 0.012893 | 0.13839 |
| put-miR-588 | TTTTGAACGTTCTTTCTT | 1.985563 | 8.910298 | 0.013328 | 0.141683 |
| put-miR-1273 | AAGGGGAGGAATTTCACGTG | 4.845324 | 8.579749 | 0.013413 | 0.141683 |
| put-miR-783 | AAGGAAAAAGCGGATA | - 3.722555 | 7.559427 | 0.013621 | 0.14275 |
| put-miR-37 | TGGTGGAGTGAAGACG | - 4.836261 | 8.547591 | 0.014174 | 0.14647 |
| put-miR-1317 | TGGAGTGTGGATTGGGG | 1.625507 | 8.870365 | 0.014196 | 0.14647 |
| put-miR-97 | CTGCGTGGCTCTGACAC | 3.27664 | 9.296956 | 0.014347 | 0.146889 |
| put-miR-1016 | AGGTAAAGCTCATGAGG | - 2.141009 | 9.304303 | 0.014567 | 0.148003 |
| put-miR-546 | CCAAGGGGTTGTAGGGCCACT | 3.880018 | 10.64815 | 0.015027 | 0.15152 |
| put-miR-1099 | AGACTCCTTTATCGTA | 4.881265 | 8.359673 | 0.015178 | 0.151894 |
| put-miR-874 | ATGAGCATTGATTTAGG | - 4.133563 | 8.481847 | 0.015742 | 0.15277 |
| put-miR-701 | TTGAGATTTGAGGGGGCCT | 2.361165 | 8.446935 | 0.015782 | 0.15277 |
| put-miR-279 | CTGTGAAGCCTGTTGGTTTGCTGCTG | - 2.002787 | 8.036423 | 0.015783 | 0.15277 |
| put-miR-184 | TGCTGAACCTCTGTATGT | 5.624113 | 9.598097 | 0.015798 | 0.15277 |
| put-miR-495 | CAGGGAGTGAAAGAGAATT | - 2.791411 | 9.973484 | 0.015923 | 0.15277 |
| put-miR-90 | GAAGTTAAATCCTTGGG | 3.643929 | 8.886684 | 0.016047 | 0.15277 |
| put-miR-468 | ATAAGGAACTGCTCTCTC | 4.936942 | 8.333361 | 0.016099 | 0.15277 |
| put-miR-469 | GCGGGGGATTAGCTCAGCTGGG | 1.223156 | 14.10575 | 0.016186 | 0.15277 |
| put-miR-1195 | ATTCTTTGACATGCAGAT | 2.561114 | 9.286221 | 0.016299 | 0.15277 |
| put-miR-250 | CTGGTGTAGAATTGAGG | - 4.411037 | 8.166635 | 0.016733 | 0.153408 |
| put-miR-1264 | CAGGTTTCAGACTTTAGG | 2.452989 | 9.271027 | 0.0168 | 0.153408 |
| put-miR-465 | CCAGTTGTCGTGGGTTTTT | 2.030242 | 8.962972 | 0.016867 | 0.153408 |
| put-miR-1243 | TGTGGATTTTTGTCATGT | 4.743252 | 8.639084 | 0.016871 | 0.153408 |
| put-miR-771 | CCTTGATCTGACTGGGGGCC | - 1.886812 | 8.602159 | 0.016943 | 0.153408 |
| put-miR-534 | CTGTTGGAGAATTTGGAATATTAGGT | -4.34848 | 8.658536 | 0.01741 | 0.155563 |
| put-miR-80 | GAAGAGGGAGTGGTCTGTAAATGCG | 6.380693 | 9.533096 | 0.017415 | 0.155563 |
| put-miR-347 | AAAGGGAAACAAGAATTCTT | 1.937026 | 10.57284 | 0.017748 | 0.156927 |
| put-miR-4 10 | GGAGAATAGAACATGCTGATT | 6.652715 | 9.666187 | 0.017824 | 0.156927 |
| put-miR-891 | TAAATGTTGGTTTGTTTGT | 1.858836 | 8.369573 | 0.017921 | 0.156927 |
| put-miR-189 | GAGCGAAACGGCAGGAT | - 2.368257 | 8.305643 | 0.018423 | 0.160266 |
| put-miR-842 | ACCCGGAGAACTGAACT | - 2.045068 | 9.280359 | 0.01862 | 0.160514 |
| put-miR-81 | ATTTGAAAGAATGCTTG | 2.468711 | 8.296721 | 0.018693 | 0.160514 |
| put-miR-824 | CTAGCTGAACCTCTGTAT | 5.277761 | 8.805933 | 0.018935 | 0.161554 |
| put-miR-958 | ACAGGGCTGTGCAAAAA | 3.475245 | 8.121044 | 0.019332 | 0.163891 |
| put-miR-742 | CAGGGCTGTGCTAACT | 2.255055 | 7.794269 | 0.020414 | 0.171266 |
| put-miR-605 | TGAGCTTTGGAAGAAGGACCA | - 2.303646 | 8.016644 | 0.020459 | 0.171266 |
| put-miR-374 | TGTGAGGATGTTCTGTAAGGAGTGTT | 4.607626 | 8.644756 | 0.020848 | 0.173428 |
| put-miR-135 | TGAATTACGGAAGTGTTGGTTAAT | - 3.311813 | 7.404022 | 0.021756 | 0.179859 |
| put-miR-323 | ATAAAATGGGCGTTGAGG | 3.262325 | 8.71569 | 0.022489 | 0.184771 |
| put-miR-506 | AAGAGGGGCTTTTAGAACC | 6.174059 | 11.22955 | 0.022884 | 0.186859 |
| put-miR-293 | GGGTAAAACTGCAGTGGGCGTTGGTAG | - 1.800636 | 10.99339 | 0.02336 | 0.189584 |
| put-miR-524 | CGTGGTGATGCTCTGACA | - 2.434924 | 7.973262 | 0.023561 | 0.189632 |
| put-miR-517 | TTGGGAAGGGCTGCCGGA | - 4.206993 | 8.774926 | 0.023651 | 0.189632 |
| put-miR-391 | ATCTCGATCCAGTAGTC | 1.801909 | 7.884667 | 0.024454 | 0.194897 |
| put-miR-92 | AGAGACTGACTTTGAGTA | 5.755643 | 9.030306 | 0.025075 | 0.198658 |
| put-miR-435 | TTTAGACCGTTTTTATGTC | - 3.735696 | 9.202301 | 0.027033 | 0.212767 |
| put-miR-1080 | TGTGGATTGATGCTCT | - 1.707634 | 8.549845 | 0.027175 | 0.212767 |
| put-miR-587 | TTTGTAGAAGAGGAAGCG | - 2.497802 | 8.421284 | 0.029781 | 0.231807 |
| put-miR-1220 | TAGAATGGGGCTTGTTGC | 3.980805 | 8.446505 | 0.030296 | 0.233459 |
| put-miR-1134 | AAAGAATGAAGTTGGTCTGG | - 1.684102 | 8.848135 | 0.030344 | 0.233459 |
| put-miR-352 | TAATGAATGACTGTTTG | 1.774841 | 8.080119 | 0.030753 | 0.23524 |
| put-miR-1162 | AAAGAACGTTCAAAGG | 2.807352 | 8.476403 | 0.030994 | 0.235731 |
| put-miR-377 | AAAGGGCTTTGACTATTT | 1.681479 | 13.05617 | 0.031488 | 0.237918 |
| put-miR-294 | TGCTTCCTCAATCGGT | 3.603279 | 8.466728 | 0.031639 | 0.237918 |
| put-miR-1229 | ACAGTAGCAATGTTCTGC | 3.278472 | 7.88184 | 0.032091 | 0.238095 |
| put-miR-1091 | GATTATGATTGTGATTGTAGC | 4.964642 | 9.493873 | 0.032144 | 0.238095 |
| put-miR-419 | AAAAAGTTAGACTTAGG | 1.437457 | 9.015693 | 0.032199 | 0.238095 |
| put-miR-249 | GCCAGGATGTTGGCTTA | - 1.617688 | 9.173125 | 0.03244 | 0.238442 |
| put-miR-122 | GGAACTCATGATTGTTGACTTTGG | - 1.814811 | 8.353927 | 0.032687 | 0.238442 |
| put-miR-1320 | TTAGAGGCCACTCAAT | 4.820365 | 8.331847 | 0.032994 | 0.238442 |
| put-miR-335 | TATTTAAATGAGAACTTTGAAGC | 2.147361 | 9.043008 | 0.033061 | 0.238442 |
| put-miR-166 | CGTTTTGGTGTTGGTTG | -3.72297 | 8.052795 | 0.033142 | 0.238442 |
| put-miR-1172 | AGAACCAAGAAGCTCTGG | 2.612818 | 8.384166 | 0.034733 | 0.246677 |
| put-miR-1207 | GTGAAAAGACATAGGGGG | 1.882735 | 9.817827 | 0.034763 | 0.246677 |
| put-miR-1130 | TAGAAGAGGGAGCTTCTTT | 2.429349 | 9.717761 | 0.034842 | 0.246677 |
| put-miR-247 | AGAGCTAGAATCCAGG | 1.990242 | 8.786356 | 0.035469 | 0.249784 |
| put-miR-246 | TTTATTAGAGACGGGACTTT | 4.838701 | 8.30995 | 0.035789 | 0.250379 |
| put-miR-870 | AAAGGATGTAGACAAGGGA | 1.480442 | 8.130793 | 0.03593 | 0.250379 |
| put-miR-704 | CAAATGAATATCTGGGA | 2.582114 | 8.282107 | 0.037366 | 0.258799 |
| put-miR-266 | TTTAGGTACTCTGAACAA | 2.505244 | 8.581082 | 0.037586 | 0.258799 |
| put-miR-928 | CACAGAAGGAACGTTTAGA | 2.539814 | 8.530779 | 0.037862 | 0.258799 |
| put-miR-959 | GAAAGAGAGTGAGACTCA | 2.359451 | 8.451334 | 0.038062 | 0.258799 |
| put-miR-676 | TGGTGGTTGGTTTTGGG | - 1.247728 | 9.504279 | 0.03818 | 0.258799 |
| put-miR-1095 | ATTGTGAATGATCTGG | 4.006586 | 8.691739 | 0.038403 | 0.258799 |
| put-miR-550 | GCACTCTGGACTCTGAATCC | - 1.460704 | 9.782298 | 0.038501 | 0.258799 |
| put-miR-835 | TTAGGAGTAGGTTACT | 1.888177 | 8.674219 | 0.038693 | 0.258799 |
| put-miR-686 | TGACTGTTTTATATGAGTAA | 1.693385 | 10.42287 | 0.038917 | 0.258993 |
| put-miR-32 | ATCCCGGACGAGCCCCCATTA | - 1.878608 | 11.03059 | 0.039456 | 0.260368 |
| put-miR-151 | TTGAAACTGATATACTGTCTTAGG | - 2.781416 | 7.308382 | 0.039515 | 0.260368 |
| put-miR-726 | GATGGGCTGATTGAGGGAT | 3.117005 | 10.49059 | 0.039816 | 0.261058 |
| put-miR-256 | ATGAGGCTGAGATTGTCC | 1.962535 | 8.272489 | 0.040595 | 0.264863 |
| put-miR-368 | AATGAGAACTTTGAAGGCCGAAG | 2.363048 | 14.35762 | 0.041097 | 0.266314 |
| put-miR-1232 | ACAGACTGTCTTTGGG | 2.165428 | 8.620526 | 0.041263 | 0.266314 |
| put-miR-1219 | GAAATTATTGATCCAGTCACGA | 1.298803 | 9.720145 | 0.041418 | 0.266314 |
| put-miR-1276 | GAAGAGTTGATCCATG | - 1.907277 | 9.309298 | 0.041682 | 0.266725 |
| put-miR-1119 | GTTTTGGGTTGAGTGAGGA | - 2.455642 | 8.629875 | 0.042052 | 0.267807 |
| put-miR-901 | GTCTATTTGGATTATCGTC | 1.728483 | 8.113002 | 0.042255 | 0.267816 |
| put-miR-356 | GTTTGAATTGGGAAGCTGGGGG | - 1.750022 | 8.007003 | 0.043232 | 0.272707 |
| put-miR-856 | AAGGATAGGGAGGTATTT | 2.182062 | 8.400665 | 0.044086 | 0.276787 |
| put-miR-1244 | CAGTTGGTAGAGCACCTGAC | -1.24774 | 10.46658 | 0.045019 | 0.281317 |
| put-miR-476 | GTGGTCAGGTAGAGAA | - 1.919551 | 8.047207 | 0.046618 | 0.288241 |
| put-miR-142 | CAGTATAATTAGGGGTTAATTGTGGGA | 1.249016 | 8.70991 | 0.046684 | 0.288241 |
| put-miR-639 | TGGAGTGTGGATTGGG | 1.474835 | 8.945467 | 0.046777 | 0.288241 |
| put-miR-622 | TGAGTGGTCAATGGGGG | - 1.336255 | 8.590784 | 0.047597 | 0.291931 |
| put-miR-337 | TGGGCTCAAGCTTTCTCT | - 2.374139 | 8.27944 | 0.047999 | 0.291931 |
| put-miR-422 | TTTAGGGTTTTAAGGTGTTA | 1.290525 | 11.98963 | 0.048034 | 0.291931 |
| put-miR-1339 | GGACTGAGAGCTCTTTCTG | 3.091573 | 10.60449 | 0.048448 | 0.293113 |
| put-miR-1356 | GTGCTGCATGGCTGTCA | - 1.345358 | 8.820822 | 0.049044 | 0.295237 |
| put-miR-29 | GAGATTTACGACCTAGG | 1.337942 | 9.63023 | 0.049243 | 0.295237 |
| put-miR-128 | TGGCTCTGACACTAGTA | 2.165955 | 8.272955 | 0.049525 | 0.295593 |
| put-miR-359 | GAAGTGGAAGGACTATGAA | 2.152809 | 8.475685 | 0.050666 | 0.301056 |
| put-miR-1003 | TGGACTTCAGAACAGC | 4.029771 | 7.874318 | 0.050924 | 0.301242 |
| put-miR-238 | AGAGACAGGAACTTTGATTTT | 3.690637 | 8.383615 | 0.051316 | 0.302217 |
| put-miR-961 | TAGCTTGATCCAGTTG | 2.181109 | 9.760789 | 0.052445 | 0.307507 |
| put-miR-751 | AGGCTGAGACTGGACAGAAAGACCT | - 1.317479 | 8.022761 | 0.053486 | 0.312239 |
| put-miR-1184 | GCCTGCCCGGTGCTGGT | 1.707305 | 8.999909 | 0.053825 | 0.312845 |
| put-miR-379 | GCGTGTGCGCGGGAGG | 2.352869 | 7.584474 | 0.054246 | 0.313921 |
| put-miR-361 | CAGGTGAAATCGTGGATGT | 2.224017 | 10.34793 | 0.05535 | 0.318896 |
| put-miR-1111 | TGATGCGTTGGGATGTAGC | - 1.424105 | 8.543294 | 0.055717 | 0.318896 |
| put-miR-938 | TTAGGAAGCTGCTGATT | - 1.317019 | 9.281869 | 0.055825 | 0.318896 |

MicroRNAs ID selected for the second qPCR validation step are shown in **Table 4A,** below.

**Table 4A**

| **microRNAs** | **Gene Number** |
|---|---|
| hsa-let-7f-5p | MIMAT0000067 |
| hsa-miR-1246 | MIMAT0005898 |
| hsa-miR-126-5p | MIMAT0000444 |
| hsa-miR-135b-5p | MIMAT0000758 |
| hsa-miR-21-5p | MIMAT0000076 |
| hsa-miR-425-5p | MIMAT0003393 |
| hsa-miR-497-5p | MIMAT0002820 |
| hsa-miR-148a-3p | MIMAT0000243 |
| hsa-let-7a-5p | MIMAT0000062 |
| hsa-let-7i-5p | MIMAT0000415 |
| hsa-miR-143-3p | MIMAT0000435 |
| hsa-miR-34b-3p | MIMAT0004676 |
| hsa-miR-449a | MIMAT0001541 |
| hsa-miR-144-3p | MIMAT0000436 |
| hsa-miR-144-5p | MIMAT0004600 |
| hsa-miR-16-1-3p | MIMAT0004489 |
| has-miR-934 | MIMAT0004977 |
| hsa-miR-206 | MIMAT0000462 |
| hsa-miR-103a-3p | MIMAT0000101 |
| hsa-miR-671-3p | MIMAT0004819 |
| hsa-miR-92a-3p | MIMAT0000092 |
| hsa-let-7b-5p | MIMAT0000063 |
| hsa-miR-142-3p | MIMAT0000434 |
| hsa-miR-142-5p | MIMAT0000433 |
| hsa-miR-29c-3p | MIMAT0000433 |
| hsa-miR-6748-3p | MIMAT0027397 |
| has-miR-339-5p | MIMAT0000764 |
| has-miR-107 | MIMAT0000104 |
| hsa-miR-126-3p | MIMAT0000445 |

Genome position and sequence of putative microRNAs selected for the second qPCR validation step are shown in **Table 4B,** below.

**Table 4B**

| **putative microRNAs** | **Chr** | **Strand** | **Start** | **Stop** | **Sequence** |
|---|---|---|---|---|---|
| put-miR-1204 | 4 | + | 1661740 | 1661756 | AGGGCTGGGCACGGGGG |
| put-miR-1207 | X | + | 12647513 | 12647530 | GTGAAAAGACATAGGGGG |
| put-miR-444 | 6 | + | 20984875 | 20984897 | CTGAAAAGGGGACGGATTGGGAA |
| put-miR-465 | 2 | + | 29475435 | 29475453 | CCAGTTGTCGTGGGTTTTT |
| put-miR-468 | 5 | + | 62391321 | 62391338 | ATAAGGAACTGCTCTCTC |
| put-miR-6 | 16 | + | 67911172 | 67911188 | GCGGACCTTGCTCAAGG |
| put-miR-958 | 16 | + | 51873548 | 51873564 | ACAGGGCTGTGCAAAAA |
| put-miR-71 | 10 | + | 132351412 | 132351432 | GACCTTGGGTTGGGTCGTGGT |
| put-miR-1003 | X | + | 96505351 | 96505366 | TGGACTTCAGAACAGC |
| put-miR-1080 | 12 | + | 131562933 | 131562948 | TGTGGATTGATGCTCT |
| put-miR-188 | 2 | + | 96290475 | 96290491 | AAATGGCGATACTCAGG |
| put-miR-323 | 12 | + | 54420991 | 54421008 | ATAAAATGGGCGTTGAGG |
| put-miR-92 | 13 | + | 28849073 | 28849090 | AGAGACTGACTTTGAGTA |
| put-miR-209 | 18 | + | 13603389 | 13603404 | GGTCCTCGGATCGGCC |
| put-miR-1084 | 14 | + | 51649977 | 51649993 | AGGACCATTGCGTTGCC |
| put-miR-1306 | 13 | + | 100000004 | 100000021 | ATAACGTCATCTAGTGTG |
| put-miR-325 | 3 | + | 193330821 | 193330843 | TTCAAATCCCACTTCTGACACCA |
| put-miR-1146 | 5 | + | 67892184 | 67892199 | ATAGGAGACTTCTATC |
| put-miR-594 | 17 | + | 46822348 | 46822364 | TGATTTTTTTTGGCGAA |
| put-miR-961 | 7 | + | 150352937 | 150352952 | TAGCTTGATCCAGTTG |
| put-miR-742 | 16 | + | 49579869 | 49579884 | CAGGGCTGTGCTAACT |
| put-miR-469 | 1 | + | 22441119 | 22441140 | GCGGGGGATTAGCTCAGCTGGG |
| put-miR-806 | 16 | + | 964420 | 964437 | CACGAGAGAACGCACACC |
| put-miR-476 | 4 | + | 100244534 | 100244549 | GTGGTCAGGTAGAGAA |
| put-miR-856 | 16 | + | 50873777 | 50873794 | AAGGATAGGGAGGTATTT |
| put-mir-893 | 5 | + | 58317814 | 58317830 | ACCATCCTCTGCTACCA |

Genome position and sequence of other sncRNAs selected for the second qPCR validation step are shown in **Table 4C,** below.

**Table 4C**

| **small non-coding RNAs** | **Chr** | **Strand** | **Start** | **Stop** | **Sequence** |
|---|---|---|---|---|---|
| RNU4-6p | X | - | 16893269 | 16893390 | TATCGTAGCCAATGAGGTTTATCCGA GGCGTGATTATTGCTAATTGAAAA |
| tRNA18Arg CCT | 17 | + | 73030001 | 73030073 | GCACTGGCCTCCTAAGCCAGGGATTG TGGGTTCGAGTCCCACCTGGGGTA |
| U6.428 | 1 | + | 180727858 | 180727953 | AAGATTAGCATGAGGATGACACGCA AATTCGTGAAGCGTTCCATTTCTTT |
| RNU6-45 | 11 | + | 63737942 | 63738048 | GGCCCTTGTGCAAGGATGACACGCA AATTCGTGAAGCGTTCCATATTTTT |
| RNU6-4 | 1 | - | 31970419 | 31970525 | GGCCCCTGCACAGGGATGACACGCA AATTCGTGAAGCGTTCCATATTTTT |
| RNU6-6 | 2 | + | 201694732 | 201694839 | GGCCCCTGTGCAAGGATGACACGCA AATTCGTGAAGCGTTCCATATTTTT |
| RNU6-7 | 3 | + | 194935516 | 194935622 | GGCCCCTGCGCAAGGATGACATGCA AATTCGTGAAGCGTTCCATATTTTT |
| RNU6-73 | 13 | + | 28402900 | 28403006 | GGCCCCTGTGCAAGGATGACATGCA AATTCGTGAAGCGTTCCATATTTTT |
| SNORD3B | 17 | + | 18965225 | 18965440 | CTTCTCTCCGTATTGGGGAGTGAGAG GGAGAGAACGCGGTCTGAGTGGTT |
| tRNA120-AlaAGC | 6 | - | 28626014 | 28626085 | GCGCATGCTTAGCATGCATGAGGTCC CGGGTTCGATCCCCAGCATCTCCA |
| tRNA73Arg CCG | 6 | + | 28849165 | 28849237 | GCGTCTGATTCCGGATCAGAAGATTG AGGGTTCGAGTCCCTTCGTGGTCG |
| U6.168 | 6 | - | 18307204 | 18307310 | ATGGCCCCTGCGCAAGGATGACACG CAAATTTGTGAAGGATTCCATATTT |
| U6.375 | 4 | + | 109573306 | 109573412 | GGCCCCTGTGCAAGAATGACTCGCA AATTCGTGAAGCGTTCCATATTTTT |
| YRNA-684 | 18 | + | 20604559 | 20604666 | GCTTCTTTTACTCTTTCCCTTCATTCT CACTACTGTACCTGATTCGTCTT |
| U6.601 | 19 | - | 39287642 | 39287749 | GGCCCCTGCGCAAGGATGACATGCA AATTTGTGAAGTGTTCCATATTTTT |
| YRNA-255 | 17 | + | 80375102 | 80375197 | GUGUCACCAACGUUGGUAUACAACC CCCCACAACUAAAUUUGACUGGCUU |
| tRNA9-TyrGTA | 7 | + | 149255133 | 149255205 | CTTTTTGACTGTAGAGCAAGAGGTCC CTGGTTCAAATCCAGGTTCTCCCT |
| U2.3 | 1 | + | 150209315 | 150209504 | TCACTTCACGCATCGATCTGGTATTG CAGTACCTCCAGGAACAGTGCACC |
| U4.64 | 9 | + | 36267780 | 36267919 | GTATCGTAGCCAATGAGGTTTATCCA AGGTGCGATTATTGCTAATTGAAA |
| SNORA57 | 10 | + | 27077946 | 27078086 | TGCTGGCGGCTTCCCATCCGCTGGTT CTATCCTCAAACGCCGGGACACCG |
| UC022CJG 1 | Y | + | 10037846 | 10037870 | CATTGATCATCGACACTTCGAACGCA CTTG |
| tRNA27-MetCAT | 6 | + | 26766444 | 26766516 | GCGTCAGTCTCATAATCTGAAGGTCC TGAGTTCGAGCCTCAGAGAGGGCA |
| tRNA8-ThrAGT | 17 | + | 8090478 | 8090551 | GCGCCTGTCTAGTAAACAGGAGATCC TGGGTTCGAATCCCAGCGGTGCCT |
| tRNA2-LeuTAA | 4 | - | 156384978 | 156385052 | GCATAAAACTTAAAATTTTATAATCA GAGGTTCAACTCCTCTTCTTAACA |
| tRNA9-TyrGTA | 7 | + | 149255133 | 149255205 | CTTTTTGACTGTAGAGCAAGAGGTCC CTGGTTCAAATCCAGGTTCTCCCT |
| YRNA-245 | 2 | - | 25919945 | 25920057 | GTCTTTGTTGAACTCTTTCCCTCCTTC TCATTACTGTACTTGACCAGTCT |
| U6.1249 | 1 | + | 67661823 | 67661926 | GATGGCATGACCCCTGATCAAGGAC GGCATGCAAATTTGTGAAGTATTTC |

SncRNAs differentially expressed in group C vs U+M at the 3 time points are shown in **Table 5,** below. This table also includes: AUC, CI, count, CT average, SD, ddcq, fold change and power analysis for each individual biomarker and STEPWISE analysis.

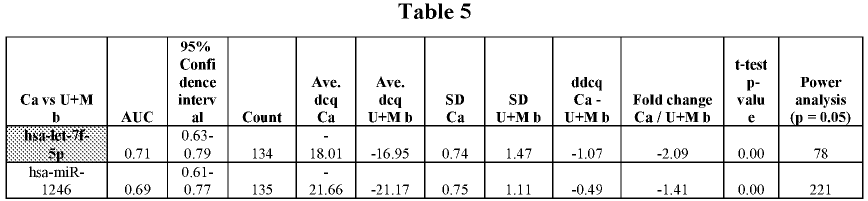

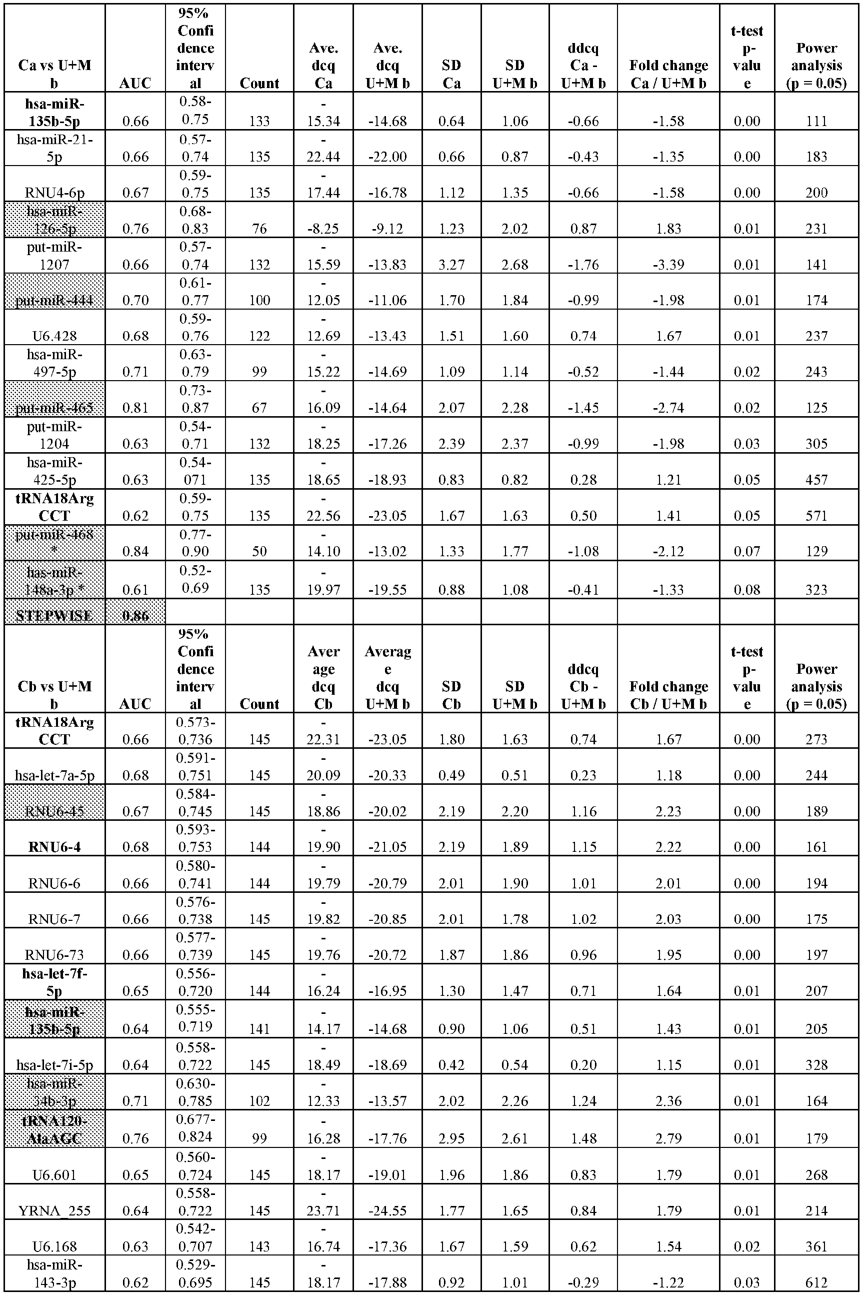

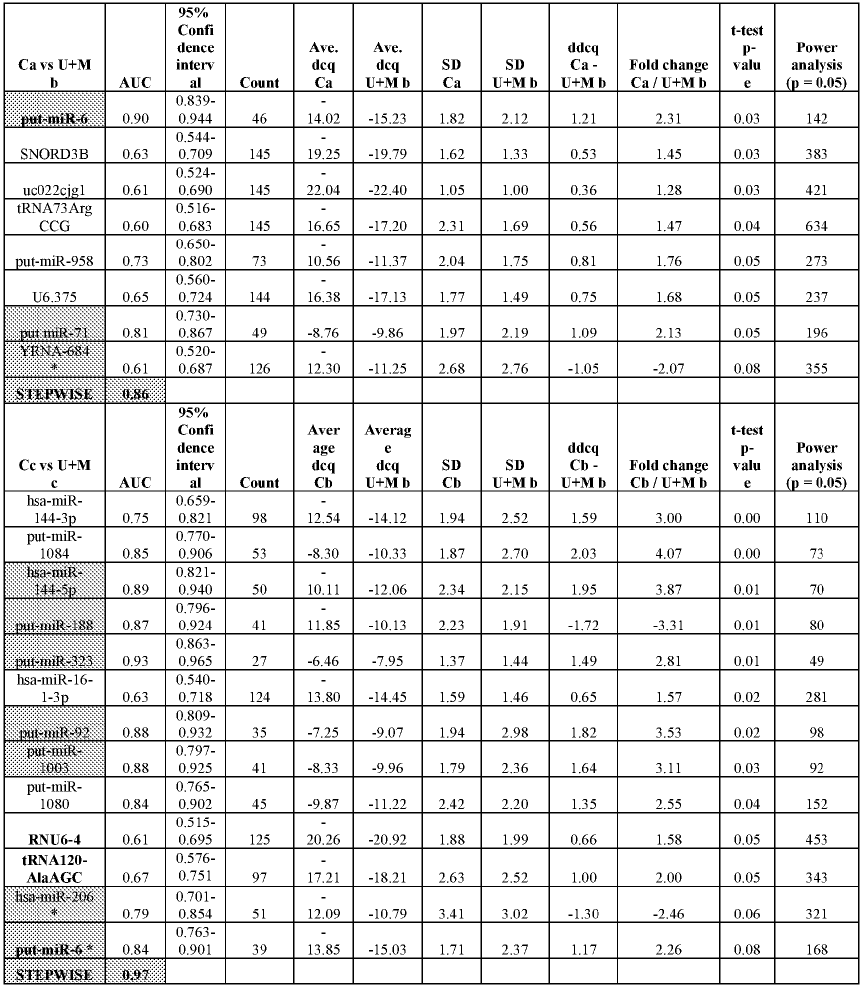

SncRNAs differentially expressed in group C vs U at the 3 time points are shown in **Table 6,** below. This table also includes: AUC, CI, count, CT average, SD, ddcq, fold change and power analysis for each individual biomarker and STEPWISE analysis.

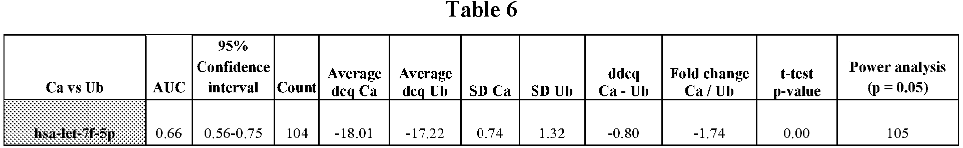

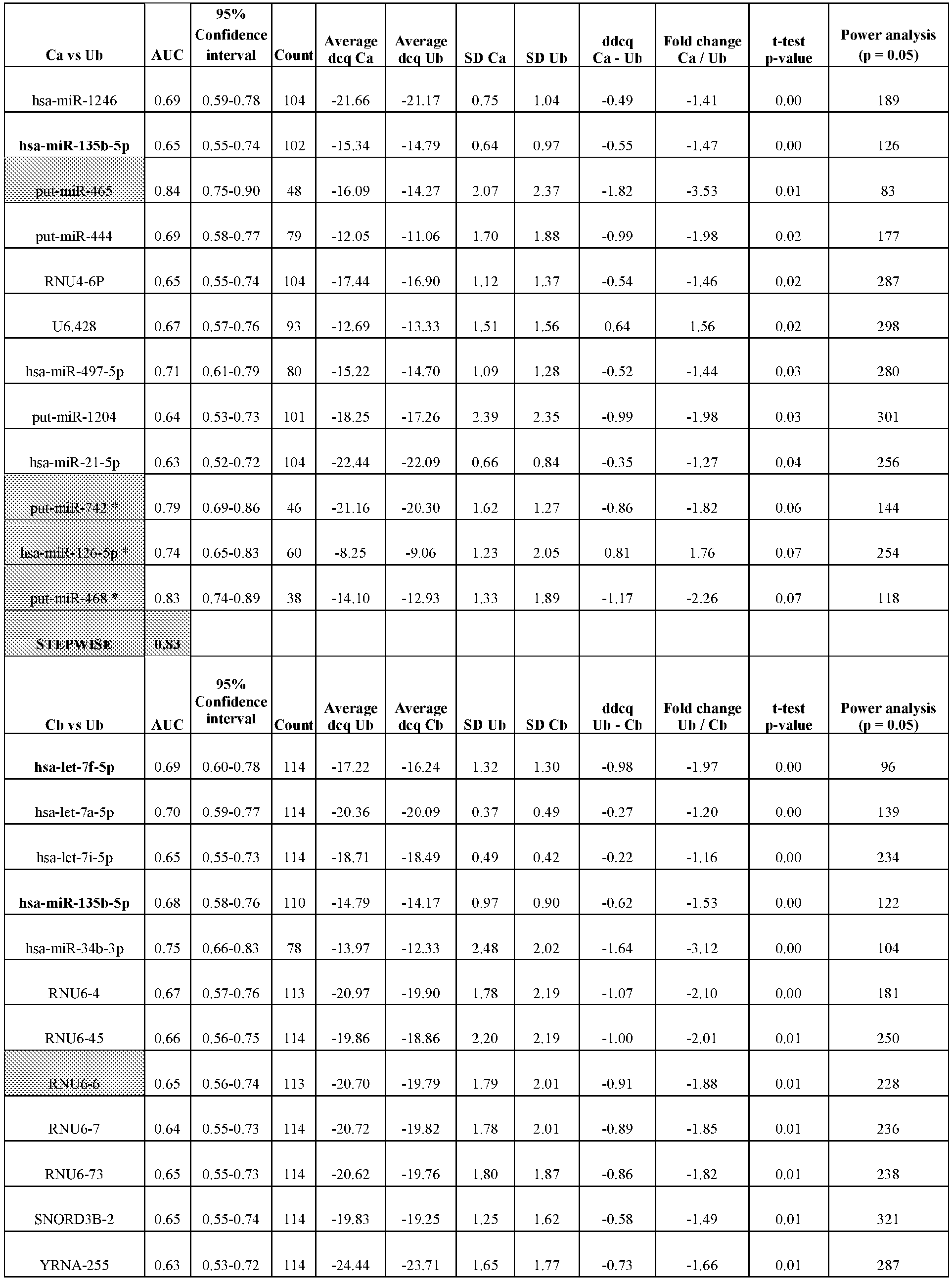

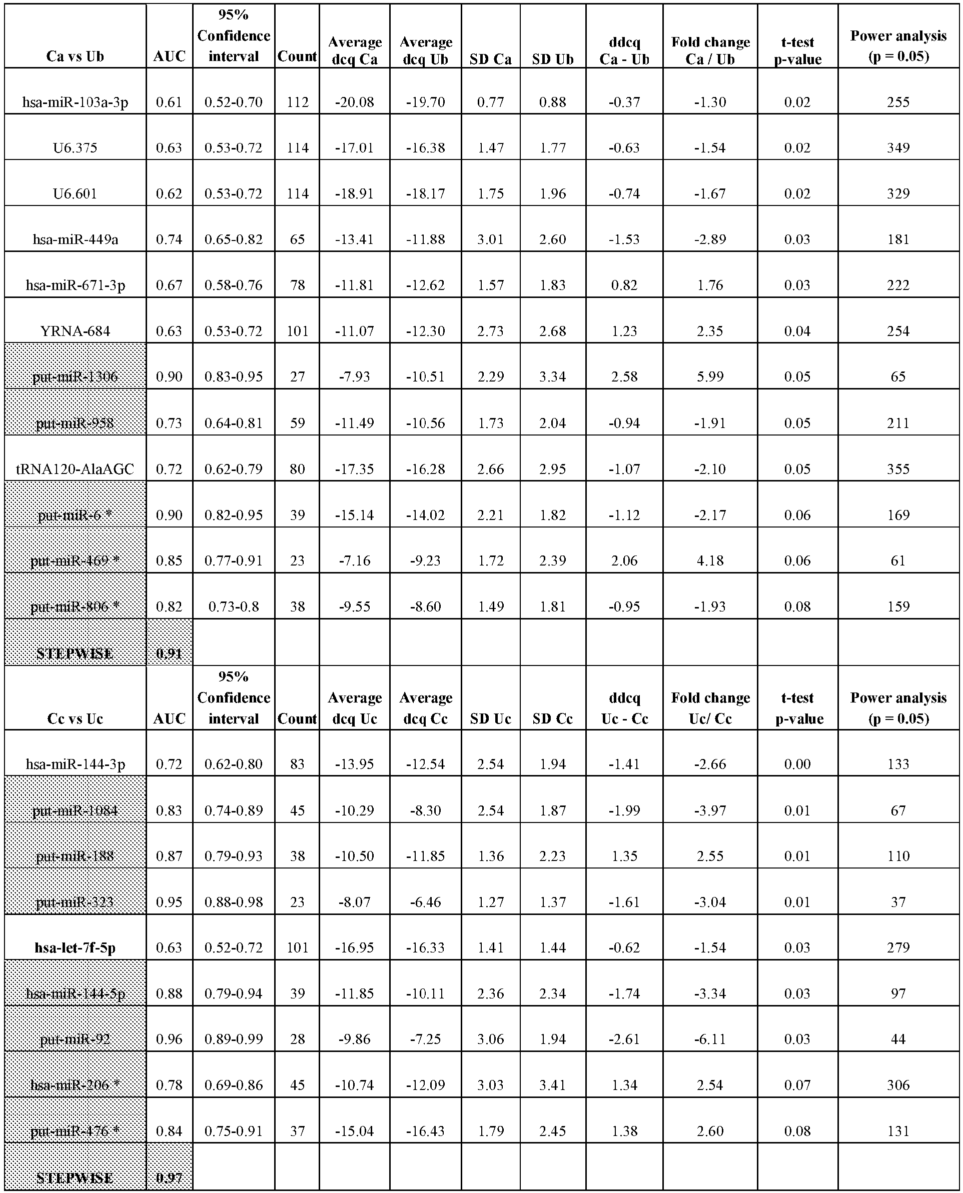

SncRNAs differentially expressed in group C vs B at the 3 time ponts are shown in **Table 7,** below. This table also includes: count, CT average, SD, ddcq, fold change and power analysis for each individual biomarker.

**Table 7**

| **Ca vs B** | **Count** | **Average dcq Ca** | **Average dcq B** | **SD Ca** | **SD B** | **ddcq Ca-B** | **Fold change Ca/B** | **Pair sample test Sig. (2-tailed)** | **Power analysis (p = 0.05)** |
|---|---|---|---|---|---|---|---|---|---|
| **hsa-miR-1246** | 219 | -21.66 | -20.79 | 0.75 | 1.04 | -0.87 | -1.83 | 0.00 | 69 |
| **put-miR-1207** | 207 | -15.59 | -12.28 | 3.27 | 2.22 | -3.30 | -9.88 | 0.00 | 31 |
| **RNU4-6p** | 219 | -17.44 | -16.85 | 1.12 | 1.09 | -0.59 | -1.51 | 0.00 | 178 |
| **hsa-miR-143-3p** | 114 | -18.00 | -18.17 | 0.99 | 0.92 | 0.16 | 1.12 | 0.00 | 1821 |
| **hsa-miR-34b-3p** | 182 | -13.93 | -12.33 | 1.51 | 1.87 | -1.60 | -3.04 | 0.00 | 69 |
| hsa-miR-449a | 155 | -13.48 | -11.38 | 1.89 | 2.62 | -2.10 | -4.28 | 0.00 | 75 |
| **U4.64** | 107 | -11.33 | -11.90 | 236 | 2.64 | 0.57 | 1.49 | 0.00 | 991 |
| **hsa-let-7b-5p** | 219 | -20.07 | -20.26 | 0.58 | 0.58 | 0.19 | 1.14 | 0.00 | 480 |
| **hsa-miR-142-3p** | 114 | -23.48 | -23.56 | 0.77 | 0.87 | 0.08 | 1.06 | 0.00 | 5191 |
| **hsa-miR-142-5p** | 219 | -20.83 | -20.14 | 0.90 | 1.03 | -0.69 | -1.61 | 0.00 | 114 |
| **hsa-miR-29c-3p** | 219 | -19.93 | -19.74 | 0.58 | 0.58 | -0.19 | -1.14 | 0.00 | 480 |
| put-miR-1146 | 122 | -12.59 | -11.17 | 2.39 | 2.97 | -1.43 | -2.69 | 0.00 | 210 |
| **tRNA9-TyrGTA** | 206 | -13.04 | -11.65 | 2.42 | 2.07 | -1.39 | -2.61 | 0.00 | 127 |
| put-miR-444 | 133 | -12.05 | -10.06 | 1.70 | 227 | -1.99 | -3.96 | 0.01 | 64 |
| has-miR-148a-3p | 219 | -19.97 | -19.67 | 0.88 | 0.66 | -0.29 | -1.22 | 0.01 | 305 |
| U2.3 | 219 | -27.18 | -27.13 | 1.32 | 1.31 | -0.05 | -1.03 | 0.01 | 43231 |
| tRNA27-MetCAT | 112 | -18.60 | -18.36 | 1.48 | 1.88 | -0.24 | -1.18 | 0.01 | 2548 |
| **tRNA2-LeuTAA** | 219 | -16.87 | -17.65 | 1.30 | 1.47 | 0.78 | 1.71 | 0.01 | 181 |
| **YRNA-245** | 219 | -15.50 | -14.70 | 1.42 | 1.36 | -0.80 | -1.74 | 0.01 | 155 |
| **hsa-let-7f-5p** | 219 | -18.01 | -17.67 | 0.74 | 1.27 | -0.35 | -1.27 | 0.02 | 598 |
| **hsa-**miR-6748-3p | 94 | -14.95 | -12.10 | 3.29 | 3.04 | -2.85 | -7.20 | 0.02 | 64 |
| **put-miR-468** | 64 | -14.10 | -12.25 | 1.33 | 1.70 | -1.85 | -3.60 | 0.05 | 44 |
| put-miR-594 | 190 | -15.15 | -13.30 | 4.79 | 3.42 | -1.85 | -3.61 | 0.05 | 212 |

| **Cb vs B** | **Count** | **Average dcq Cb** | **Average dcq B** | **SD Cb** | **SD B** | **ddcq Cb - B** | **Fold change Cb/B** | **Pair sample test Sig. (2-tailed)** | **Power analysis (p = 0.05)** |
|---|---|---|---|---|---|---|---|---|---|
| **hsa-miR-135b-5p** | 226 | -14.17 | -15.45 | 0.90 | 0.96 | 1.28 | 2.43 | 0.00 | 31 |
| hsa-miR-425-5p | 229 | -18.87 | -18.37 | 0.71 | 0.86 | -0.50 | -1.41 | 0.00 | 146 |
| hsa-let-7a-5p | 229 | -20.09 | -20.65 | 0.49 | 0.41 | 0.56 | 1.47 | 0.00 | 33 |
| **hsa-miR-143-3p** | 229 | -18.17 | -17.60 | 0.92 | 1.18 | -0.57 | -1.48 | 0.00 | 206 |
| **YRNA-684** | 196 | -12.30 | -10.58 | 2.68 | 2.12 | -1.72 | -3.29 | 0.00 | 93 |
| **U4.64** | 221 | -11.90 | -10.76 | 2.64 | 1.89 | -1.14 | -2.20 | 0.00 | 176 |
| **hsa-miR-142-3p** | 229 | -23.56 | -22.94 | 0.87 | 1.11 | -0.63 | -1.55 | 0.00 | 151 |
| **hsa-miR-142-5p** | 229 | -20.82 | -20.14 | 0.97 | 1.03 | -0.68 | -1.61 | 0.00 | 117 |

| **Ca vs B** | **Count** | **Average dcq Ca** | **Average dcq B** | **SD Ca** | **SD B** | **ddcq Ca-B** | **Fold change Ca/ B** | **Pair sample test Sig. (2-tailed)** | **Power analysis (p = 0.05)** |
|---|---|---|---|---|---|---|---|---|---|
| **tRNA9-TyrGTA** | 213 | -13.06 | -11.65 | 2.84 | 2.07 | -1.41 | -2.65 | 0.00 | 137 |
| **YRNA-245** | 229 | -15.18 | -14.70 | 1.83 | 1.36 | -0.48 | -1.40 | 0.00 | 493 |
| put-miR-961 | 144 | -12.72 | -11.03 | 3.34 | 2.55 | -1.68 | -3.21 | 0.00 | 143 |
| **hsa-let-7f-5p** | 229 | -16.24 | -17.67 | 1.30 | 1.27 | 1.43 | 2.69 | 0.01 | 44 |
| **hsa-miR-1246** | 229 | -21.39 | -20.79 | 1.33 | 1.04 | -0.60 | -1.52 | 0.01 | 179 |
| hsa-miR-126-5p | 144 | -9.35 | -8.15 | 1.87 | 1.60 | -1.20 | -2.30 | 0.01 | 101 |
| hsa-miR-144-3p | 208 | -14.19 | -13.70 | 2.60 | 2.06 | -0.49 | -1.41 | 0.01 | 1021 |
| **hsa-let-7b-5p** | 229 | -20.21 | -20.26 | 0.90 | 0.58 | 0.05 | 1.04 | 0.01 | 8840 |
| **hsa-miR-29c-3p** | 229 | -19.79 | -19.74 | 0.90 | 0.58 | -0.05 | -1.04 | 0.01 | 8840 |
| SNORD3B | 229 | -19.25 | -20.20 | 1.62 | 1.03 | 0.94 | 1.92 | 0.02 | 86 |
| hsa-miR-671-3p | 157 | -12.62 | -11.09 | 1.83 | 1.35 | -1.53 | -2.89 | 0.02 | 49 |
| UC022CJG1 | 229 | -22.04 | -22.64 | 1.05 | 0.94 | 0.60 | 1.51 | 0.02 | 139 |
| **tRNA2-LeuTAA** | 227 | -16.87 | -17.65 | 1.99 | 1.47 | 0.78 | 1.72 | 0.02 | 220 |
| hsa-miR-339-5p | 229 | -15.89 | -15.50 | 1.60 | 1.30 | -0.39 | -1.31 | 0.02 | 639 |
| **hsa-miR-21-5p** | 229 | -21.86 | -22.45 | 0.99 | 0.74 | 0.59 | 1.51 | 0.03 | 98 |
| **hsa-miR-34b-3p** | 180 | -12.33 | -12.33 | 2.02 | 1.87 | 0.00 | -1.00 | 0.03 | 11336078 |
| **U6.168** | 228 | -16.74 | -16.82 | 1.67 | 1.56 | 0.07 | 1.05 | 0.03 | 25072 |
| hsa-miR-16-1-3p | 226 | -14.16 | -13.71 | 1.41 | 1.49 | -0.45 | -1.36 | 0.03 | 564 |
| **put-miR-1207** | 217 | -13.48 | -12.28 | 3.36 | 2.22 | -1.20 | -2.30 | 0.04 | 234 |
| **put-miR-468** | 69 | -13.67 | -12.25 | 1.68 | 1.70 | -1.42 | -2.67 | 0.04 | 77 |
| put-miR-893 | 229 | -28.04 | -27.27 | 2.65 | 1.94 | -0.77 | -1.71 | 0.04 | 396 |
| SNORA57 | 229 | -20.82 | -20.63 | 1.15 | 1.06 | -0.20 | -1.15 | 0.04 | 1593 |

| **Cc vs B** | **Count** | **Average dcq Cc** | **Average dcq B** | **SD Cc** | **SD B** | **ddcq Cc - B** | **Fold change Cc / B** | **Pair sample test Sig. (2-tailed)** | **Power analysis (p = 0.05)** |
|---|---|---|---|---|---|---|---|---|---|
| **hsa-miR-135b-5p** | 228 | -14.95 | -15.45 | 0.96 | 0.96 | 0.50 | 1.41 | 0.00 | 194 |
| **hsa-miR-1246** | 231 | -21.19 | -20.79 | 1.21 | 1.04 | -0.40 | -1.32 | 0.01 | 378 |
| **hsa-miR-21-5p** | 231 | -21.88 | -22.45 | 0.92 | 0.74 | 0.57 | 1.48 | 0.01 | 101 |
| **hsa-let-7f-5p** | 231 | -16.33 | -17.67 | 1.44 | 1.27 | 1.34 | 2.52 | 0.02 | 52 |
| U6.428 | 215 | -13.10 | -12.76 | 1.53 | 1.69 | -0.34 | -1.26 | 0.02 | 1253 |
| YRNA-684 | 201 | -11.24 | -10.58 | 3.03 | 2.12 | -0.66 | -1.58 | 0.02 | 681 |
| **U4.64** | 231 | -16.98 | -16.85 | 1.49 | 1.09 | -0.13 | -1.09 | 0.02 | 4432 |
| U6.1249 | 160 | -10.16 | -8.96 | 3.17 | 2.18 | -1.20 | -2.30 | 0.02 | 223 |
| **RNU4-6P** | 231 | -16.98 | -16.85 | 1.49 | 1.08 | -0.13 | -1.09 | 0.03 | 4432 |
| U6.168 | 231 | -16.78 | -16.82 | 1.57 | 1.56 | 0.04 | 1.03 | 0.04 | 84454 |
| hsa-miR-92a-3p | 231 | -20.47 | -20.50 | 0.68 | 0.46 | 0.03 | 1.02 | 0.04 | 11846 |
| put-miR-209 | 97 | -8.94 | -8.42 | 2.79 | 2.89 | -0.52 | -1.43 | 0.04 | 1580 |

SncRNAs differentially expressed in group C vs M at the 3 time points are shown in **Table 8,** below. This table also includes: AUC, CI, count, CT average, SD, ddcq, fold change and power analysis for each individual biomarker and STEPWISE analysis.

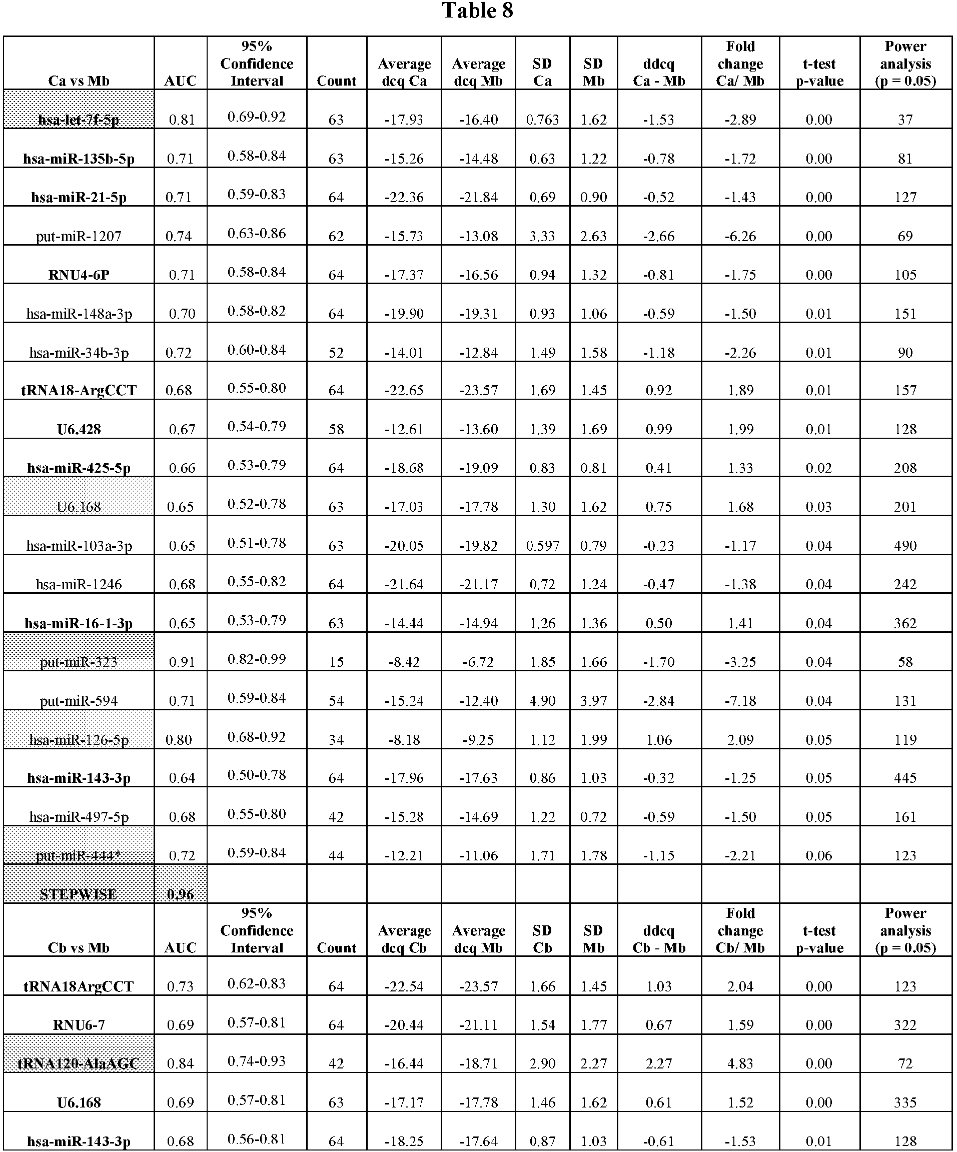

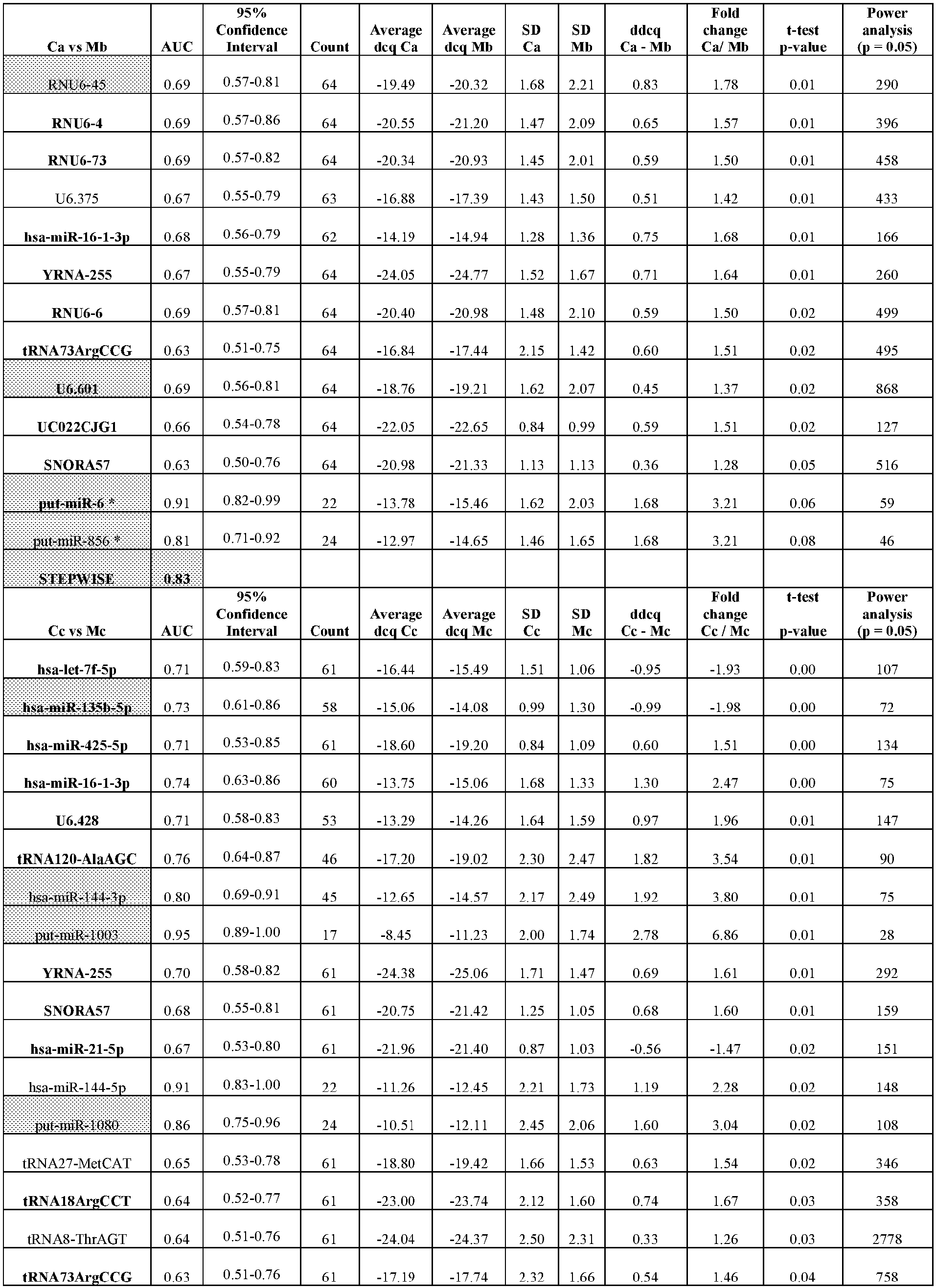

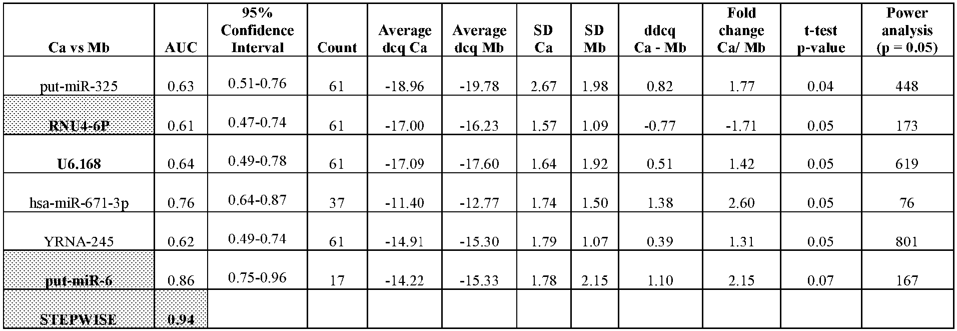

### First Set of Examples of Embodiments of the Present Disclosure

A1. A method of diagnosing and treating traumatic brain injury (TBI) in a human subject in need thereof, the method comprising:
obtaining a saliva sample from the subject;
contacting the saliva sample with a probe comprising a nucleic acid able to bind to at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352;
determining an amount of the at least one RNA biomarker in the saliva sample;
identifying the subject as having TBI where the amount of the at least one RNA biomarker is increased or decreased relative to a predetermined threshold value or relative to the amount of the RNA biomarker in a control sample; and
treating the subject identified as having TBI according to one or more of the following:
   subjecting the subject to a verbal, cognitive, motor, or optical test, or any combination of the foregoing;
   subjecting the subject to diagnostic imaging in the form of a CT or MRI, or a combination thereof; and/or
   administering to the subject one or more neuroprotective therapies.

A2. The method according to claim A1 further comprising identifying the human subject as being fit for normal activity after undergoing successful treatment for TBI.

B1. A method of diagnosing and/or monitoring traumatic brain injury (TBI) in a subject, the method comprising determining a level of at least one RNA biomarker in a saliva sample obtained from the subject, wherein the at least one RNA biomarker is selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352, or any combination thereof.

B2. The method of claim B1, wherein either an upregulated level or a downregulated level of the at least one RNA biomarker is indicative of TBI.

B3. The method of claim B1, wherein the subject is diagnosed as having TBI if the level of the at least one RNA biomarker is either above or below a predetermined threshold or increased or decreased relative to a control.

B4. The method according to claim B1 further comprising identifying the human subject as being fit for normal activity after undergoing successful treatment for TBI.

B5. The method of any one of claims B1-B4, wherein the at least one RNA biomarker further comprises one or more miRNA selected from the group consisting of hsa-miR-143-3p and hsa-miR-135b-5p.

C1. A sensor element for a detection system for diagnosing and/or monitoring TBI, the sensor element comprising a substrate functionalized with a probe specific for at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352.

C2. The sensor element of claim C1, wherein the probe comprises a nucleic acid able to bind to the at least one RNA biomarker.

C3. The sensor element of claim C1 or C2, wherein the probe comprises a nucleic acid having at least 70% identity with a sequence which is the complement of the sequence of the target RNA biomarker.

C4. The sensor element of claim C1 or C2, wherein the probe comprises a nucleic acid having at least 70% identity with a sequence which is the complement of SEQ ID NO: 70, 71, 72, 73, 74, 75, 76, 77, 78, 79, 80, 81, 82, 83, 84, 85, 86, 87, 88, 89, 90, 91, 92, 93, 94, 95, 96, 97, 98, and 99.

C5. The sensor element of any one of claims C1-C4, wherein the at least one RNA biomarker further comprises one or more miRNA selected from the group consisting of hsa-miR-143-3p and hsa-miR-135b-5p.

D1. A detection system for diagnosing and/or monitoring TBI, comprising a sensor element according to the present disclosure, and a detection device capable of detecting the binding of a target RNA biomarker to the probe.

D2. The detection system according to claim D1, further comprising means to determine whether the target RNA biomarker is upregulated or downregulated.

E1. A method for determining a course of action for a subject suspected of having TBI, comprising applying a saliva sample obtained from the subject to a detection system according to the present disclosure , and if an upregulated level or a downregulated level of the at least one RNA biomarker is detected, providing a treatment for TBI.

F1. A method of treating a subject with suspected TBI, the method comprising
determining whether an upregulated level or a downregulated level of at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352 is detectable in a saliva sample obtained from the subject, and
if an upregulated level or a downregulated level of at least one RNA biomarker is detected, providing treatment for TBI to the subject.

F2. The method of claim F1, wherein the at least one RNA biomarker further comprises one or more miRNA selected from the group consisting of hsa-miR-143-3p and hsa-miR-135b-Sp.

G1. A method of detecting an RNA biomarker in a saliva sample, the method comprising
obtaining a saliva sample from a human subject,
contacting the saliva sample with at least one oligonucleotide primer complementary to at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352;
amplifying the at least one RNA biomarker using a polymerase chain reaction; and
detecting the amplified RNA biomarker.

G2. The method of claim G1, wherein the at least one RNA biomarker further comprises one or more miRNA selected from the group consisting of hsa-miR-143-3p and hsa-miR-135b-Sp.

H1. A kit for use in a method of diagnosing and/or monitoring traumatic brain injury (TBI) in saliva from a human subject, the kit comprising at least one probe specific for at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352.

H2. The kit of claim H1, wherein the probe comprises a nucleic acid able to bind to the at least one RNA biomarker.

H3. The kit of claim H1 or H2, wherein the probe comprises a nucleic acid having at least 70% identity with a sequence which is the complement of the sequence of the target RNA biomarker.

H4. The kit of claim H1 or H2, wherein the probe comprises a nucleic acid having at least 70% identity with a sequence which is the complement of SEQ ID NO: 70, 71, 72, 73, 74, 75, 76, 77, 78, 79, 80, 81, 82, 83, 84, 85, 86, 87, 88, 89, 90, 91, 92, 93, 94, 95, 96, 97, 98, and 99.

H5. The kit of any one of claims H1-H4, wherein the at least one RNA biomarker further comprises one or more miRNA selected from the group consisting of hsa-miR-143-3p and hsa-miR-135b-5p.

I1. A composition for use in a method of diagnosing and/or monitoring traumatic brain injury (TBI) in saliva from a human subject, the composition comprising at least one probe specific for at least one RNA biomarker selected from the group consisting of Y_RNA.255, RNU6-7, RNU6-4, RNU6-6, RNU6-73, RNU6-45, U6.375, put-miR-1207, U6.428, put-miR-742, hsa-miR-6748-3p, put-miR-6, put-miR-410, put-miR-476, put-miR-293, hsa-miR-34b-3p, hsa-miR-1271-5p, hsa-miR-449a, put-miR-806, put-miR-71, put-miR-468, put-miR-1306, put-miR-1146, put-miR-1084, put-miR-92, put-miR-209, put-miR-961, U6.1249, put-miR-188, and put-miR-1352.

I2. The composition of claim I1, wherein the probe comprises a nucleic acid able to bind to the at least one RNA biomarker.

I3. The composition of claim I1 or I2, wherein the probe comprises a nucleic acid having at least 70% identity with a sequence which is the complement of the sequence of the target RNA biomarker.

I4. The composition of claim I1 or I2, wherein the probe comprises a nucleic acid having at least 70% identity with a sequence which is the complement of SEQ ID NO: 70, 71, 72, 73, 74, 75, 76, 77, 78, 79, 80, 81, 82, 83, 84, 85, 86, 87, 88, 89, 90, 91, 92, 93, 94, 95, 96, 97, 98, and 99.

I5. The composition of any one of claims 11-14, wherein the at least one RNA biomarker further comprises one or more miRNA selected from the group consisting of hsa-miR-143-3p and hsa-miR-135b-5p.

### Second Set of Examples of Embodiments of the Present Disclosure

A1. A method of diagnosing and treating traumatic brain injury (TBI) in a human subject in need thereof, the method comprising:
obtaining a saliva sample from the subject;
contacting the saliva sample with a probe comprising a nucleic acid able to bind to at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof;
determining an amount of the at least one RNA biomarker in the saliva sample;
identifying the subject as having TBI where the amount of the at least one RNA biomarker is increased or decreased relative to a predetermined threshold value or relative to the amount of the RNA biomarker in a control sample; and
treating the subject identified as having TBI according to one or more of the following:
   subjecting the subject to a verbal, cognitive, motor, or optical test, or any combination of the foregoing;
   subjecting the subject to diagnostic imaging in the form of a CT or MRI, or a combination thereof; and/or
   administering to the subject one or more neuroprotective therapies.

A2. The method of claim A1, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

A3. The method according to claim A1 further comprising identifying the human subject as being fit for normal activity after undergoing successful treatment for TBI.

B1. A method of diagnosing and/or monitoring traumatic brain injury (TBI) in a subject, the method comprising determining a level of at least one RNA biomarker in a saliva sample obtained from the subject, wherein the at least one RNA biomarker is:
a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

B2. The method of claim B1, wherein either an upregulated level or a downregulated level of the at least one RNA biomarker is indicative of TBI.

B3. The method of claim B1, wherein the subject is diagnosed as having TBI if the level of the at least one RNA biomarker is either above or below a predetermined threshold or increased or decreased relative to a control.

B4. The method of any one of claims B1-B3, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

B5. The method according to claim B1 further comprising identifying the human subject as being fit for normal activity after undergoing successful treatment for TBI.

B6. The method of any one of claims B1-B7, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

C1. A sensor element for a detection system for diagnosing and/or monitoring TBI, the sensor element comprising a substrate functionalized with a probe specific for at least one RNA biomarker:
a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

C2. The sensor element of claim C1, wherein the probe comprises a nucleic acid able to bind to the at least one RNA biomarker.

C3. The sensor element of claim C1 or C2, wherein the probe comprises a nucleic acid having at least 70% identity with a sequence which is the complement of the sequence of the target RNA biomarker.

C4. The sensor element of claim C1 or C2, wherein the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.

C5. The sensor element of any one of claims C1-C4, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

D1. A detection system for diagnosing and/or monitoring TBI, comprising a sensor element according to the present disclosure, and a detection device capable of detecting the binding of a target RNA biomarker to the probe.

D2. The detection system according to claim D1, further comprising means to determine whether the target RNA biomarker is upregulated or downregulated.

E1. A method for determining a course of action for a subject suspected of having TBI, comprising applying a saliva sample obtained from the subject to a detection system according to the present disclosure, and if an upregulated level or a downregulated level of the at least one RNA biomarker is detected, providing a treatment for TBI.

F1. A method of treating a subject with suspected TBI, the method comprising
determining whether an upregulated level or a downregulated level of at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof,
is detectable in a saliva sample obtained from the subject, and
if an upregulated level or a downregulated level of at least one RNA biomarker is detected, providing treatment for TBI to the subject.

F2. The method of claim F1, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

G1. A method of detecting an RNA biomarker in a saliva sample, the method comprising
obtaining a saliva sample from a human subject,
contacting the saliva sample with at least one oligonucleotide primer complementary to at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof;
amplifying the at least one RNA biomarker using a polymerase chain reaction; and
detecting the amplified RNA biomarker.

G2. The method of claim G1, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

H1. A kit for use in a method of diagnosing and/or monitoring traumatic brain injury (TBI) in saliva from a human subject, the kit comprising at least one probe specific for at least one RNA biomarker:
a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

H2. The kit of claim H1, wherein the probe comprises a nucleic acid able to bind to the at least one RNA biomarker.

H3. The kit of claim H1 or H2, wherein the probe comprises a nucleic acid having at least 70% identity with a sequence which is the complement of the sequence of the target RNA biomarker.

H4. The kit of claim H1 or H2, wherein the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.

H5. The kit of any one of claims H1-H4, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

I1. A composition for use in a method of diagnosing and/or monitoring traumatic brain injury (TBI) in saliva from a human subject, the composition comprising at least one probe specific for at least one RNA biomarker:
a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.

I2. The composition of claim I1, wherein the probe comprises a nucleic acid able to bind to the at least one RNA biomarker.

I3. The composition of claim I1 or I2, wherein the probe comprises a nucleic acid having at least 70% identity with a sequence which is the complement of the sequence of the target RNA biomarker.

I4. The composition of claim I1 or I2, wherein the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.

I5. The composition of any one of claims 11-14, wherein the at least one RNA biomarker further comprises one or more miRNA:
a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.

Other advantages that are obvious and/or inherent to the disclosure will be evident to one skilled in the art. It will be understood that certain features and subcombinations are of utility and may be employed without reference to other features and subcombinations. This is contemplated by and is within the scope of the claims. Since many possible embodiments may be made of the disclosure without departing from the scope thereof, it is to be understood that all matter herein set forth or shown in the accompanying drawings is to be interpreted as illustrative and not in a limiting sense.

### Embodiments of the invention

1. A method of diagnosing and/or monitoring traumatic brain injury (TBI) in a subject, the method comprising determining a level of at least one RNA biomarker in a saliva sample obtained from the subject, wherein the at least one RNA biomarker: a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, and YRNA-255, or any combination thereof.
2. The method of embodiment 1, wherein either an upregulated level or a downregulated level of the at least one RNA biomarker is indicative of TBI.
3. The method of embodiment 1, wherein the subject is diagnosed as having TBI if the level of the at least one RNA biomarker is either above or below a predetermined threshold or increased or decreased relative to a control.
4. The method of embodiment 1, wherein the saliva sample is obtained during a period of time after injury selected from immediately after the injury to any period post-injury.
5. The method of embodiment 4, wherein the saliva sample is obtained during a period of time after injury selected from immediately after the injury until the expression level of at least one of the RNA biomarkers returns to the predetermined threshold value or to the amount of the RNA biomarker in the control sample.
6. The method of embodiment 4, wherein the saliva sample is obtained during a period of time after injury selected from immediately after the injury to up to 1 year, up to 10 months, up to 8 months, up to 6 months, up to 5 months, up to 4 months, up to 3 months, up to 2 months, up to 1 month, up to 25 days, up to 20 days, up to 15 days, up to 7 days, up to 5 days, up to 3 days, up to 2 days, and/or up to 24 hours post-injury.
7. The method of embodiment 6, wherein the saliva sample is obtained during a period of time after injury selected from immediately after the injury to 15 days, from 1 hour to 15 days, from 24 hours to 15 days, from 24 hours to 7 days, and from 2 to 5 days.
8. The method of embodiment 1, further comprising obtaining one or more additional saliva samples from the subject at one or more additional times after the injury and repeating the detecting and amplifying steps for each additional sample.
9. The method of embodiment 8, wherein the one or more additional saliva sample is obtained during a period of time after injury selected from immediately after the injury to any period post-injury.
10. The method of embodiment 8, wherein the one or more additional saliva sample is obtained during a period of time after injury selected from immediately after the injury until the expression level of at least one of the RNA biomarkers returns to the predetermined threshold value or to the amount of the RNA biomarker in the control sample.
11. The method of embodiment 8, wherein the one or more additional saliva sample is obtained during a period of time after injury selected from immediately after the injury to up to 1 year, up to 10 months, up to 8 months, up to 6 months, up to 5 months, up to 4 months, up to 3 months, up to 2 months, up to 1 month, up to 25 days, up to 20 days, up to 15 days, up to 7 days, up to 5 days, up to 3 days, up to 2 days, and/or up to 24 hours post-injury.
12. The method of embodiment 11, wherein the one or more additional saliva sample is obtained during a period of time after injury selected from immediately after the injury to 15 days, from 1 hour to 15 days, from 24 hours to 15 days, from 24 hours to 7 days, and from 2 to 5 days.
13. The method of embodiment 8, wherein the one or more additional saliva samples is obtained at day 1, 1.5, 2, 3, 4, 5, 6, 7, 8, 9, 10, 11, 12, 13, 14, or 15 after the injury.
14. The method of any one of embodiments 1-13, wherein the detecting an amount of the at least one RNA biomarker is performed using a PCR-based assay, a light array assay, a laminar flow chip assay, or any assay suitable for detecting that at least one RNA biomarker.
15. The method of embodiment 14, wherein when using the PCR-based assay the predetermined threshold is equivalent to a fold change of 1.5 or more using the 2-delta delta CT (2-ΔΔCT) method.
16. The method of embodiment 14, wherein the predetermined threshold is equivalent to a fold change of 2 or more using the 2-delta delta CT (2-ΔΔCT) method.
17. The method of any one of embodiments 1-16, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
18. The method according to embodiment 33 further comprising identifying the human subject as being fit for normal activity after undergoing successful treatment for TBI.
19. The method of any one of embodiments 1-18, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
20. A sensor element for a detection system for diagnosing and/or monitoring TBI, the sensor element comprising a substrate functionalized with a probe specific for at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.
21. The sensor element of embodiment 20, wherein the probe comprises a nucleic acid able to bind to the at least one RNA biomarker.
22. The sensor element of embodiment 20 or 21, wherein the probe comprises a nucleic acid having at least 70% identity with a sequence which is the complement of the sequence of the target RNA biomarker.
23. The sensor element of embodiment 20 or 21, wherein the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.
24. The sensor element of any one of embodiments 21-23, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
25. A detection system for diagnosing and/or monitoring TBI, comprising a sensor element according to any one of embodiments 20-24, and
   a detection device capable of detecting the binding of a target RNA biomarker to the probe.
26. The detection system according to embodiment 25, further comprising means to determine whether the target RNA biomarker is upregulated or downregulated.
27. A method for determining a course of action for a subject suspected of having TBI, comprising applying a saliva sample obtained from the subject to a detection system according to embodiment 25 or 26, and if an upregulated level or a downregulated level of the at least one RNA biomarker is detected, providing a treatment for TBI.
28. A method of treating a subject with suspected TBI, the method comprising
   determining whether an upregulated level or a downregulated level of at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof, is detectable in a saliva sample obtained from the subject, and
   if an upregulated level or a downregulated level of at least one RNA biomarker is detected, providing treatment for TBI to the subject.
29. The method of embodiment 28, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
30. A method of detecting an RNA biomarker in a saliva sample, the method comprising
   obtaining a saliva sample from a human subject,
   contacting the saliva sample with at least one oligonucleotide primer complementary to at least one RNA biomarker:
      a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
      b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof;
   amplifying the at least one RNA biomarker using a polymerase chain reaction; and
   detecting the amplified RNA biomarker.
31. The method of embodiment 30, wherein the at least one RNA biomarker further comprises one or more miRNA:
   a. selected from the group consisting of has-let-7i-5hashsa-miR-107, hsa-miR-126-5p (=miR-126*), hsa-miR-135hasp, hsa-miR-142-3p, hsa-miR-142-5p, hsa-miR-143-3p (=mhas143), hsa-mhas148a-3phassa-miR-206hassa-miR-29chas, hsa-miR-has-3p, hsahasR-425-5p, has-miR-449a, hsa-has-671-3p, hsa-miR-6748-3p, and hsa-miR-934, or any combination thereof; and/or
   b. selected from the group consisting of hsa-let-7a-5p, hsa-let-7b-has hsa-let-7f-5hashsa-miR-103a-3p, hsa-miR-21-5p, and hsa-miR-92a-3p, or any combination thereof.
32. A kit for use in a method of diagnosing and/or monitoring traumatic brain injury (TBI) in saliva from a human subject, the kit comprising at least one probe specific for at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.
33. The kit of embodiment 32, wherein the probe comprises a nucleic acid able to bind to the at least one RNA biomarker.
34. The kit of embodiment 32 or 33, wherein the probe comprises a nucleic acid having at least 70% identity with a sequence which is the complement of the sequence of the target RNA biomarker.
35. The kit of embodiment 32 or 33, wherein the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.
36. The kit of any one of embodiments 32-35, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
37. A composition for use in a method of diagnosing and/or monitoring traumatic brain injury (TBI) in saliva from a human subject, the composition comprising at least one probe specific for at least one RNA biomarker:
   a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
   b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof.
38. The composition of embodiment 37, wherein the probe comprises a nucleic acid able to bind to the at least one RNA biomarker.
39. The composition of embodiment 37 or 38, wherein the probe comprises a nucleic acid having at least 70% identity with a sequence which is the complement of the sequence of the target RNA biomarker.
40. The composition of embodiment 37 or 38, wherein the probe comprises a nucleic acid having at least 70% identity with a sequence which is the complement of SEQ ID NO: 1, 2, 14, 16, 23, 26, 27, 29, 39, 40, 48, 71, 72, 73, 74, 75, 77, 78, 79, 80, 81, 82, 84, 85, 86, 87, 89, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150.
41. The composition of any one of embodiments 37-40, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
42. A method of diagnosing and treating traumatic brain injury (TBI) in a human subject in need thereof, the method comprising:
   obtaining a saliva sample from the subject;
   contacting the saliva sample with a probe comprising a nucleic acid able to bind to at least one RNA biomarker:
      a. selected from the group consisting of hsa-miR-1246, hsa-miR-126-3p (miR-126*), hsa-miR-144-3p (miR-144*), hsa-miR-144-5p (=miR-144*), hsa-miR-16-1-3p, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, SNORD3B-2, tRNA120-AlaAGC, tRNA18-ArgCCT, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
      b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, RNU6-4, RNU6-45, RNU6-6, RNU6-7, RNU6-73, U6.1249, U6.375, U6.428, andYRNA-255, or any combination thereof;
   determining an amount of the at least one RNA biomarker in the saliva sample;
   identifying the subject as having TBI where the amount of the at least one RNA biomarker is increased or decreased relative to a predetermined threshold value or relative to the amount of the RNA biomarker in a control sample; and
   treating the subject identified as having TBI according to one or more of the following:
      administering to the subject one or more neuroprotective therapies.
43. The method of embodiment 42, wherein the saliva sample is obtained during a period of time after injury selected from immediately after the injury to any period post-injury.
44. The method of embodiment 43, wherein the saliva sample is obtained during a period of time after injury selected from immediately after the injury until the expression level of at least one of the RNA biomarkers returns to the predetermined threshold value or to the amount of the RNA biomarker in the control sample.
45. The method of embodiment 43, wherein the saliva sample is obtained during a period of time after injury selected from immediately after the injury to up to 1 year, up to 10 months, up to 8 months, up to 6 months, up to 5 months, up to 4 months, up to 3 months, up to 2 months, up to 1 month, up to 25 days, up to 20 days, up to 15 days, up to 7 days, up to 5 days, up to 3 days, up to 2 days, and/or up to 24 hours post-injury.
46. The method of embodiment 45, wherein the saliva sample is obtained during a period of time after injury selected from immediately after the injury to 15 days, from 1 hour to 15 days, from 24 hours to 15 days, from 24 hours to 7 days, and from 2 to 5 days.
47. The method of embodiment 42, further comprising obtaining one or more additional saliva samples from the subject at one or more additional times after the injury and repeating the detecting and amplifying steps for each additional sample.
48. The method of embodiment 47, wherein the one or more additional saliva sample is obtained during a period of time after injury selected from immediately after the injury to any period post-injury.
49. The method of embodiment 47, wherein the one or more additional saliva sample is obtained during a period of time after injury selected from immediately after the injury until the expression level of at least one of the RNA biomarkers returns to the predetermined threshold value or to the amount of the RNA biomarker in the control sample.
50. The method of embodiment 47, wherein the one or more additional saliva sample is obtained during a period of time after injury selected from immediately after the injury to up to 1 year, up to 10 months, up to 8 months, up to 6 months, up to 5 months, up to 4 months, up to 3 months, up to 2 months, up to 1 month, up to 25 days, up to 20 days, up to 15 days, up to 7 days, up to 5 days, up to 3 days, up to 2 days, and/or up to 24 hours post-injury.
51. The method of embodiment 50, wherein the one or more additional saliva sample is obtained during a period of time after injury selected from immediately after the injury to 15 days, from 1 hour to 15 days, from 24 hours to 15 days, from 24 hours to 7 days, and from 2 to 5 days.
52. The method of embodiment 47, wherein the one or more additional saliva samples is obtained at day 1, 1.5, 2, 3, 4, 5, 6, 7, 8, 9, 10, 11, 12, 13, 14, or 15 after the injury.
53. The method of any one of embodiments 42-52, wherein the detecting an amount of the at least one RNA biomarker is performed using a PCR-based assay, a light array assay, a laminar flow chip assay, or any assay suitable for detecting that at least one RNA biomarker.
54. The method of embodiment 53, wherein when using the PCR-based assay the predetermined threshold is equivalent to a fold change of 1.5 or more using the 2-delta delta CT (2-ΔΔCT) method.
55. The method of embodiment 53, wherein the predetermined threshold is equivalent to a fold change of 2 or more using the 2-delta delta CT (2-ΔΔCT) method.
56. The method of any one of embodiments 42-55, wherein the at least one RNA biomarker further comprises one or more miRNA:
   a. selected from the group consisting of hsa-let-7i-5p, hsa-miR-107, hsa-miR-126-5p (=miR-126*), hsa-miR-135b-5p, hsa-miR-142-3p, hsa-miR-142-5p, hsa-miR-143-3p (=miR-143), hsa-miR-148a-3p, hsa-miR-206, hsa-miR-29c-3p, hsa-miR-34b-3p, hsa-miR-425-5p, hsa-miR-449a, hsa-miR-671-3p, hsa-miR-6748-3p, and hsa-miR-934, or any combination thereof; and/or
   b. selected from the group consisting of hsa-let-7a-5p, hsa-let-7b-5p, hsa-let-7f-5p, hsa-miR-103a-3p, hsa-miR-21-5p, and hsa-miR-92a-3p, or any combination thereof.
57. The method according to embodiment 42 further comprising identifying the human subject as being fit for normal activity after undergoing successful treatment for TBI.

## Claims

1. A method of diagnosing and/or monitoring traumatic brain injury (TBI) in a subject, the method comprising determining a level of at least one RNA biomarker in a saliva sample obtained from the subject, wherein the at least one RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, wherein the saliva sample is obtained no more than 12 hours after injury.

2. The method of claim 1, wherein either an upregulated level or a downregulated level of the at least one RNA biomarker is indicative of TBI.

3. The method of claim 1, wherein the subject is diagnosed as having TBI if the level of the at least one RNA biomarker is either above or below a predetermined threshold or increased or decreased relative to a control.

4. The method of claim 1, wherein the saliva sample is obtained immediately after injury and/or at 4-12 hours after injury.

5. The method of claim 1, wherein the saliva sample is obtained up to 1 hour after injury.

6. The method of claim 1, further comprising obtaining one or more additional saliva samples from the subject at one or more additional times after the injury and repeating the detecting and amplifying steps for each additional sample, and wherein the one or more additional saliva sample is obtained during a period of time after injury selected from immediately after the injury until the expression level of at least one of the RNA biomarkers returns to the predetermined threshold value or to the amount of the RNA biomarker in the control sample.

7. The method of any one of claims 1-6, wherein the detecting an amount of the at least one RNA biomarker is performed using a PCR-based assay, a light array assay, a laminar flow chip assay, or any assay suitable for detecting that at least one RNA biomarker, optionally wherein when using the PCR-based assay the predetermined threshold is equivalent to a fold change of 1.5 or more using the 2-delta delta CT (2- AACT) method, and further optionally wherein the predetermined threshold is equivalent to a fold change of 2 or more using the 2-delta delta CT (2-AACT) method.

8. The method of any one of claims 1-7, wherein the at least one RNA biomarker further comprises one or more miRNA:
a. selected from the group consisting of hsa-miR-1246, hsa-miR-339-5p, hsa-miR-497-5p, put-miR-1204, put-miR-323, put-miR-325, put-miR-444, put-miR-465, put-miR-469, put-miR-594, put-miR-856, put-miR-893, put-miR-958, RNU4-6p, SNORA57, tRNA120-AlaAGC, tRNA27-MetCAT, tRNA2-LeuTAA, tRNA73-ArgCCG, tRNA8-ThrAGT, tRNA9-TyrGTA, U2.3, U4.64, U6.168, U6.601, UC022CJG1, YRNA-245, and YRNA-684, or any combination thereof; and/or
b. selected from the group consisting of put-miR-1003, put-miR-1080, put-miR-1084, put-miR-1146 (2), put-miR-1207, put-miR-1306, put-miR-188, put-miR-209, put-miR-468, put-miR-476, put-miR-6, put-miR-71, put-miR-742, put-miR-806, put-miR-92, put-miR-961, , U6.1249, U6.375, U6.428, and YRNA-255, or any combination thereof.

9. Use of a sensor element in the method of claims 1-8 for diagnosing and/or monitoring TBI, the sensor element comprising a substrate functionalized with a probe specific for at least one RNA biomarker, and
wherein the at least RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, preferably wherein the probe comprises a nucleic acid able to bind to the at least one RNA biomarker, and further preferably wherein the probe comprises a nucleic acid having at least 70% identity with a sequence which is the complement of one of the following sequences selected from the group consisting of: SEQ ID NO: 113, SEQ ID NO: 111, SEQ ID NO: 114, SEQ ID NO: 98, SEQ ID NO: 101, SEQ ID NO: 100, SEQ ID NO: 99, SEQ ID NO: 102, SEQ ID NO: 136, and SEQ ID NO: 138.

10. Use of a detection system in the method of diagnosing and/or monitoring TBI according to claims 1-8, comprising
the sensor element comprising a substrate functionalized with a probe specific for at least one RNA biomarker, and
wherein the at least RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, and
a detection device capable of detecting the binding of a target RNA biomarker to the probe, preferably wherein the probe comprises a nucleic acid able to bind to the at least one RNA biomarker probe, and further preferably wherein the probe comprises a nucleic acid having at least 70% identity with a sequence which is the complement of one of the following sequences selected from the group consisting of: SEQ ID NO: 113, SEQ ID NO: 111, SEQ ID NO: 114, SEQ ID NO: 98, SEQ ID NO: 101, SEQ ID NO: 100, SEQ ID NO: 99, SEQ ID NO: 102, SEQ ID NO: 136, and SEQ ID NO: 138.

11. Use of the detection system according to claim 10, wherein the detection system further comprises means to determine whether the target RNA biomarker is upregulated or downregulated.

12. A neuroprotective drug for use in a method of treatment in a subject with suspected TBI, the method comprising determining whether an upregulated level or a downregulated level of at least one RNA biomarker is detectable in a saliva sample obtained from the subject no more than 12 hours after injury, wherein the at least RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, and if an upregulated level or a downregulated level of at least one RNA biomarker is detected, providing the neuroprotective drug as treatment for TBI to the subject.

13. The method of claims 1-8, wherein the method further comprises detecting the at least one RNA biomarker in a saliva sample by
obtaining a saliva sample from a human subject,
contacting the saliva sample with at least one oligonucleotide primer complementary to the at least one RNA biomarker, and wherein the at least one RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, amplifying the at least one RNA biomarker using a polymerase chain reaction; and detecting the amplified RNA biomarker.

14. Use of a kit in the method of diagnosing and/or monitoring traumatic brain injury (TBI) in saliva from a human subject according to claims 1-8, the kit comprising at least one probe specific for at least one RNA biomarker:
wherein the at least RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, preferably
wherein the probe comprises a nucleic acid able to bind to the at least one RNA biomarker, and further preferably wherein the probe comprises a nucleic acid having at least 70% identity with a sequence which is the complement of one of the following sequences selected from the group consisting of: SEQ ID NO: 113, SEQ ID NO: 111, SEQ ID NO: 114, SEQ ID NO: 98, SEQ ID NO: 101, SEQ ID NO: 100, SEQ ID NO: 99, SEQ ID NO: 102, SEQ ID NO: 136, and SEQ ID NO: 138.

15. Use of a composition in a method of diagnosing and/or monitoring traumatic brain injury (TBI) in saliva from a human subject according to claims 1-8, the composition comprising at least one probe specific for at least one RNA biomarker, wherein the at least RNA biomarker is selected from the group consisting of: hsa-miR-144-3p (miR-144*), hsa-miR-126-3p (miR-126*), hsa-miR-16-1-3p, RNU6-4, RNU6-45, RNU6-6, RNU6-7, RNU6-73, SNORD3B-2, and tRNA18-ArgCCT, preferably wherein the probe comprises a nucleic acid able to bind to the at least one RNA biomarker, and
further preferably wherein the probe comprises a nucleic acid
having at least 70% identity with a sequence which is the complement of one of the following sequences selected from the group consisting of: SEQ ID NO: 113, SEQ ID NO: 111, SEQ ID NO: 114, SEQ ID NO: 98, SEQ ID NO: 101, SEQ ID NO: 100, SEQ ID NO: 99, SEQ ID NO: 102, SEQ ID NO: 136, and SEQ ID NO: 138.
